# EUROPEAN PATENT APPLICATION

(11) **EP 2 495 305 A1**
(43) Date of publication of application: **05.09.2012**
(21) Application number: 10826848.3
(22) Date of filing: 29.10.2010
(51) Int. Cl.: C12N 1/21, C12N 1/15, C12N 1/19, C12N 15/09, C12P 7/18

(54) **TRANSGENIC MICROORGANISM PROVIDED WITH THE ABILITY TO PRODUCE 1,3-BUTANEDIOL, AND USAGE THEREFOR**

(30) Priority: 30.10.2009 JP 2009251276
(71) Applicant: Daicel Corporation, Osaka 530-0001 (JP)
(72) Inventor: OKABAYASHI, Tomohito, Myoko-shi Niigata 944-8550 (JP); NAKAJIMA, Takanori, Myoko-shi Niigata 944-8550 (JP); YAMAMOTO, Hiroaki, Myoko-shi Niigata 944-8550 (JP)
(74) Representative: Grünecker, Kinkeldey, Stockmair & Schwanhäusser
(86) International application number: PCT/JP2010/069274
(87) International publication number: WO 2011/052718

(57) **Abstract**

An objective of the present invention is to provide recombinant microorganisms efficiently producing optically active 1,3-butanediol, which is useful as a material for synthesizing pharmaceuticals and such, and provide methods for efficiently producing optically active 1,3-butanediol using the recombinant microorganisms. As a result of dedicated research for achieving the above objective, the present inventors succeeded in producing recombinant microorganisms in which the activity of an enzyme catalyzing the reduction reaction represented by Formula 1 is enhanced and which produce a diol compound represented by Formula 2. 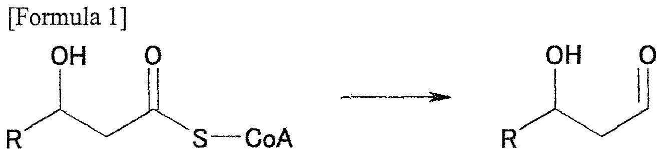 (wherein R represents a C₁₋₃ alkyl group or hydrogen) 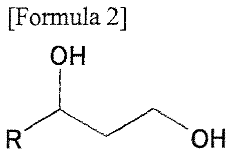 (wherein R represents a C₁₋₃ alkyl group or hydrogen)

## Description

### Technical Field

The present invention is related to recombinant microorganisms with a 1,3-butanediol-producing function, and methods for producing 1,3-butanediol using the microorganisms.

### Background Art

1,3-Butanediol is useful as a chemical with various applications, such as moisturizers, resin materials, surfactants, moisture absorbents, and solvents, and as a raw material thereof. Also, its optically active molecules, (R)-1,3-butanediol and (S)-1,3-butanediol, are useful as raw materials for synthesizing pharmaceuticals, agrochemicals, and such.

Conventionally, 1,3-butanediol is made by chemical production in which acetaldehyde chemically manufactured from petroleum, a fossil resource, is used as a raw material to produce acetaldol, which is then hydrogenated. Meanwhile, optically active 1,3-butanediol can be produced by methods represented by Patent Document 1, in which (R)- or (S)-1,3-butanediol is produced by allowing a microorganism capable of preferentially assimilating either its (S)- or (R)-isomer, such as *Candida palapsilosis* or *Kluyveromyces lactis,* to act on racemic 1,3-butanediol chemically synthesized from fossil resources, and then collecting the remaining enantiomer.

In other methods represented by Patent Document 2, (R)- or (S)-1,3-butanediol is produced by allowing a microorganism such as *Kluyveromyces lactis* or *Candida palapsilosis* to act on 4-hydroxy-2-butanone chemically synthesized from fossil resources, and utilizing the asymmetric reduction activity of the microorganism.

Furthermore, there are methods of producing optically active 1,3-butanediol in one or two steps using stereo-selective oxidoreductases or recombinant bacteria overexpressing such enzymes. Such methods include: the production of (R)-1,3-butanediol from the racemic form using a (S)-specific secondary alcohol dehydrogenase derived from *Geotricum sp.* (Non-patent Document 1); the production of (R)-1,3-butanediol from the racemic form using recombinant *Escherichia coli* expressing a (S)-specific secondary alcohol dehydrogenase derived from *Candida palapsilosis* (Patent Document 3), the production of (R)-1,3-butanediol from 4-hydroxy-2-butanone or the production of (S)-1,3-butanediol from the racemic form using recombinant *E. coli* expressing an (R)-specific 2,3-butanediol dehydrogenase derived from *Kluyveromyces lactis* (Patent Document 4). However, all these methods use a non-natural compound as a substrate, which needs to be chemically synthesized.

Meanwhile, in Non-patent Document 2, 1,3-butanediol was detected in the culture fluid of *Geotricum fragrans* cultured in a medium containing cassava waste. However, 1,3-butanediol was found as one of the volatile substances, and the culture was not intended to produce 1,3-butanediol.

In recent years, from the view point of the depletion of fossil resources and global warming, there has been an increasing social demand for establishment of chemical production systems that use biomass (botanical resource)-derived materials as renewable resources.

For example, it is known that solvents can be produced from glucose, one of the biomass-derived materials, via a CoA-derivative using the acetone-butanol fermentation pathway, as represented by *Clostridium acetobutylicum.* For the type strain of this species, *C*. *acetobutylicum* ATCC824, the entire genomic DNA sequence has been sequenced, and a solvent-producing gene characteristic of acetone-butanol fermentation bacteria, adhE (an aldehyde-alcohol dehydrogenase that has the functions of EC1.2.1.10 and EC1.1.1.1), has been revealed (Non-patent Document 3). There has been few enzymological reports for the gene product of adhE derived from *C. acetobutylicum* (aldehyde-alcohol dehydrogenase that has the functions of EC 1.2.1.10 and EC1.1.1.1), and only the assessment of the cell-free extract of *C*. *acetobutylicum* DSM1732 has been performed for the butanol dehydrogenase activity using butanol or butylaldehyde as a substrate and for the butylaldehyde dehydrogenase activity using butylaldehyde or butyryl-CoA as a substrate, in Non-patent Document 4. The adhE gene product has never been used for other purposes than producing 1-butanol as represented by Non-patent Document 5.

There has been no report of producing 1,3-butanediol from biomass-derived materials, and there has been a demand for a method of producing 1,3-butanediol from renewable resources such as biomass.

### Prior Art Documents

### Patent Documents

[Patent Document 1] Japanese Patent Application Kokai Publication No. (JP-A) H02-195897 (unexamined, published Japanese patent application)
[Patent Document 2] JP-A (Kokai) H02-31684
[Patent Document 3] JP-A (Kokai) 2000-197485
[Patent Document 4] JP-A (Kokai) 2002-345479

### Non-patent Documents

[Non-patent Document 1] Biosci. Biotech. Biochem., 1996, 60(7), 1191-1192
[Non-patent Document 2] Proc. Biochem., 2003, 39, 411-414
[Non-patent Document 3] J. Bacteriol., 2001, 183(16), 4823-4838
[Non-patent Document 4] Appl. Microbiol. Biotechnol., 1987, 26, 268-272
[Non-patent Document 5] Metabol. Engineer., 2008, 10, 305-311

### Summary of the Invention

### [Problems to be Solved by the Invention]

An objective of the present invention is to provide microorganisms capable of producing 1,3-butanediol from renewable resources of biomass (botanical resources). Another objective of the present invention is to provide methods by which 1,3-butanediol can be produced using microorganisms having a desired function(s).

### [Means for Solving the Problems]

As a result of dedicated research to develop a method for producing 1,3-butanediol from biomass, which is a renewable resource, the present inventors have discovered that AdhE derived from *Clostridium acetobutylicum* (CaAdhE) not only generates acetaldehyde and butylaldehyde by reducing acetyl-CoA and butyryl-CoA in an NADH-dependent manner, but also surprisingly generates 3-hydroxybutylaldehyde by reducing 3-hydroxybutyryl-CoA, which has a hydroxyl group at position 3. The present inventors have also discovered that CaAdhE has an activity of reducing 3-hydroxybutylaldehyde in an NADH-dependent manner, thereby catalyzing a reaction of producing 1,3-butanediol. They have successfully achieved efficient production of 1,3-butanediol by culturing, with glucose as a carbon source, recombinant *E. coli* capable of coexpressing CaAdhE with β-ketothiolase, which catalyzes the reaction of generating acetoacetyl-CoA from two molecules of acetyl-CoA, and 3-hydroxybutyryl-CoA dehydrogenase, which catalyzes the reaction of reducing the 3-carbonyl group of acetoacetyl-CoA in an NAD(P)H-dependent manner and thereby generating 3-hydroxybutyryl-CoA.

Based on the above findings, the present inventors have succeeded in producing 1,3-butanediol by allowing the reaction of Formula 8 shown below to occur in microorganisms carrying the above enzymes, thereby completing the present invention.

Thus, the present invention provides 1,3-butanediol-producing microorganisms. Also, the present invention provides methods for efficiently producing 1,3-butanediol using such microorganisms.

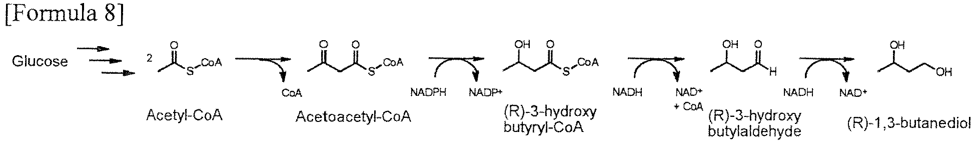

More specifically, the present inventions provides:
[1] a recombinant microorganism in which the enzymatic activity of (1) shown below is enhanced, wherein the microorganism produces 1,3-alkyldiol represented by Formula 2 from a fermentation substrate:
   (1) activity of an enzyme that catalyzes production of 3-hydroxyalkylaldehyde by reducing 3-hydroxyacyl-CoA using NADH and/or NADPH as a coenzyme, as shown by Formula 1: 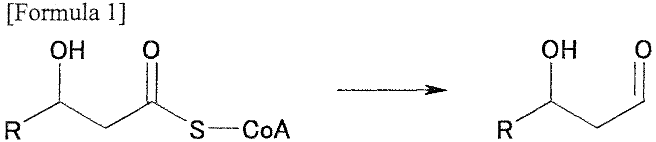 (wherein R represents a C₁₋₃ alkyl group or hydrogen; and CoA represents coenzyme A) 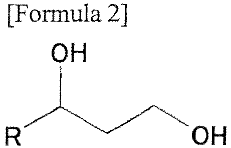 (wherein R represents a C₁₋₃ alkyl group or hydrogen);
[2] the recombinant microorganism of [1], wherein R is methyl in Formulas 1 and 2 recited in [1], and the microorganism produces 1,3-butanediol from a fermentation substrate;
[3] the recombinant microorganism of [1] or [2], wherein 1,3-butanediol produced from the fermentation substrate recited in [2] is (R)- or (S)-1,3-butanediol;
[4] the recombinant microorganism of any one of [1] to [3], wherein the enzyme catalyzing the reaction of Formula 1 recited in [1](1) is classified as EC 1.2.1.10 under the international classification of enzyme;
[5] the recombinant microorganism of any one of [1] to [4], wherein the enzyme catalyzing the reaction of Formula 1 recited in [1](1) is any one of:
   (a) a protein comprising the amino acid sequence of SEQ ID NO: 1, 65, or 67;
   (b) an enzyme comprising an amino acid sequence in which one or more amino acids are substituted, deleted, inserted, or added in the amino acid sequence of SEQ ID NO: 1, 65, or 67;
   (c) an enzyme comprising an amino acid sequence encoded by the nucleotide sequence of SEQ ID NO: 2, 66, or 68;
   (d) an enzyme comprising an amino acid sequence encoded by a DNA which hybridizes under stringent conditions with a DNA comprising the nucleotide sequence of SEQ ID NO: 2, 66, or 68; and
   (e) a protein having an identity of 85% or higher with the amino acid sequence of SEQ ID NO: 1, 65, or 67;
[6] the recombinant microorganism of any one of [1] to [5], in which the enzymatic activity of (2) shown below is enhanced in addition to the enzymatic activity of (1) recited in [1], wherein the microorganism produces 1,3-alkyldiol represented by Formula 2 from a fermentation substrate:
   (1) activity of an enzyme that catalyzes production of 3-hydroxyalkylaldehyde by reducing 3-hydroxyacyl-CoA using NADH and/or NADPH as a coenzyme, as shown by Formula 1; and
   (2) activity of an enzyme that catalyzes production of 1,3-alkyldiol represented by Formula 2 by reducing 3-hydroxyalkylaldehyde using NADH and/or NADPH as a coenzyme, as shown by Formula 3 (Formula 3 does not show two reactions, but only shows the production of alcohol from aldehyde): 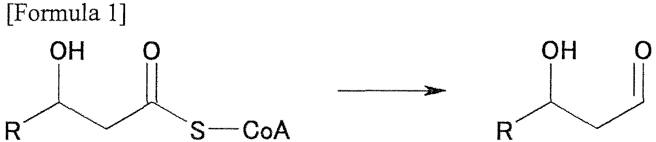
   (wherein R represents a C₁₋₃ alkyl group or hydrogen; and CoA represents coenzyme A) 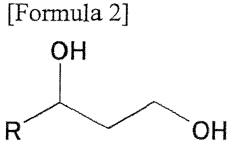 (wherein R represents a C₁₋₃ alkyl group or hydrogen) 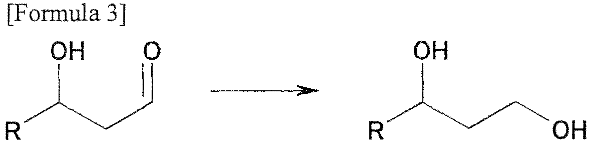 (wherein R represents a C₁₋₃ alkyl group or hydrogen);
[7] the recombinant microorganism of [6], wherein R is methyl in Formulas 1 to 3 recited in [6], and the microorganism produces 1,3-butanediol from a fermentation substrate;
[8] the recombinant microorganism of [6] or [7], wherein 1,3-butanediol produced from the fermentation substrate recited in [7] is (R)- or (S)-1,3-butanediol;
[9] the recombinant microorganism of any one of [6] to [8], wherein the enzyme catalyzing the reaction of Formula 3 recited in [6] is any one of:
   (a) a protein comprising the amino acid sequence of SEQ ID NO: 3, 5, or 7;
   (b) an enzyme comprising an amino acid sequence in which one or more amino acids are substituted, deleted, inserted, or added in the amino acid sequence of SEQ ID NO: 3, 5, or 7;
   (c) an enzyme comprising an amino acid sequence encoded by the nucleotide sequence of SEQ ID NO: 4, 6, or 8;
   (d) an enzyme comprising an amino acid sequence encoded by a DNA which hybridizes under stringent conditions with a DNA comprising the nucleotide sequence of SEQ ID NO: 4, 6, or 8; and
   (e) a protein having an identity of 85% or higher with the amino acid sequence of SEQ ID NO: 3, 5, or 7;
[10] the recombinant microorganism of any one of [1] to [5], in which the enzymatic activity of (3) shown below is enhanced in addition to the enzymatic activity of (1) recited in [1], wherein the microorganism produces 1,3-alkyldiol represented by Formula 2 from a fermentation substrate:
   (1) activity of an enzyme that catalyzes production of 3-hydroxyalkylaldehyde by reducing 3-hydroxyacyl-CoA using NADH and/or NADPH as a coenzyme, as shown by Formula 1; and (3) activity of an enzyme that produces 3-hydroxyacyl-CoA by reducing 3-oxoacyl-CoA in an NADH- and/or NADPH-dependent manner as shown by Formula 4: 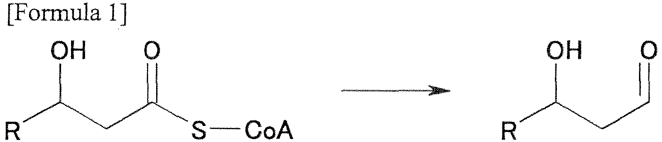 (wherein R represents a C₁₋₃ alkyl group or hydrogen; and CoA represents coenzyme A) 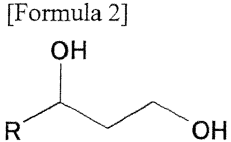 (wherein R represents a C₁₋₃ alkyl group or hydrogen) 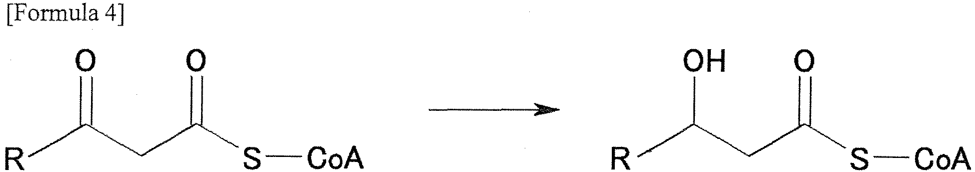 (wherein R represents a C₁₋₃ alkyl group or hydrogen; and CoA represents coenzyme A);
[11] the recombinant microorganism of [10], wherein R is methyl in Formulas 1 and 2 recited in [10], and the microorganism produces (R)-1,3-butanediol represented by Formula 5 from a fermentation substrate: 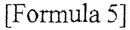 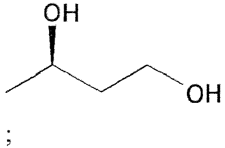
[12] the recombinant microorganism of [10] or [11], wherein the enzyme catalyzing the reaction of Formula 4 recited in [10] is an R-form-specific reductase, and is any one of:
   (a) a protein comprising the amino acid sequence of SEQ ID NO: 9, 11, 13, 15, or 17;
   (b) an enzyme comprising an amino acid sequence in which one or more amino acids are substituted, deleted, inserted, or added in the amino acid sequence of SEQ ID NO: 9, 11, 13, 15, or 17:
   (c) an enzyme comprising an amino acid sequence encoded by the nucleotide sequence of SEQ ID NO: 10, 12, 14, 16, or 18;
   (d) an enzyme comprising an amino acid sequence encoded by a DNA which hybridizes under stringent conditions with a DNA comprising the nucleotide sequence of SEQ ID NO: 10, 12, 14, 16, or 18 ; and
   (e) an enzyme having an identity of 85% or higher with the amino acid sequence of SEQ ID NO: 9, 11, 13, 15, or 17;
[13] the recombinant microorganism of [10], wherein R is methyl in Formulas 1 and 2 recited in [10], and the microorganism produces (S)-1,3-butanediol represented by Formula 6 from a fermentation substrate: 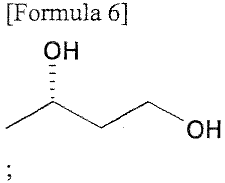
[14] the recombinant microorganism of [10] or [13], wherein the enzyme catalyzing the reaction of Formula 4 recited in [10] is an S-form-specific reductase, and is any one of:
   (a) a protein comprising the amino acid sequence of SEQ ID NO: 19;
   (b) an enzyme comprising an amino acid sequence in which one or more amino acids are substituted, deleted, inserted, or added in the amino acid sequence of SEQ ID NO: 19;
   (c) an enzyme comprising an amino acid sequence encoded by the nucleotide sequence of SEQ ID NO: 20;
   (d) an enzyme comprising an amino acid sequence encoded by a DNA which hybridizes under stringent conditions with a DNA comprising the nucleotide sequence of SEQ ID NO: 20; and
   (e) an enzyme having an identity of 85% or higher with the amino acid sequence of SEQ ID NO: 19;
[15] the recombinant microorganism of any one of [1] to [5], in which the enzymatic activity of (4) shown below is enhanced in addition to the enzymatic activity of (1) recited in [1], wherein the microorganism produces (R)- or (S)-1,3-butanediol represented by Formula 2 from a fermentation substrate:
   (1) activity of an enzyme that catalyzes production of 3-hydroxybutylaldehyde by reducing 3-hydroxybutyryl-CoA using NADH and/or NADPH as a coenzyme, as shown by Formula 1; and
   (4) activity of a β-ketothiolase that catalyzes production of acetoacetyl-CoA from two molecules of acetyl-CoA as shown by Formula 7: 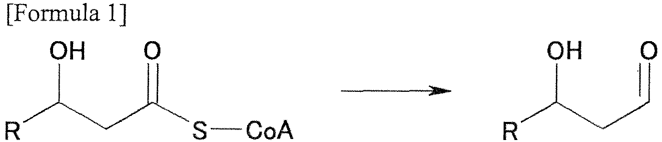
   (wherein R represents methyl; and CoA represents coenzyme A) 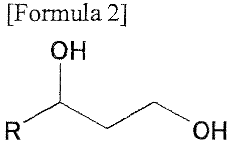 (wherein R represents methyl) 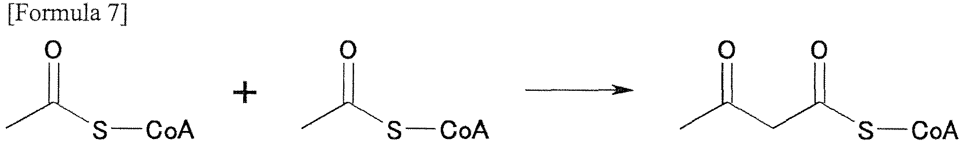 (wherein CoA represents coenzyme A);
[16] the recombinant microorganism of [15], wherein the enzyme catalyzing the reaction of Formula 7 recited in [15] is any one of:
   (a) a protein comprising the amino acid sequence of SEQ ID NO: 21, 23, or 25;
   (b) an enzyme comprising an amino acid sequence in which one or more amino acids are substituted, deleted, inserted, or added in the amino acid sequence of SEQ ID NO: 21, 23, or 25;
   (c) an enzyme comprising an amino acid sequence encoded by the nucleotide sequence of SEQ ID NO: 22, 24, or 26;
   (d) an enzyme comprising an amino acid sequence encoded by a DNA which hybridizes under stringent conditions with a DNA comprising the nucleotide sequence of SEQ ID NO: 22, 24, or 26; and
   (e) an enzyme having an identity of 85% or higher with the amino acid sequence of SEQ ID NO: 21, 23, or 25;
[17] the recombinant microorganism of any one of [1] to [5], in which the enzymatic activities of (2) to (4) shown below are enhanced in addition to the enzymatic activity of (1) recited in [1], wherein the microorganism produces (R)- or (S)-1,3-butanediol represented by Formula 2 from a fermentation substrate:
   (1) activity of an enzyme that catalyzes production of 3-hydroxybutylaldehyde by reducing β-hydroxybutyryl-CoA using NADPH and/or NADPH as a coenzyme, as shown by Formula 1;
   (2) activity of an enzyme that catalyzes production of 1,3-butanediol represented by Formula 2 by reducing 3-hydroxybutylaldehyde using NADH and/or NADPH as a coenzyme, as shown by Formula 3 (Formula 3 does not show two reactions, but only shows the production of alcohol from aldehyde);
   (3) activity of an enzyme that produces 3-hydroxybutyryl-CoA by reducing acetoacetyl-CoA in an NADH- and/or NADPH-dependent manner as shown by Formula 4;
   (4) activity of a β-ketothiolase that catalyzes production of acetoacetyl-CoA from two molecules of acetyl-CoA as shown by Formula 7: 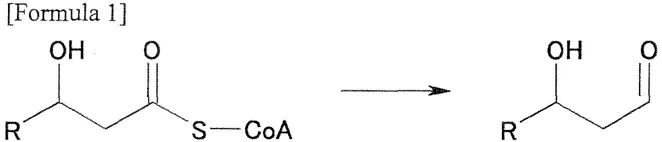
   (wherein R represents methyl; and CoA represents coenzyme A) 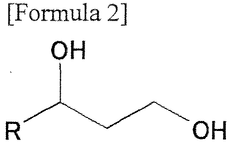 (wherein R represents methyl) 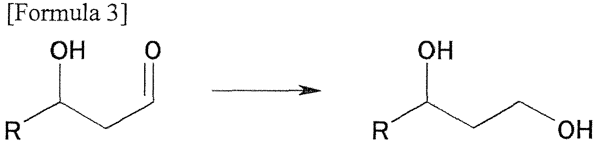 (wherein R represents methyl) 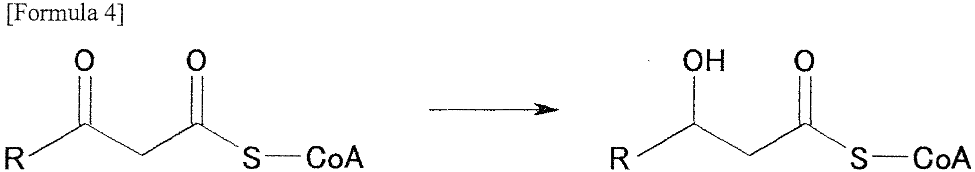 (wherein R represents methyl, and CoA represents coenzyme A) 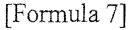 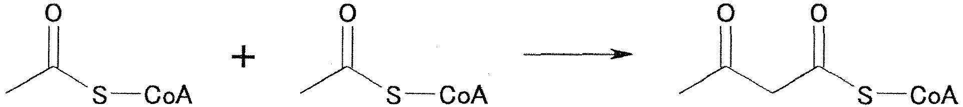 (wherein CoA represents coenzyme A);
[18] the recombinant microorganism of any one of [1] to [17], wherein the host cell is *Escherichia coli;*
[19] a method for producing a diol compound represented by Formula 2, comprising the steps of:
   contacting a fermentation substrate with at least one active material selected from the group consisting of a culture of the recombinant microorganism of any one of [1] to [18], a cell of the recombinant microorganism, and a processed product thereof; and
   collecting 1,3-alkyodiol represented by Formula 2: 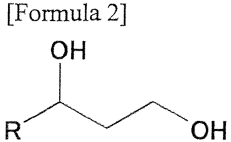
   (wherein R represents a C₁₋₃ alkyl group or hydrogen);
[20] the method of [19], wherein the diol compound is (R)-1,3-butanediol represented by Formula 5 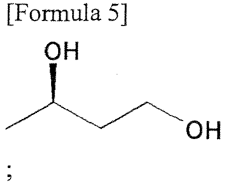
[21] the method of [19], wherein the diol compound is (S)-1,3-butanediol represented by Formula 6;
[22] the method of any one of [19] to [21], wherein the fermentation substrate is selected from the group consisting of sugars and glycerol;
[23] the method of [22], wherein the fermentation substrate is selected from the group consisting of glucose, lactose, xylose, sucrose, and glycerol; and
[24] the method of any one of [19] to [23], wherein the culturing of the recombinant microorganism and the production of the diol compound are carried out separately.

### Brief Description of the Drawings

Fig. 1 shows the production of a plasmid containing the entire ReTHL gene.
Fig. 2 shows the production of a plasmid containing the entire ZrTHL gene.
Fig. 3 shows the production of a plasmid containing the entire EcTHL gene.
Fig. 4 shows the production of a plasmid containing the entire ReTHL and ReAR genes.
Fig. 5 shows the production of a plasmid containing the entire ReTHL and ZrAR1 genes.
Fig. 6 shows the production of a plasmid containing the entire ReTHL and SvKR1 genes.
Fig. 7 shows the production of a plasmid containing the entire ReTHL and BstKR1 genes.
Fig. 8 shows the production of a plasmid containing the entire ReTHL and CaHBD genes.
Fig. 9 shows the production of a plasmid containing the entire ReTHL and PfODH genes.
Fig. 10 shows the production of a plasmid containing the entire CaAdhE gene.
Fig. 11 shows the production of a plasmid containing the entire TpAdhE gene.
Fig. 12 shows the production of a plasmid containing the entire PfALD gene.
Fig. 13 shows the production of a plasmid containing the entire ReTHL, ReAR1, and CaAdhE genes.
Fig. 14 shows the production of a plasmid containing the entire CaBDHB gene.
Fig. 15 shows the production of a plasmid containing the entire ReTHL, ReAR1, CaAdhE, and CaBDHB genes.

### Mode for Carrying Out the Invention

The present invention is related to recombinant microorganisms in which the enzymatic activity of (1) shown below is enhanced, and which produce a diol compound represented by Formula 2 (1,3-alkyldiol) from a fermentation substrate.
(1) activity of an enzyme that catalyzes production of3-hydroxyalkylaldehyde by reducing 3-hydroxyacyl-CoA using NADH and/or NADPH as a coenzyme, as shown by Formula 1:

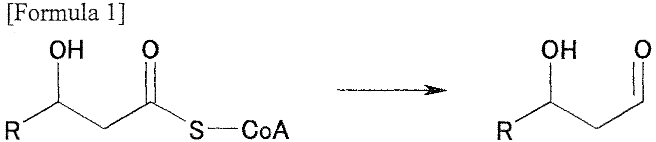

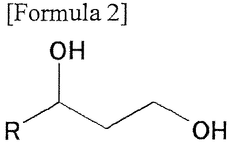

In the present invention, the diol compound represented by Formula 2 is preferably produced via the reduction reaction of Formula 1. In the present invention, R in Formula 1 is preferably a C₁₋₃ alkyl group (methyl group, ethyl group, propyl group, or isopropyl group) or hydrogen. In Formula 1, CoA represents coenzyme A.

Preferred diol compounds produced in the present invention include compounds of Formula 2 in which, for example, R is a C₁₋₃ alkyl group (methyl group, ethyl group, propyl group, or isopropyl group) or hydrogen. More specifically, preferred examples of the diol compounds produced in the present invention include 1,3-butanediol (where R is methyl in both Formulas 1 and 2). The optical activity of 1,3-butanediol produced in the present invention is not particularly limited, and the R-form and S-form of 1,3-butanediol ((R)- and (S)-1,3-butanediol) are included in 1,3-butanediol produced in the present invention.

In the present invention, the enzymes that produce 3-hydroxybutylaldehyde by reducing 3-hydroxybutyryl-CoA using NADH and/or NADPH as a coenzyme as shown by Formula 1 include enzymes classified as EC 1.2.1.10, which have the systematic name "acetaldehyde: NAD⁺ oxidoreductase (CoA-acetylating)" given by the International Union of Biochemistry and Molecular Biology (IUBMB) (http://www.chem.qmul.ac.uk:/iubmb/), and catalyze the following reaction: aldehyde + CoA + NAD⁺ = acyl-CoA + NADH + H⁺. EC numbers are sets of four numbers for systematically classifying enzymes according to their reaction schemes, and are defined by the Enzyme Commission of IUBMB. Specifically, the above enzymes include those of microorganisms having a butanol fermentation pathway which catalyze the production of butylaldehyde by NADH- or NADPH-dependent reduction of butyryl-CoA (for example, butylaldehyde dehydrogenase) in the butanol biosynthesis pathway. Genes encoding these enzymes or enzymes catalyzing a similar reaction are generally termed adhE.

More specifically, the above enzymes include gene products of adhE derived from *Clostridium* bacteria such as *Clostridium acetobutylicum, Clostridium beijerinckii, Clostridium saccharoacetobutylicum,* and *Clostridium saccharoperbutylacetonicum.*

Preferred enzymes having the function of EC1.2.1.10 are enzymes with the recommended name "aldehyde-alcohol dehydrogenase" according to UniProt (http://www.uniprot.org/), including bifunctional enzymes catalyzing both the reaction of producing aldehyde from acyl-CoA and the reaction of producing alcohol from aldehyde, and enzymes only catalyzing the reaction of producing aldehyde from acyl-CoA. Specifically, the gene product of adhE1 or the gene product of adhE derived from *Clostridium acetobutylicum* can be used for the present purposes. The adhE gene and mhpF gene possessed by *Escherichia coli,* lactic acid bacteria, and such can also be used. Specifically, the adhE gene and mhpF gene derived from *Escherichia coli,* and the adhE gene product derived from *Leuconostoc mesenteroides* can be used. In addition to the above-described genes, genes suitable for the present purposes can also be selected from microorganisms whose genomic DNA has been sequenced. Specifically, genes from *Thermoanaerobacter pseudethanolicus* and *Propionibacterium freudenreichii subsp. freudenreichii* can be suitably used.

In the present invention, the enzymes having a butylaldehyde dehydrogenase activity include:
(a) proteins having the amino acid sequence of SEQ ID NO: 1, 65, or 67;
(b) enzymes having an amino acid sequence in which one or more (2 or more, preferably 2 to 20, and more preferably 2 to 5) amino acids are substituted, deleted, inserted, or added in the amino acid sequence of SEQ ID NO: 1, 65, or 67;
(c) enzymes having an amino acid sequence encoded by the nucleotide sequence of SEQ ID NO: 2, 66, or 68;
(d) enzymes having an amino acid sequence encoded by a DNA which hybridizes under stringent conditions with a DNA comprising the nucleotide sequence of SEQ ID NO: 2, 66, or 68; and
(e) proteins having an identity of 85% or higher with the amino acid sequence of SEQ ID NO: 1, 65, or 67.

In the present invention, a homologue of an "enzyme comprising the amino acid sequence of a certain sequence identification number (SEQ ID NO)" can be rephrased as "an enzyme having an amino acid sequence in which one or more (2 or more, preferably 2 to 20, and more preferably 2 to 5) amino acids are substituted, deleted, inserted, or added in the amino acid sequence of a certain sequence identification number. This homologue refers to a protein that is functionally equivalent to an enzyme composed of the amino acid sequence of the specified sequence identification number. In the present invention, "functionally equivalent" means that a protein has the same enzymatic activity (catalytic reaction, chemical reaction, and such) of each enzyme described herein.

In the amino acid sequence of a certain sequence identification number, for example, 100 amino acid residues or less, usually 50 amino acid residues or less, preferably 30 amino acid residues or less, more preferably 15 amino acid residues or less, even more preferably 10 amino acid residues or less, or 5 amino acid residues or less may be mutated. Generally, in order to maintain the function of protein, an amino acid is preferably substituted with an amino acid having similar properties. Such amino acid substitution is referred to as conservative substitution. For example, Ala, Val, Leu, Ile, Pro, Met, Phe, and Trp are all classified as non-polar amino acids, and therefore possess similar properties. Uncharged amino acids include Gly, Ser, Thr, Cys, Tyr, Asn, and Gln. Acidic amino acids include Asp and Glu. Basic amino acids include Lys, Arg, and His. Substitution of amino acids within each group is acceptable.

A person skilled in the art can obtain a polynucleotide encoding a homologue of each enzyme by appropriately introducing substitution, deletion, insertion, and/or addition to the DNA of the enzyme which is composed of a nucleotide sequence disclosed herein, using methods such as site-specific mutagenesis (Nucleic Acid Res. 10, pp.6487 (1982), Methods in Enzymol.100, pp.448 (1983), Molecular Cloning 2nd Edt., Cold Spring Harbor Laboratory Press (1989), PCR A Practical Approach IRL Press pp.200 (1991)). The polynucleotide encoding a homologue of each enzyme can be introduced and expressed in a host to obtain the homologue.

Furthermore, in the present invention, a homologue of each enzyme refers to a protein which has an identity of at least 50%, preferably at least 70%, more preferably 80%, more preferably 85%, more preferably 90%, even more preferably 95% or higher (for example, 95%, 96%, 97%, 98%, or 99% or higher) with the amino acid sequence of SEQ ID NO corresponding to the enzyme. Protein homology searches can be carried out, for example, through the Internet by using a program such as BLAST and FASTA on amino acid sequence databases such as SWISS-PROT, PIR, and DAD, DNA sequence databases such as DDBJ, EMBL, and Genbank, databases of deduced amino acid sequences based on DNA sequences, or such.

Each of the enzymes described herein can be attached to an additional amino acid sequence as long as it retains an activity functionally equivalent to that of the enzyme. For example, a tag sequence such as histidine tag and HA tag can be added. Alternatively, the enzyme can be fused with another protein. Each enzyme of the present invention or a homologue thereof may be a fragment as long as it retains an activity functionally equivalent to that of the enzyme.

A polynucleotide encoding each of the enzymes described herein can be isolated by methods described below. For example, DNA of the present invention can be obtained by performing PCR using PCR primers designed based on the nucleotide sequence of SEQ ID NO corresponding to the enzyme, and using as a template a chromosomal DNA or a cDNA library from a strain producing the enzyme. Moreover, a polynucleotide of each enzyme can be obtained by performing colony hybridization or plaque hybridization using the obtained DNA fragment as a probe and using a cDNA library or a library obtained by transforming E. *coli* with phages or plasmids containing restriction enzyme digests of the chromosomal DNA of a strain producing the enzyme.

In addition, a polynucleotide of each enzyme can also be obtained as follows: The DNA fragment obtained by PCR is sequenced, and the obtained nucleotide sequence is used to design PCR primers to extend outwardly from a known DNA. The chromosomal DNA of a strain producing the enzyme is digested by appropriate restriction enzymes, and DNAs resulting from self-circularization of the digests are used as templates to perform inverse PCR (Genetics 120, 621-623 (1988)). Alternatively, the RACE (Rapid Amplification of cDNA End) method ("PCR Experiment Manual", p. 25-33, HBJ Publication Office) and such can also be performed.

In the present invention, the polynucleotides of each enzyme include genomic DNAs and cDNAs cloned by the above methods, and synthetically produced DNAs.

Hybridization is performed on a subject nucleic acid by using as a probe a nucleic acid (DNA or RNA) consisting of a complementary sequence or a partial sequence thereof of the nucleotide sequence of SEQ ID NO corresponding to each enzyme, and the presence or absence of significant hybridization between the probe and the subject nucleic acid is confirmed after washes under stringent conditions. The length of a probe used is, for example, consecutive 20 nucleotides or more, preferably 25 nucleotides or more, more preferably 30 nucleotides or more, more preferably 40 nucleotides or more, more preferably 80 nucleotides or more, more preferably 100 nucleotides or more (for example, a full length of the nucleotide sequence of SEQ ID NO corresponding to the enzyme). If a probe contains a sequence irrelevant to the nucleotide sequence of SEQ ID NO corresponding to the enzyme or its complementary sequence (such as a vector-derived sequence), this sequence can be used alone as a negative control probe to perform hybridization in the same manner, and the absence of significant hybridization between the probe and the subject sequence may be confirmed after washes under the same conditions. Hybridization can be carried out by common methods using nitrocellulose or nylon membranes (Sambrook et al. (1989) Molecular Cloning, Cold Spring Harbor Laboratories; Ausubel, F.M. et al. (1994) Current Protocols in Molecular Biology, Greene Publishe Associates/ John Wiley and Sons, New York. NY).

Specific examples of stringent hybridization conditions are, for example, overnight hybridization in a solution containing 6 x SSC, 0.5%(w/v) SDS, 100 µg/ml denatured salmon sperm DNA, and 5 x Denhardt's solution (1 x Denhardt's solution contains 0.2% polyvinylpyrrolidone, 0.2% bovine serum albumin, and 0.2% Ficoll) at 45°C, preferably 55°C, more preferably 60°C, and even more preferably 65°C, followed by three 20-min post-hybridization washes in 4 x SSC and 0.5% SDS at the same temperature as hybridization. More preferably, post-hybridization washes are carried out with two 20-min washes in 4 x SSC and 0.5% SDS followed by one 20-min wash in 2 x SSC and 0.5% SDS, at the same temperature as hybridization. More preferably, post-hybridization washes are carried out with two 20-min washes in 4 x SSC and 0.5% SDS, followed by one 20-min wash in 1 x SSC and 0.5% SDS, at the same temperature as hybridization. More preferably, post-hybridization washes are carried out with one 20-min wash in 2 x SSC and 0.5% SDS, followed by one 20-min wash in 1 x SSC and 0.5% SDS, and one subsequent 20-min wash in 0.5 x SSC and 0.5% SDS, at the same temperature as hybridization. More preferably, post-hybridization washes are carried out with one 20-min wash in 2 x SSC and 0.5% SDS, followed by one 20-min wash in 1 x SSC and 0.5% SDS, one 20-min wash in 0.5 x SSC and 0.5% SDS, and one 20-min wash in 0.1 x SSC and 0.5% SDS, at the same temperature as hybridization.

The activity of an enzyme having the function of EC 1.2.1.10 which can be utilized in the present purposes can be confirmed, for example, as described below.

### Butylaldehyde dehydrogenase (CoA-acetylation) (BCDH activity assay (enzymatic activity shown by Formula 1)

A mixed solution of 100 mM Tris-HCl buffer (pH 6.5), 70 mM semicarbazide (pH 6.5), 0.2 mM NADH, 0.2 mM 3-hydroxybutyryl-CoA or butyryl-CoA, and as necessary, 1 mM DTT, is equilibrated at 30°C for three minutes. A cell-free extract containing BCDH is added to the solution, and a decrease in absorbance at 340 nm associated with a decrease of NADH due to the reduction of acyl-CoA is measured. If an enzyme of interest may be inactivated in the presence of oxygen, the reaction solution is prepared and reacted under an anaerobic atmosphere (under a nitrogen atmosphere). One unit of an enzyme is defined as an amount of the enzyme which catalyzes a decrease of 1 µmol NADH per minute under these conditions. Quantification of protein is carried out by a dye-binding assay using a protein assay kit manufactured by BioRad, and using bovine plasma albumin as a reference protein.

The present invention is related to recombinant microorganisms in which the enzymatic activity of (2) shown below is enhanced in addition to the enzymatic activity of (1) described above, and which produce 1,3-alkyldiol represented by Formula 2 from a fermentation substrate: (1) activity of an enzyme that catalyzes production of 3-hydroxyalkylaldehyde by reducing 3-hydroxyacyl-CoA using NADH and/or NADPH as a coenzyme, as shown by Formula 1; and (2) activity of an enzyme that catalyzes production of 1,3-alkyldiol represented by Formula 2 by reducing 3-hydroxyalkylaldehyde using NADH and/or NADPH as a coenzyme, as shown by Formula 3 (Formula 3 does not show two reactions, but only shows the production of alcohol from aldehyde). 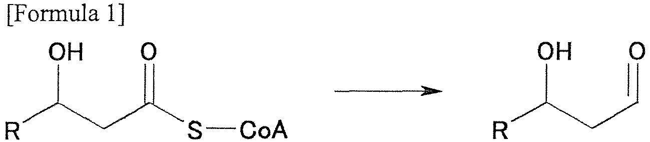 (wherein R represents C₁₋₃ alkyl or hydrogen; and CoA represents coenzyme A) 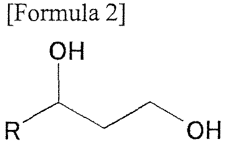 (wherein R represents C₁₋₃ alkyl or hydrogen) 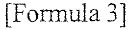 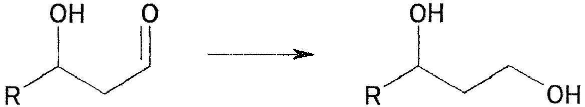 (wherein R represents C₁₋₃ alkyl or hydrogen)

Preferred diol compounds produced in the present invention include compounds of Formula 2 in which R is a C₁₋₃ alkyl group (methyl group, ethyl group, propyl group, or isopropyl group) or hydrogen. More specifically, preferred examples of the diol compounds produced in the present invention include 1,3-butanediol (where R is methyl in Formulas 1 to 3). The optical activity of 1,3-butanediol produced in the present invention is not particularly limited, and the R-form and S-form of 1,3-butanediol ((R)- and (S)-1,3-butanediol) are included in 1,3-butanediol produced in the present invention.

In the present invention, the enzymes that catalyze the production of 1,3-alkyldiol represented by Formula 2 by reducing 3-hydroxyalkylaldehyde using NADH and/or NADPH as a coenzyme as shown by Formula 3 include enzymes classified as EC1.1.1.1, which have the systematic name of "alcohol: NAD⁺ oxidoreductase" given by the International Union of Biochemistry and Molecular Biology (IUBMB) (http:/www.chem.qmul.ac.uk/iubmb/), and catalyze the following reaction: alcohol + NAD⁺ = aldehyde or keton + NADH + H⁺. Specifically, the above enzymes include butanol dehydrogenase derived from *Clostridium acetobutylicum.*

In the present invention, the enzymes having a butanol dehydrogenase activity include:
(a) proteins having the amino acid sequence of SEQ ID NO: 3, 5, or 7;
(b) enzymes having an amino acid sequence in which one or more amino acids are substituted, deleted, inserted, or added in the amino acid sequence of SEQ ID NO: 3, 5, or 7;
(c) enzymes having an amino acid sequence encoded by the nucleotide sequence of SEQ ID NO: 4, 6, or 8;
(d) enzymes having an amino acid sequence encoded by a DNA which hybridizes under stringent conditions with a DNA comprising the nucleotide sequence of SEQ ID NO: 4, 6, or 8; and
(e) enzymes having an identity of 85% or higher with the amino acid sequence of SEQ ID NO: 3, 5, or 7.

The activity of an enzyme having the function of EC1.1.1.1 which can be utilized in the present purposes can be confirmed, for example, as described below.

### Butanol dehydrogenase) (BDH) activity assay (enzymatic activity shown by Formula 2)

A mixed solution of 50 mM MES buffer (pH 6.0), 0.2 mM NADH, 20 mM 3-hydroxybutylaldehyde or butylaldehyde, and as necessary, 1 mM DTT, is equilibrated at 30°C for three minutes. A cell-free extract containing BDH is added to the solution, and a decrease in absorbance at 340 nm associated with a decrease of NADH due to the reduction of alkylaldehyde is measured. If an enzyme of interest may be inactivated in the presence of oxygen, the reaction solution is prepared and reacted under an anaerobic atmosphere (under a nitrogen atmosphere). One unit of an enzyme is defined as an amount of the enzyme which catalyzes a decrease of 1 µmol NADH per minute. Quantification of protein is carried out by a dye-binding assay using a protein assay kit manufactured by BioRad, and using bovine plasma albumin as a reference protein.

The present invention is related to recombinant microorganisms in which the enzymatic activity of (3) shown below is enhanced in addition to the enzymatic activity of (1) described above, and which produce 1,3-alkyldiol represented by Formula 2 from a fermentation substrate: (1) activity of an enzyme that catalyzes production of 3-hydroxyalkylaldehyde by reducing 3-hydroxyacyl-CoA using NADH and/or NADPH as a coenzyme, as shown by Formula 1; and (3) activity of an enzyme that produces 3-hydroxyacyl-CoA by reducing 3-oxoacyl-CoA in an NADH- and/or NADPH-dependent manner as shown by Formula 4. 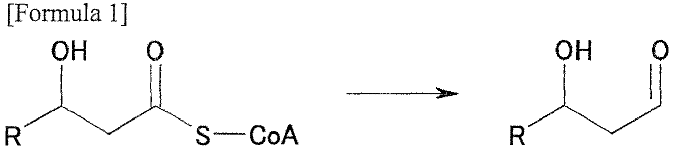 (wherein R represents C₁₋₃ alkyl or hydrogen; and CoA represents coenzyme A) 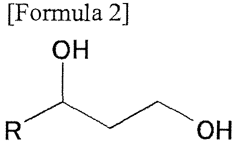 (wherein R represents C₁₋₃ alkyl or hydrogen) 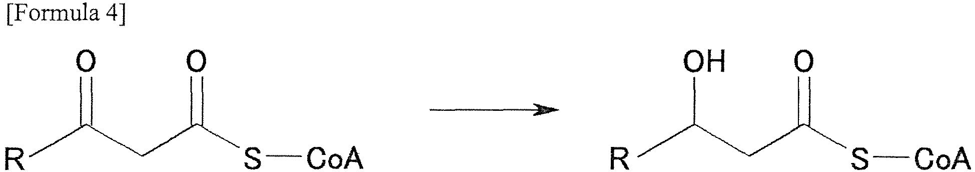 (wherein R represents C₁₋₃ alkyl or hydrogen; and CoA represents coenzyme A)

Preferred diol compounds produced in the present invention include compounds of Formula 2 in which R is a C₁₋₃ alkyl group (methyl group, ethyl group, propyl group, or isopropyl group) or hydrogen. More specifically, preferred examples of the diol compounds produced in the present invention include 1,3-butanediol (where R is methyl in Formulas 1, 2, and 4). The optical activity of 1,3-butanediol produced in the present invention is not particularly limited, and the R-form and S-form of 1,3-butanediol ((R)- and (S)-1,3-butanediol) are included in 1,3-butanediol produced in the present invention.

In the present invention, any enzymes capable of catalyzing the reaction of producing 3-hydroxybutyryl-CoA by reducing the 3-carbonyl group of acetoacetyl-CoA can be used for the reaction of producing 3-hydroxyacyl-CoA by reducing 3-oxoacyl-CaA as shown by Formula 4 (for example, when R is methyl, the reaction of producing 3-hydroxybutyryl-CoA from acetoacetyl-CoA). Preferably, such enzymes are usually possessed by microorganisms having a poly(3-hydroxybutanoic acid) (PHB) synthesis pathway or a butanol fermentation pathway. Microorganisms having a PHB synthesis pathway include *Ralstonia eutropha* and *Zoogloea ramigera,* and the acetoacetyl-CoA reductases (whose genes are generally denoted as phaB or phbB) of such microorganisms are examples of preferred enzymes. Microorganisms having a butanol fermentation pathway include *Clostridium* bacteria, known as acetone-butanol fermentation bacteria as mentioned above, and the 3-hydroxybutyryl-CoA dehydrogenases (whose genes are generally denoted as hbd) of such microorganisms are included. Moreover, from the viewpoint of enzyme-substrate specificity, enzymes catalyzing a similar reaction can also be used. For example, β-ketoacyl-ACP reductases in fatty acid synthesis pathways, specifically, β-ketoacyl-ACP reductase (BstKR1) derived from *Bacillus stearothermophilus,* can be used. In addition, β-ketoacyl reductase derived from *Streptomyces violαceoruber* (SvKR1), an enzyme in the polyketide actinorhodin synthesis pathway, can also be used. As other examples, carbonyl reductases that reduce β-ketocarboxylic acid or the 3-carbonyl group of its ester can be used. More specifically, (R)-2-octanol dehydrogenase derived from *Pichia finlandica* (PfODH) can be used. If these reductases have high stereoselectivity, they are suitable for obtaining optically active 1,3-butanediol. To obtain (R)-1,3-butanediol, acetoacetyl-CoA reductase derived from *Ralstonia eutropha* (ReAR1), acetoacetyl-CoA reductase derived from *Zoogloea ramigera* (ZrAR1), BstKR1, SvKR1, and PfODH, which are highly (R)-selective, and more preferably, ReAR1, can be used as suitable enzymes for producing (R)-1,3-butanediol. To obtain (S)-1,3-butanediol, HBD derived from *Clostridium,* which is highly (S)-selective, and more preferably, 3-hydroxybutyryl-CoA dehydrogenase derived from *Clostridium acetobutylicum* (CaHBD), can be used as suitable enzymes for producing (S)-1,3-butanediol. These compounds are preferred optically-active alcohols that can be produced by the present invention.

Of the enzymes producing 3-hydroxyacyl-CoA by reducing 3-oxoacyl-CoA in the present invention (for example, when R is methyl, the reaction of producing 3-hydroxybutyryl-CoA from acetoacetyl-CoA), preferred enzymes for producing (R)-1,3-butanediol as a final product include:
(a) proteins having the amino acid sequence of SEQ ID NO: 9, 11, 13, 15, or 17;
(b) enzymes having an amino acid sequence in which one or more amino acids are substituted, deleted, inserted, or added in the amino acid sequence of SEQ ID NO: 9, 11, 13, 15, or 17;
(c) enzymes having an amino acid sequence encoded by the nucleotide sequence of SEQ ID NO: 10, 12, 14, 16, or 18;
(d) enzymes having an amino acid sequence encoded by a DNA which hybridizes under stringent conditions with a DNA comprising the nucleotide sequence of SEQ ID NO: 10, 12, 14, 16, or 18; and
(e) enzymes having an identity of 85% or higher with the amino acid sequence of SEQ ID NO: 9, 11, 13, 15, or 17.

Of the enzymes producing 3-hydroxyacyl-CoA by reducing 3-oxoacyl-CoA in the present invention (for example, when R is methyl, the reaction of producing 3-hydroxybutyryl-CoA from acetoacetyl-CoA), preferred enzymes for producing (S)-1,3-butanediol as a final product include:
(a) proteins having the amino acid sequence of SEQ ID NO: 19;
(b) enzymes having an amino acid sequence in which one or more amino acids are substituted, deleted, inserted, or added in the amino acid sequence of SEQ ID NO: 19;
(c) enzymes having an amino acid sequence encoded by the nucleotide sequence of SEQ ID NO: 20;
(d) enzymes having an amino acid sequence encoded by a DNA which hybridizes under stringent conditions with a DNA comprising the nucleotide sequence of SEQ ID NO: 20; and
(e) enzymes having an identity of 85% or higher with the amino acid sequence of SEQ ID NO: 19.

The activity of an enzyme reducing the 3-carbonyl group of 3-hydroxybutyryl-CoA which can be utilized in the present purposes can be confirmed, for example, as described below.

### 3-Hydroxybutyryl-CoA dehydrogenase 3HBD) activity assay

A mixed solution of 100 mM potassium phosphate buffer (pH 6.5), 0.2 mM NAD(P)H, 0.2 mM acetoacetyl-CoA, and as necessary, 1 mM DTT, is equilibrated at 30°C for three minutes. A cell-free extract containing 3HBD is added to the solution, and a decrease in absorbance at 340 nm associated with a decrease of NAD(P)H due to the reduction of acetoacetyl-CoA is measured. One unit of an enzyme is defined as an amount of the enzyme which catalyzes a decrease of 1 µmol NAD(P)H per minute. Quantification of protein is carried out by a dye-binding assay using a protein assay kit manufactured by BioRad, and using bovine plasma albumin as a reference protein.

The present invention is related to recombinant microorganisms in which the enzymatic activity of (4) shown below is enhanced in addition to the enzymatic activity of (1), and which produce (R)- or (S)-1,3-butanediol represented by Formula 2 from a fermentation substrate:
(1) activity of an enzyme that catalyzes production of 3-hydroxybutylaldehyde by reducing β-hydroxybutyryl-CoA using NADH and/or NADPH as a coenzyme, as shown by Formula 1; and
(4) activity of a β-ketothiolase that catalyzes production of acetoacetyl-CoA from two molecules of acetyl-CoA, as shown by Formula 7. 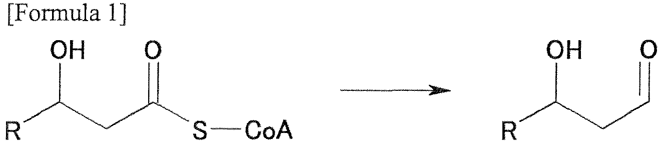
(wherein R represents methyl; and CoA represents coenzyme A) 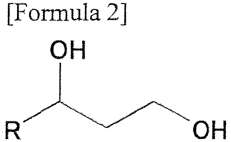 (wherein R represents methyl) 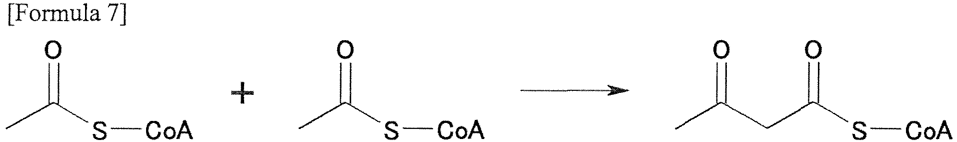 (wherein CoA represents coenzyme A)

Preferred diol compounds produced in the present invention include 1,3-butanediol (where R is methyl in Formulas 1, 2, and 7). The optical activity of 1,3-butanediol produced in the present invention is not particularly limited, and the R-form and S-form of 1,3-butanediol ((R)- and (S)-1,3-butanediol) are included in 1,3-butanediol produced in the present invention.

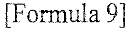

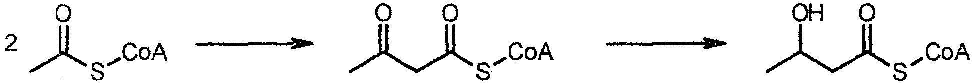

Of the reactions shown by Formula 9, the reaction from acetyl-CoA to acetoacetyl-CoA (the reaction of Formula 7) can be performed using any enzyme capable of producing acetoacetyl-CoA by condensing two molecules of acetyl-CoA. Preferred enzymes include enzymes classified as EC2.3.1.9, which have the systematic name of "acetyl-CoA:acetyl-CoA C-acetyltransferase" given by IUBMB and catalyze the following reaction: 2 acetyl-CoA = CoA + acetoacetyl-CoA; and enzymes classified as EC2.3.1.16, which have the systematic name of "acyl-CoA:acetyl-CoA C-acyltransferase" and catalyze the following reaction: acyl-CoA + acetyl-CoA = CoA + 3-oxoacyl-CoA (http://www.chem.qmul.ac.uk/iubmb/).

Specifically, enzymes of poly(3-hydroxybutanoic acid) (PHB) synthesis pathways, butanol fermentation pathways, and such, which are possessed by microorganisms and the like, can be usually used. More specifically, microorganisms having a PHB synthesis pathway include *Ralstonia eutropha* and *Zoogloea ramigera,* and acetyl-CoA acetyltransferases or β-ketothiolases of such microorganisms (whose genes are generally denoted as phaA and phbA) are examples of preferred enzymes. In addition, microorganisms having a butanol fermentation pathway include *Clostridium* bacteria, which are known as acetone-butanol fermentation bacteria as described above, and acetyl-CoA acetyltransferases or β-ketothiolases of such microorganisms (whose genes are generally denoted as thl and thi) are examples of preferred enzymes. Other examples include the product of a gene generally denoted as atoB, which is produced by *Escherichia coli* and such. Moreover, β-ketoacyl-ACP synthases of fatty acid biosynthesis systems, which are typically possessed by microorganisms, can also be used.

In the present invention, the enzymes having an acetyl-CoA acetyltransferase activity include:
(a) proteins having the amino acid sequence of SEQ ID NO: 21, 23, or 25;
(b) enzymes having an amino acid sequence in which one or more amino acids are substituted, deleted, inserted, or added in the amino acid sequence of SEQ ID NO: 21, 23, or 25;
(c) enzymes having an amino acid sequence encoded by the nucleotide sequence of SEQ ID NO: 22, 24, or 26;
(d) enzymes having an amino acid sequence encoded by a DNA which hybridizes under stringent conditions with a DNA comprising the nucleotide sequence of SEQ ID NO: 22, 24, or 26; and
(e) enzymes having an identity of 85% or higher with the amino acid sequence of SEQ ID NO: 21, 23, or 25.

The activity of an acetyl-CoA acetyltransferase (or β-ketothiolase) which can be utilized in the present purposes can be confirmed, for example, as described below.

### β-Ketothiolase (THL) activity assay-1

A mixed solution of 100 mM Tris-HCl buffer (pH 8.0), 10 mM magnesium chloride, 0.2 mM CoA, 0.05 mM acetoacetyl-CoA, and as necessary, 1 mM DTT, is equilibrated at 30°C for three minutes. A cell-free extract containing β-ketothiolase is added to the solution, and degradation of Mg²⁺-acetoacetyl-CoA complex is measured as a decrease in absorbance at 303 nm. One unit of an enzyme is defined as an amount of the enzyme which catalyzes a decrease of 1 µmol acetoacetyl-CoA per minute. Quantification of protein is carried out by a dye-binding assay using a protein assay kit manufactured by BioRad, and using bovine plasma albumin as a reference protein.

### β-Ketothiolase (THL) activity assay-2

A mixed solution of 100 mM Tris-HCl buffer (pH 7.5), 2.0 mM NADH, 0.2 mM acetyl-CoA, and 2.0 U of 3-hydroxybutyryl-CoA dehydrogenase derived from *Clostridium acetobutylicum,* is equilibrated at 30°C for three minutes. A cell-free extract containing β-ketothiolase is added to the solution, and a decrease in absorbance at 340 nm associated with a decrease of NADH during the reduction of acetoacetyl-CoA following the condensation of acetyl-CoA, is measured. One unit of an enzyme is defined as an amount of the enzyme which catalyzes a decrease of 1 µmol NADH per minute. Quantification of protein is carried out by a dye-binding assay using a protein assay kit manufactured by BioRad, and using bovine plasma albumin as a reference protein.

The present invention is related to recombinant microorganisms in which the enzymatic activities of (2) to (4) shown below are enhanced in addition to the enzymatic activity of (1) shown below, and which produce (R)- or (S)-1,3-butanediol represented by Formula 2 from a fermentation substrate:
(1) activity of an enzyme that catalyzes production of 3-hydroxybutylaldehyde by reducing β-hydroxybutyryl-CoA using NADH and/or NADPH as a coenzyme, as shown by Formula 1;
(2) activity of an enzyme that catalyzes production of 1,3-butanediol represented by Formula 2 by reducing 3-hydroxybutylaldehyde using NADH and/or NADPH as a coenzyme, as shown by Formula 3 (Formula 3 does not show two reactions, but only shows the production of alcohol from aldehyde);
(3) activity of an enzyme that produces 3-hydroxybutyryl-CoA by reducing acetoacetyl-CoA in an NADH- and/or NADPH-dependent manner as shown by Formula 4; and
(4) activity of a β-ketothiolase that catalyzes production of actoacetyl-CoA from two molecules of acetyl-CoA as shown by Formula 7. 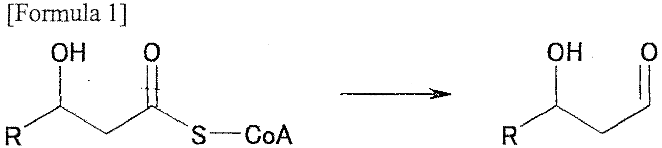
(wherein R represents methyl; and CoA represents coenzyme A) 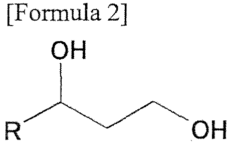 (wherein R represents methyl) 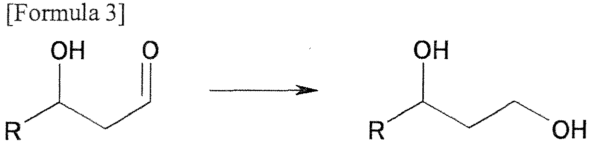 (wherein R represents methyl) 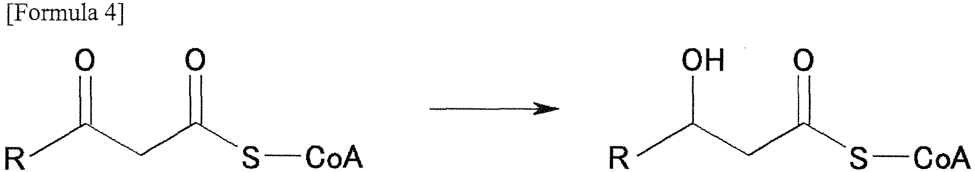 (wherein R represents methyl; and CoA represents coenzyme A) 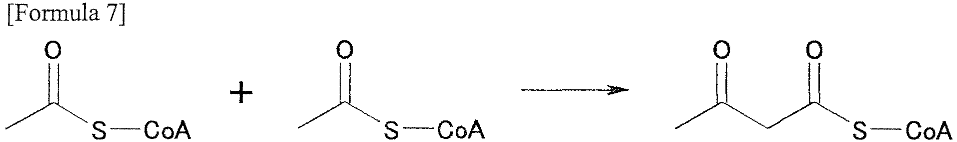 (wherein CoA represents coenzyme A)

In each of the above enzymatic reactions, the above-described enzymes suitable for each step can be used.

For the recombinant microorganisms of the present invention, host cells are not particularly limited, but more preferably, *Escherichia coli* can be used as a host cell.

In the present invention, the phrase "activity is enhanced" refers to a recombinant microorganism that is produced by introducing into a host a gene of interest that is derived from the same or different species and is not possessed by or is expressed at an extremely low level by the host, such that the recombinant microorganism has twice or more, preferably three times or more, more preferably five times or more, even more preferably 10 times or more the activity of the host.

In the present invention, an "optically active alcohol" refers to an alcohol containing a greater amount of one of its optical isomers than the other optical isomers. In the present invention, preferred optically active amine derivatives have, for example, 60%, typically 70% or higher, preferably 80% or higher, and still more preferably 90% or higher enantiomeric excess (ee). The "optical isomers" of the present invention may also be generally referred to as "enantiomers".

Polynucleotides encoding the enzymes of the present invention (for example, enzymes having the function of EC 1.2.1.10) can be isolated by methods as described below.

DNAs of the present invention can be obtained by desigining PCR primers based on a known nucleotide sequence corresponding to each enzyme, and then performing PCR by using as a template a chromosomal DNA or a cDNA library obtained from a strain producing the enzyme.

Furthermore, polynucleotides of the present invention can be obtained by performing colony hybridization or plaque hybridization using the obtained DNA fragment as a probe and using a cDNA library or a library obtained by transforming *E. coli* with phages or plasmids containing restriction enzyme digests of the chromosomal DNA of an enzyme-producing strain.

In addition, polynucleotides of the present invention can also be obtained as follows: The DNA fragment obtained by PCR is sequenced, and the obtained nucleotide sequence is used to design PCR primers to extend outwardly from a known DNA. The chromosomal DNA of an enzyme-producing strain is digested by appropriate restriction enzymes, and DNAs resulting from self-circularization of the digests are used as templates to perform inverse PCR (Genetics 120, 621-623 (1988)). Alternatively, the RACE (Rapid Amplification of cDNA End) method ("PCR Experiment Manual", p. 25-33, HBJ Publication Office) and such can also be performed.

The polynucleotides of the present invention include genomic DNAs and cDNAs cloned by the above methods, and synthetically produced DNAs.

Expression vectors for the enzymes of the present invention (for example, enzymes having the function of EC 1.2.1.10) are provided by introducing a polynucleotide encoding each enzyme isolated as described above into known expression vectors. Preferably, a polynucleotide encoding the above enzyme and a polynucleotide(s) encoding acetyl-CoA-acetyltransferase (or β-ketothiolase) and/or stereoselective 3-hydroxybutyryl-CoA dehydrogenase and/or butanol dehydrogenase as obtained by the same methods described above are inserted into a known vector at the same time.

In the present invention, microorganisms subjected to transformation at least for expressing each enzyme (for example, an enzyme having the function of EC1.2.1.10) are not particularly limited as long as they can be transformed with a recombinant vector containing a polynucleotide encoding a polypeptide having the enzyme (for example, an enzyme having the function of EC 1.2.1.10), and can express the activity of the enzyme (for example, an enzyme having the function of EC1.2.1.10). Microorganisms that can be used include, for example:
*Escherichia;*
*Bacillus;*
*Pseudomonas;*
*Serratia;*
*Brevibacterium;*
*Corynebacterium;*
*Streptococcus;*
Bacteria for which host-vector systems have been developed, such as *Lactobacillus;*
*Rhodococcus;*
Actinomycetes for which host-vector systems have been developed, such as *Streptomyces;*
*Saccharomyces;*
*Kluyveromyces;*
*Schizosaccharomyces;*
*Zygosaccharomyces;*
*Yarrowia;*
*Trichosporon;*
*Rhodosporidium;*
*Pichia;*
Yeasts for which host-vector systems have been developed, such as *Candida;*
*Neurospora;*
*Aspergillus;*
*Cephalosporium;* and
Fungi for which host-vector systems have been developed, such as *Trichoderma.*

Preparation of transformants and construction of recombinant vectors adapted to hosts can be carried out according to techniques commonly used in the fields of molecular biology, bioengineering, and genetic engineering (for example, Sambrook et al., Molecular Cloning, Cold Spring Harbor Laboratories).

In order to express a gene of each enzyme of the present invention (for example, an enzyme having the function of EC1.2.1.10) in microbial cells or such, DNA of the present invention is first introduced into a plasmid or phage vector that can be stably present within a microorganism and allow the organism to transcribe and translate its genetic information. To achieve this, a promoter, which is a regulatory unit for transcription/translation, is inserted 5'-upstream of the DNA strand of the present invention, and more preferably, a terminator is inserted 3'-downstream of the DNA. Any promoters or terminators known to be functional in a microorganism to be used as a host are used. Vectors, promoters, terminators, and such that can be used in various microorganisms are described in detail in, for example, "Fundamental Microbiology (Biseibutsugaku Kiso-kouza) 8: Genetic Engineering, KYORITSU SHUPPAN CO., LTD.". In particular, those to be used with yeasts are described in detail in Adv. Biochem. Eng. 43, 75-102 (1990); Yeast 8, 423-488 (1992); and such.

For the genus *Escherichia,* in particular *Escherichia coli,* available plasmid vectors include the pBR series and pUC series plasmids. Available promoters include promoters derived from lac (β-galactosidase), trp (tryptophan operon), tac and trc (fusion of lac and trp), and PL and PR of λ phage. Available terminators are those derived from trpA, phages, rrnB ribosomal RNA, etc.

For the genus *Bacillus,* available vectors include pUB 110 series and pC 194 series plasmids, and they can be integrated into a chromosome. Available promoters and terminators are those derived from apr (alkaline protease), npr (neutral protease), amy (α-amylase), etc.

For the genus *Pseudomonas,* host-vector systems have been developed for *Pseudomonas putida, Pseudomonas cepacia,* and such. It is possible to use wide host-range vectors such as pKT240 (containing genes required for autonomous replication derived from RSF1010 and such) constructed based on the TOL plasmid, which is involved in the degradation of toluene compounds. Available promoters and terminators include those from the lipase gene (JP-A (Kokai) H05-284973).

For the genus *Brevibacterium,* in particular *Brevibacterium lactofermentum,* it is possible to use plasmid vectors such as pAJ43 (Gene 39, 281 (1985)). Promoters and terminators used for *E. coli* can be used without modification.

For the genus *Corynebacterium,* in particular *Corynebacterium glutamicum,* it is possible to use plasmid vectors such as pCS11 (JP-A (Kokai) S57-183799) and pCB101 (Mol. Gen. Genet. 196, 175 (1984)).

For the genus *Streptococcus,* it is possible to use plasmid vectors such as pHV1301 (FEMS Microbiol. Lett. 26, 239 (1985)) and pGK1 (Appl. Environ. Microbiol. 50, 94 (1985)).

For the genus *Lactobacillus,* it is possible to use pAMβ1 (J. Bacteriol. 13 7, 614 (1979)), which has been developed for *Streptococcus,* and such. Promoters used for E. *coli* can be used.

For the genus *Rhodococcus,* it is possible to use plasmid vectors and such isolated from *Rhodococcus rhodochrous* (J. Gen. Microbiol. 138, 1003 (1992)).

For the genus *Streptomyces,* plasmids can be constructed by the method described in Hopwood et al., Genetic Manipulation of Streptomyces: A Laboratory Manual, Cold Spring Harbor Laboratories (1985). In particular, for *Streptomyces lividans,* it is possible to use pIJ486 (Mol. Gen. Genet. 203, 468-478, 1986), pKC1064 (Gene 103, 97-99 (1991)), pUWL-KS (Gene 165, 149-150 (1995)), and such. The same plasmids can also be used for *Streptomyces virginia* (Actinomycetol. 11, 46-53 (1997)).

For the genus *Saccharomyces,* in particular *Saccharomyces cerevisiae,* it is possible to use YRp series plamids, YEp series plasmids, YCp series plasmids, YIp series plasmids and such. Integration vectors that can be homologously recombined with ribosomal DNA (e.g. EP 537456), multiple copies of which are present within chromosomes, are highly useful since they allow multiple copies of a gene to be introduced and stably maintained. In addition, promoters and terminators such as those derived from ADH (alcohol dehydrogenase), GAPDH (glyceraldehyde-3-phosphate dehydrogenase), PHO (acid phosphatase), GAL (β-galactosidase), PGK (phosphoglycerate kinase), and ENO (enolase) can be used.

For the genus *Kluyveromyces,* in particular *Kluyveromyces lactis,* it is possible to use *Saccharomyces cerevisiae-derived* 2µm plasmids, pKD1 series plasmids (J. Bacteriol. 145, 382-390 (1981)), plasmids derived from pGK11 which is involved in the killer activity, plasmids based on KARS which is an autonomously replicating gene in *Kluyveromyces,* vector plasmids that can be integrated into chromosomes by homologous recombination with ribosome DNA or such (e.g. EP 537456), and the like. Promoters and terminators derived from ADH, PGK, and such can also be used.

For the genus *Schizosaccharomyces,* it is possible to use ARS (a gene involved in autonomous replication) derived from *Schizosaccharomyces pombe,* plasmid vectors derived from *Saccharomyces cerevisiae* that contain an auxotrophy-complementing selection marker (Mol. Cell. Biol. 6, 80 (1986)), and such. In addition, the ADH promoter derived from *Schizosaccharomyces pombe* and such can be used (EMBO J. 6, 729 (1987)). In particular, pAUR224 is commercially available from Takara and can be readily used.

For the genus *Zygosaccharomyces,* it is possible to use plasmid vectors derived from pSB3 derived from *Zygosaccharomyces rouxii* (Nucleic Acids Res. 13, 4267 (1985)), and such. The PHO5 promoter derived from *Saccharomyces cerevisiae,* the promoter of GAP-Zr (glyceraldehyde-3-phosphate dehydrogenase) (Agri. Biol. Chem. 54, 2521 (1990)), and such can be used.

For the genus *Pichia,* host-vector systems have been developed for *Pichia angusta* (previously called *Hansenula polymorpha).* Although *Pichia angusta-derived* genes involved in autonomous replication (HARS1 and HARS2) are available as vectors, they are rather unstable and thus multicopy chromosomal integration is effective (Yeast 7, 431-443 (1991)). In addition, methanol-inducible promoters of alcohol oxidase (AOX) and formate dehydrogenase (FDH) and the like are available. Furthermore, host-vector systems based on Pichia-derived genes involved in autonomous replication (PARS1, PARS2) have been developed (Mol. Cell. Biol. 5, 3376 (1985)). Strong promoters such as AOX promoter, which is inducible by high-cell-density-culture and methanol, can also be employed (Nucleic Acids Res. 15, 3859 (1987)).

In the genus *Candida,* host-vector systems have been developed for *Candida maltosa, Candida albicans, Candida tropicalis, Candida utilis,* etc. An ARS originating from *Candida maltosa* has been cloned (Agri. Biol. Chem. 51, 51, 1587 (1987)), and a vector using this sequence has been developed for *Candida maltosa.* Furthermore, a strong promoter for a chromosomal integration vector has been developed for *Candida utilis* (JP-A (Kokai) Hei 48-173170).

For the genus *Aspergillus, Aspergillus niger* and *Aspergillus oryzae* are the most studied fungi, and plasmid vectors and chromosome-integration vectors are available. Promoters derived from an extracellular protease gene and amylase gene can be used (Trends in Biotechnology 7, 283-287 (1989)).

For the genus *Trichoderma,* host-vector systems have been developed for *Trichoderma reesei,* and promoters such as those derived from extracellular cellulase genes are available (Biotechnology 7, 596-603 (1989)).

Various host-vector systems have also been developed for plants and animals. In particular, systems for expressing a large amount of foreign protein in insects such as silkworm (Nature 315, 592-594 (1985)), and plants such as rapeseed, maize, and potato, have been developed and they can be suitably used.

The present invention is related to methods for producing 1,3-butanediol using a recombinant microorganism in which the above-mentioned enzymatic activity(ies) is enhanced, and which produces an alcohol compound represented by Formula 2. More specifically, the present invention is related to methods for producing a diol compound represented by Formula 2, comprising the steps of contacting a fermentation substrate with at least one active material selected from the group consisting of a culture of a recombinant microorganism which functionally expresses the above enzymatic activity(ies), a cell of the microorganism, and a processed product thereof, and collecting 1,3-alkyldiol represented by Formula 2. 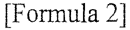 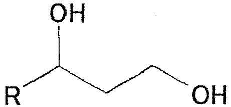 (wherein R represents C₁₋₃ alkyl or hydrogen)

Preferred diol compounds produced in the present invention include compounds of Formula 2 in which, for example, R is a C₁₋₃ alkyl group (methyl group, ethyl group, propyl group, or isopropyl group) or hydrogen. More specifically, preferred examples of the diol compounds produced in the present invention include 1,3-butanediol (where R is methyl in Formulas 1, 2, and 4). The optical activity of 1,3-butanediol produced in the present invention is not particularly limited, and the R-form and S-form of 1,3-butanediol ((R)- and (S)-1,3-butanediol) are included in 1,3-butanediol produced in the present invention.

Production of 1,3-butanediol can be carried out by contacting a fermentation substrate with, for example, a recombinant microorganism that functionally expresses the above enzyme(s), thereby allowing the recombinant microorganism to assimilate the fermentation substrate and perform a desired enzymatic reaction(s). The mode of contact between the recombinant microorganism and the fermentation substrate is not limited to these specific examples. The fermentation substrate is dissolved in a suitable solvent to provide a desirable environment for the recombinant microorganism to assimilate the fermentation substrate and express the desired enzymatic activity.

Preferred microorganisms used in the above methods include transformants that functionally express an enzyme(s) suitable for the above-mentioned step(s).

In the present invention, processed products of a transformant in which the enzymatic activity catalyzing the reduction reaction of Formula 1 is enhanced include, specifically, a microorganism with cellular membrane permeability altered by treatment with surfactant or organic solvent such as toluene; dried cells prepared by lyophilization or spray drying; a cell-free extract obtained by disrupting cells with glass beads or by enzymatic treatment, or a partially-purified product thereof; a purified enzyme; and an immobilized enzyme or immobilized microorganism.

Fermentation substrates used as a raw material in the methods for producing 1,3-butanediol in the present invention include sugars such as glucose, lactose, xylose, and sucrose. In addition, depending on the host microorganism used, it is possible to use any substrates such as glycerol and CO₂ which can be catabolized by the microorganism into acetyl-CoA and/or acetoacetyl-CoA and/or 3-hydroxybutyryl-CoA.

3-Hydroxybutylaldehyde, a substrate used in the activity assay for enzymes having only the function of EC1.1.1.1 according to the present invention, can be synthesized by, for example, reacting two equivalents of acetaldehyde in an ether solvent.

Furthermore, 1,3-butanediol can be produced more efficiently by simultaneously introducing an acetyl-CoA acetyltransferase (or β-ketothiolase) gene and/or 3-hydroxybutyryl-CoA dehydrogenase gene into a transformant in which the enzymatic activity catalyzing the reduction reaction of Formula 1 is enhanced. Moreover, butyrylaldehyde dehydrogenase and/or butanol dehydrogenase genes may be introduced simultaneously. Two or more of these genes can be introduced into a host by methods such as the following: transforming the host with genes that have been separately inserted into multiple recombinant vectors having different replication origins in order to avoid incompatibility; introducing all genes into a single vector; and introducing one or more genes into chromosomes.

When multiple genes are introduced into a single vector, each gene can be ligated to expression regulatory regions such as a promoter and terminator. Multiple genes can also be expressed as a polycistronic operon like the lactose operon.

When 1,3-butanediol is produced by contacting a fermentation substrate with a transformant of the present invention in which the enzymatic activity catalyzing the reduction reaction of Formula 1 is enhanced, or with a processed product thereof, it is possible to select conditions preferred for the activity and stability of the enzyme catalyzing the reduction reaction of Formula 1, and/or for the activity of the transformant to assimilate the fermentation substrate.

The concentration of a fermentation substrate used as a raw material for producing 1,3-butanediol is not particularly limited, but is usually about 0.1-30%, preferably 0.5-15%, and more preferably 1-10%.

In the present invention, "%" always means "weight/volume (w/v)". In the case of (R)-1,3-butanediol, "%e.e." refers to a value calculated by (([concentration of (R)-1,3-butanediol]-[concentration of (S)-1,3-butanediol])/([concentration of (R)-1,3-butanediol]+[concentration of (S)-1,3 -butanediol])) x 100. Likewise, in the case of (S)-1,3-butanediol, "%e.e." refers to a value calculated by (([concentration of (S)-l,3-butanediol]-[concentration of (R)-1,3-butanediol])/([concentration of (S)-1,3 -butanediol]+[concentration of (R)-1,3-butanediol])) x 100.

A raw material may be added all at once when starting fermentation, but may also be added continuously or intermittently to the fermentation liquid.

As a recombinant microorganism of the present invention, preferably, it is possible to use recombinant *E*. *coli* prepared by isolating a gene of an enzyme catalyzing the reduction reaction of Formula 1 and enhancing its activity in host *E*. *coli.* Recombinant *E*. *coli* used for the present purposes can be cultured in media that are commonly used to culture *E*. *coli,* and induced to overexpress the gene by known methods. For example, *E*. *coli* cells in which the above-mentioned enzymatic activity is enhanced are cultured in 2 x YT medium (2.0% Bacto-tryptone, 1.0% Bacto-yeast extract, 1.0% sodium chloride, pH 7.2), and are induced to express the enzyme by isopropyl-thio-β-D-galactopyranoside (IPTG). After the cells are proliferated sufficiently, the culture itself or cells harvested therefrom can be used for 1,3-butanediol production.

Recombinant microorganisms used for 1,3-butanediol production in the present invention may be allowed to produce 1,3-butanediol while being grown in a fermentation liquid for 1,3-butanediol production which contains a fermentation substrate as described below. It is possible to use a previously-grown culture or harvested cells. The amount of a previously-grown culture or harvested cells is usually about 0.1%-100%, preferably 0.5%-50%, and more preferably 1-20%, relative to the amount of the fermentation liquid for 1,3-butanediol production containing a fermentation substrate ("%" mentioned here refers to the ratio of inoculated cells to the fermentation liquid for 1,3-butanediol production, which is represented by [previously-grown culture]/[fermentation liquid for 1,3-butanediol production] (v/v)).

In addition to a fermentation substrate for 1,3-butanediol production, the fermentation liquid for 1,3-butanediol production preferably contains, as necessary, materials that promote the production of 1,3-butanediol. It may contain culture medium components that serve as nutrients for recombinant *E*. *coli* used for the present purposes. Specifically, such components include those of media used for culturing *E*. *coli* such as LB (1.0% Bacto-tryptone, 0.5% Bacto-yeast extract, 1.0% sodium chloride, pH 7.2), 2 x YT (2.0% Bacto-tryptone, 1.0% Bacto-yeast extract, 1.0% sodium chloride, pH 7.2), and M9 medium (6.8 g/L Na₂HPO₄, 3.0 g/L KH₂PO₄, 0.5 g/L NaCl, 1.0 g/L NH₄Cl, 0.493 g/L MgSO₄·7H₂O, 14.7 mg/L CaCl₂·2H₂O, pH7.5). When a sufficient amount of previously-grown cells are provided in the fermentation liquid for 1,3-butanediol production, 1,3-butanediol can be produced in the presence of a higher concentration of a fermentation substrate, and it is also possible to remove the above-mentioned media. In addition, the fermentation liquid may also contain a component to maintain pH suitable for the 1,3-butanediol production during fermentation, such as a buffer agent at a concentration of 10 mM to 800 mM, preferably 50 mM to 500 mM, and more preferably 100 mM to 250 mM. Specifically, such buffer agents include MOPS buffer, HEPES buffer, MES buffer, Tris buffer, and phosphate buffer. Fermentation may be performed at any temperature that allows a recombinant microorganism of the present invention to exhibit its ability to assimilate a fermentation substrate, express the enzymatic activity to catalyze the reduction reaction of Formula 1, and thereby produce 1,3-butanediol. Such temperature may be usually in the range of 5°C to 60°C, preferably 10°C to 50°C, and more preferably 20°C to 40°C. Also, fermentation may be performed at any pH that allows the expression of the enzymatic activity catalyzing the reduction reaction of Formula 1 and the production of 1,3-butanediol. Such pH may be usually in the range of pH 4 to 12, preferably pH 5 to 11, and more preferably pH 6 to 9. Fermentation can be performed while stirring or standing still. In addition, in order to efficiently convert a fermentation substrate into 1,3-butanediol, fermentation can be performed in a reaction medium under aerobic conditions in which a sufficient amount of oxygen is supplied, under microaerobic conditions in which the supply of oxygen is limited, or under anaerobic conditions in which no oxygen is supplied.

The production of 1,3-butanediol in the present invention can be performed in water, in a water-insoluble organic solvent such as ethyl acetate, butyl acetate, toluene, chloroform, n-hexane, methyl isobutyl ketone, methyl tertiary butyl ether, and diisopropyl ether; in a two-phase system with an aqueous medium, or in a mixture system with a water-soluble organic solvent such as methanol, ethanol, isopropyl alcohol, acetonitrile, acetone, and dimethyl sulfoxide. The reaction in the present invention may be performed in batch, fed-batch, or continuous systems, and it is also possible to use immobilized cells, immobilized enzymes, or membrane reactors.

Purification of 1,3-butanediol produced by the reaction can be performed by appropriately combining the following: separation by centrifugation, filtration etc., organic solvent extraction, chromatographies such as ion exchange chromatography, adsorption using adsorbents, dehydration or agglomeration using dehydrating or agglomerating agents, crystallization, distillation, etc.

For example, after a fermentation liquid containing microorganism cells is subjected to centrifugation or membrane filtration to remove microorganism cells and proteins, 1,3-butanediol can be purified from this aqueous solution by known methods such as concentration and distillation.

All prior art documents cited in the present specification are incorporated herein by reference.

### [Example]

Hereinbelow, the present invention will be specifically described using Examples, but it is not to be construed as being limited thereto.

### [Example 1] Cloning of β-ketothiolase gene derived from Ralstonia eutropha

*Ralstonia eutropha* DSM 531 was inoculated in 50 mL of liquid medium consisting of 5 g/L peptone and 3 g/L meat extract, which had been adjusted to pH 7.0. The cells were cultured with shaking at 30°C for 21 hours.

The cells were collected from the resulting culture by centrifugation, and genomic DNA was obtained from the cells. The genomic DNA was prepared using Genomic Tip-100/G Kit (QIAGEN).

In order to clone the phbA gene present in the genomic DNA obtained from *Ralstonia eutropha* DSM 531 (hereinafter referred to as ReTHL gene; DDBJ ID=J04987; amino acid sequence: SEQ ID NO: 21; nucleotide sequence: SEQ ID NO: 22), two PCR primers (ReTHL-A3 and ReTHL-T3) were designed. The nucleotide sequences of the designed sense and antisense primers are shown below:
ReTHL-A3 (SEQ ID NO: 27)
   gacggtacctatatATGACTGATGTTGTCATCGTATCC
ReTHL-T3 (SEQ ID NO: 28)
   cacaagcttaTTATTTACGTTCAACTGCCAGCGC

The ReTHL gene was cloned using the two PCR primers and the genomic DNA of *Ralstonia eutropha* DSM 531 as a template. Specifically, a 50 µL solution containing the two PCR primers, the genomic DNA, 0.2 mM dNTP, and 2.5 U PfuUltra in PfuUltra reaction buffer was prepared, and subjected to 30 cycles of 95°C for 30 seconds; 55°C for 30 seconds; and 72°C for 1 minute 10 seconds. As a result, a DNA fragment of about 1.2 kb was amplified.

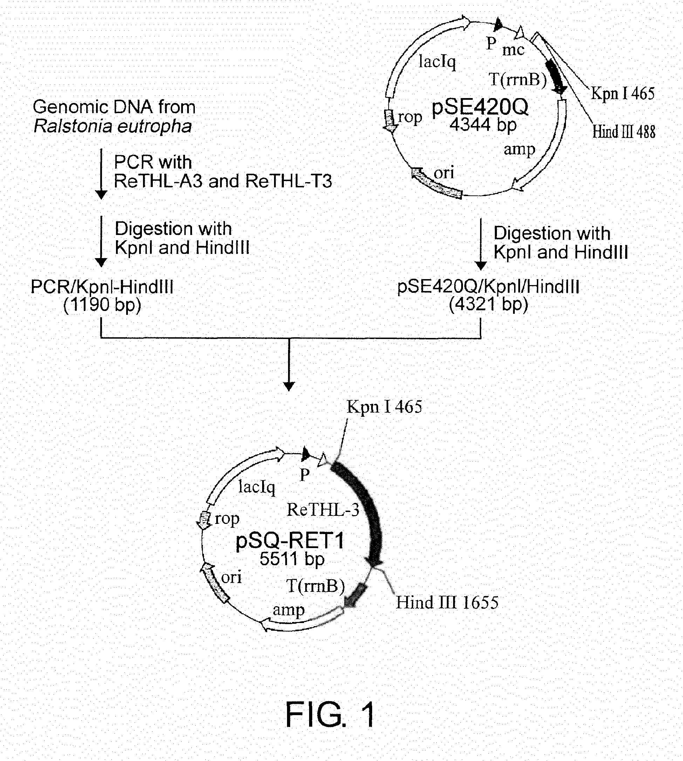

A plasmid containing the whole ReTHL gene was prepared as shown in Fig. 1. Specifically, the DNA fragment obtained by PCR was double-digested with KpnI and HindIII, and ligated with Kpnl-HindIII-treated pSE420Q vector (WO 2006-132145) to prepare pSQ-RET1, an expression plasmid for the ReTHL gene.

### [Example 2] Expression of ReTHL gene in E. coli

The plasmid pSQ-RET1 prepared in Example 1 was introduced into *E. coli* JM109 by the Hanahan method to obtain a transformant *E.coli* JM109 (pSQ-RET1). This transformant was cultured by the following method:

The transformant was inoculated into a 21 mm diameter test tube containing 7 ml of LB medium consisting of 10 g/L tryptone, 5 g/L yeast extract, and 10 g/L NaCl, which had been adjusted to pH 7.2. The cells were cultured at 30.0°C for 18 hours while stirring at 250 rpm under aerobic conditions. IPTG was then added at a final concentration of 0.1 mM and incubated at 30.0°C for 4 hours while stirring at 250 rpm under aerobic conditions to induce the expression of the introduced gene.

The resulting culture was dispensed into a 2 ml Eppendorf tube, and centrifuged to collect the cells. Then, 50 mM potassium phosphate buffer (pH 7.0) containing 1 mM DTT was added to the cells, and subjected to ultrasonication to disrupt the cells. Intact cells and cell debris were removed by centrifugation to obtain a cell-free extract.

The ReTHL activity of the obtained cell-free extract was measured by THL activity assay-1, and determined to be 47.1 U/mg.

### [Example 3] Cloning of β-ketothiolase gene derived from Zoogloea ramigera

Genomic DNA was obtained from *Zoogloea ramigera* DSM 287 in the same manner as in Example 1.

In order to clone the phbA gene present in the genomic DNA (hereinafter referred to as ZrTHL gene; DDBJ ID=J02631; amino acid sequence: SEQ ID NO: 23; nucleotide sequence: SEQ ID NO: 24), six PCR primers (ZrTHL-A2, ZrTHL-T2, ZrTHL-Nco-F1, ZrTHL-Nco-F2, ZrTHL-Nco-R1, and ZrTHL-Nco-R2) were designed. The nucleotide sequences of the designed sense and antisense primers are shown below:
ZrTHL-A2 (SEQ ID NO: 29)
   gacggtacctatatATGAGTACTCCATCAATCGTC
ZrTHL-T2 (SEQ ID NO: 30)
   cacaagcttaTTAAAGACTTTCGATGCACATCGC
ZrTHL-Nco-F1 (SEQ ID NO: 31)
   GGAATCCATGTCAATGGCCCCG
ZrTHL-Nco-F2 (SEQ ID NO: 32)
   GCTCGATTCAATGGCGAAGC
ZrTHL-Nco-R1 (SEQ ID NO: 33)
   CAATGCGGGGCCATTGACATGG
ZrTHL-Nco-R2 (SEQ ID NO: 34)
   CGGAGCTTCGCCATTGAATCGAG

DNA fragments were amplified using the genomic DNA of *Zoogloea ramigera* DSM 287 as a template. Specifically, a 50 µL solution containing two PCR primers (ZrTHL-A2 and ZrTHL-Nco-R1), the genomic DNA, 0.2 mM dNTP, and 2.5 U PfuUltra in PfuUltra reaction buffer was prepared, and subjected to 30 cycles of 95°C for 30 seconds; 55°C for 30 seconds; and 72°C for 30 seconds. As a result, DNA fragment 1 of about 400 bp was amplified. Another PCR was carried out using two primers (ZrTHL-Nco-F1 and ZrTHL-Nco-R2) in the same manner to amplify DNA fragment 2 of about 300 bp. Another PCR was carried out using two primers (ZrTHL-Nco-F2 and ZrTHL-T2) in the same manner to amplify DNA fragment 3 of about 500 bp.

These three DNA fragments were used as templates to construct the entire ORF of the ZrTHL gene. Specifically, a 50 µL solution containing the three DNA fragments, two PCR primers (ZrTHL-A2 and ZrTHL-T2), 0.2 mM dNTP, and 3.0 U PfuUltra in PfuUltra reaction buffer was prepared, and subjected to 30 cycles of 95°C for 30 seconds; 55°C for 30 seconds; and 72°C for 1 minute 20 seconds. As a result, a DNA fragment of about 1.2 kb was amplified.

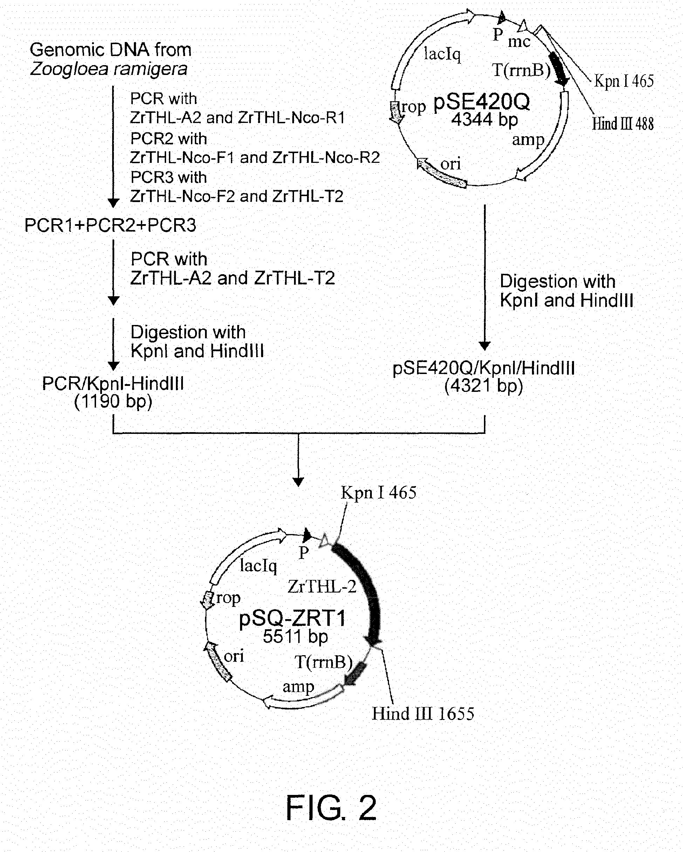

A plasmid containing the ZrTHL gene was prepared as shown in Fig. 2. Specifically, the DNA fragment obtained by PCR was double-digested with KpnI and HindIII, and ligated with KpnI-HindIII-treated pSE420Q vector (WO 2006-132145) to prepare pSQ-ZRT1, an expression plasmid for the ZrTHL gene.

### [Example 4] Expression of ZrTHL gene in E. coli

The plasmid pSQ-ZRT1 prepared in Example 3 was introduced into *E.coli* JM109 by the Hanahan method to obtain a transformant *E.coli* JM109 (pSQ-ZRT1). This transformant was cultured by the method described in Example 2.

The culture obtained by the above-described method was dispensed into a 2 ml Eppendorf tube, and centrifuged to collect the cells. Then, 50 mM potassium phosphate buffer (pH 7.0) containing 1 mM DTT was added to the cells, and subjected to ultrasonication to disrupt the cells. Intact cells and cell debris were removed by centrifugation to obtain a cell-free extract.

The ZrTHL activity of the obtained cell-free extract was measured by THL activity assay-1, and determined to be 31.8 U/mg.

### [Example 5] Cloning of β-ketothiolase derived from Escherichia coli

Genomic DNA was obtained from *Escherichia coli* by the method described in Example 1.

In order to clone the atoB gene present in the obtained genomic DNA (hereinafter referred to as EcTHL gene; DDBJ ID= AP009048; amino acid sequence: SEQ ID NO: 25; nucleotide sequence: SEQ ID NO: 26), two PCR primers (EcTHL-A1 and EcTHL-T1) were designed. The nucleotide sequences of the designed sense and antisense primers are shown below:
EcTHL-A1 (SEQ ID NO: 35)
   gacggtacctatatATGAAAAATTGTGTCATCGTCAG
EcTHL-T1 (SEQ ID NO: 36)
   cacaagcttaTTAATTCAAGCGTTCAATCACCATC

The EcTHL gene was cloned using the two PCR primers and the genomic DNA of *E. coli* JM109 as a template. Specifically, a 50 µL solution containing the two PCR primers (EcTHL-A1 and EcTHL-T1), the genomic DNA, 0.2 mM dNTP, and 2.5 U PfuUltra in PfuUltra reaction buffer was prepared, and subjected to 30 cycles of 95°C for 30 seconds; 50°C for 30 seconds; and 72°C for 1 minute 20 seconds. As a result, a DNA fragment of about 1.2 kb was amplified.

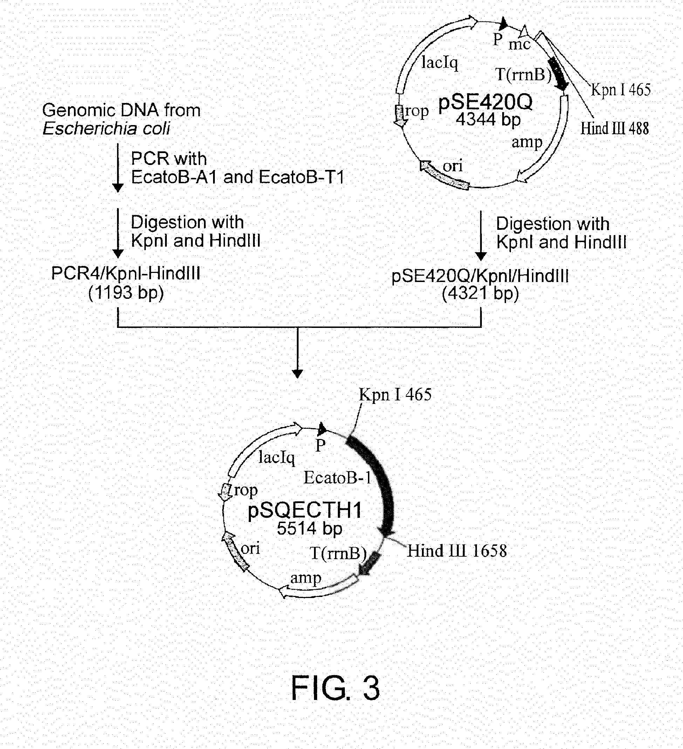

A plasmid containing the EcTHL gene was prepared as shown in Fig. 3. Specifically, the DNA fragment obtained by PCR was double-digested with KpnI and HindIII, and ligated with KpnI-HindIII-treated pSE420Q vector (WO 2006-132145) to prepare pSQECTH1, an expression plasmid for the EcTHL gene.

### [Example 6] Expression of EcTHL gene in E. coli

The plasmid pSQECTH1 prepared in Example 5 was introduced into *E.coli* JM109 by the Hanahan method to obtain a transformant *E. coli* JM109 (pSQECTH1). This transformant was cultured by the method described in Example 2.

The culture obtained by the above-described method was dispensed into a 2 ml Eppendorf tube, and centrifuged to collect the cells. Then, 50 mM potassium phosphate buffer (pH 7.0) containing 1 mM DTT was added to the cells, and subjected to ultrasonication to disrupt the cells. Intact cells and cell debris were removed by centrifugation to obtain a cell-free extract.

The EcTHL activity of the obtained cell-free extract was measured by THL activity assay-2, and determined to be 5.61 U/mg.

### [Example 7] Cloning of phbB gene derived from Ralstonia eutropha

In order to clone the phbB gene present in the genomic DNA of *Ralstonia eutropha* DSM 531 prepared in Example 1 (hereinafter referred to as ReAR1 gene; DDBJ ID= J04987; amino acid sequence: SEQ ID NO: 9; nucleotide sequence: SEQ ID NO: 10), four PCR primers (ReAR1-A3, ReAR-T3, ReAR-Nco-F1, and ReAR-Nco-R1) were designed. The nucleotide sequences of the designed sense and antisense primers are shown below:
ReAR-A3 (SEQ ID NO: 37)
   gaggaattcatatATGACTCAACGTATTGCGTATGTG
ReAR-T3 (SEQ ID NO: 38)
   cagactagtaTTAGCCCATGTGCAGGCCG
ReAR-Nco-F1 (SEQ ID NO: 39)
   CATGGCTTCACTATGGCACTGGC
ReAR-Nco-Rl (SEQ ID NO: 40)
   GCCAGTGCCATAGTGAAGCCATG

DNA fragments were amplified using the genomic DNA of *Ralstonia eutropha* DSM 531 as a template. Specifically, a 50 µL solution containing two PCR primers (ReAR-A3 and ReAR-Nco-R1), the genomic DNA, 0.2 mM dNTP, and 2.5 U PfuUltra in PfuUltra reaction buffer was prepared, and subjected to 30 cycles of 95°C for 30 seconds; 55°C for 30 seconds; and 72°C for 40 seconds. As a result, DNA fragment 1 of about 500 bp was amplified. Another PCR was carried out using two primers (ReAR-Nco-F1 and ReAR-T3) to amplify DNA fragment 2 of about 300 bp.

These two DNA fragments were used as templates to construct the entire ORF of the ZrTHL gene. Specifically, a 50 µL solution containing the two DNA fragments, two PCR primers (ReAR-A3 and ReAR-T3), 0.2 mM dNTP, and 3.0 U PfuUltra in PfuUltra reaction buffer was prepared, and subjected to 30 cycles of 95°C for 30 seconds; 55°C for 30 seconds; and 72°C for 1 minute. As a result, a DNA fragment of about 800 bp was amplified.

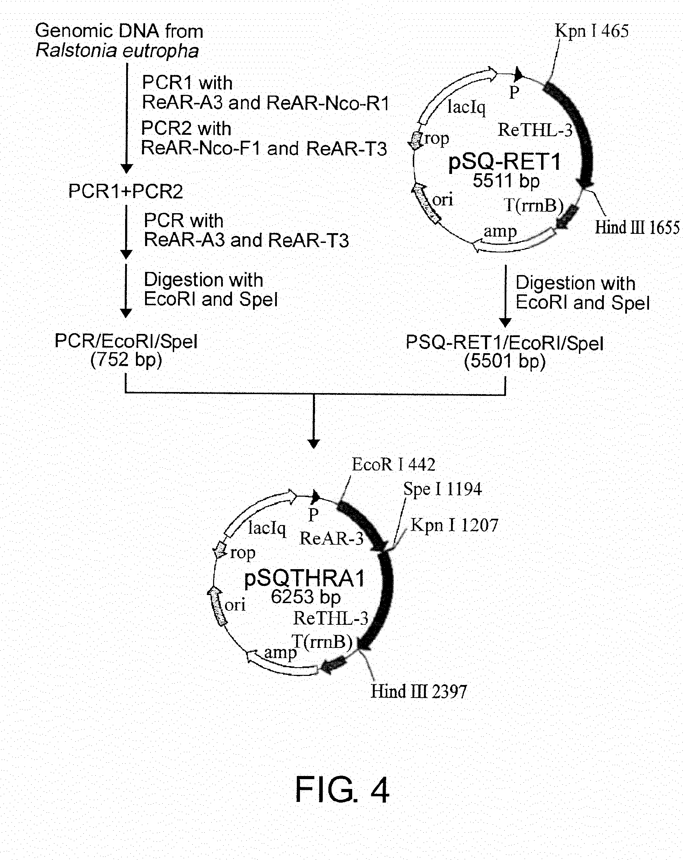

A plasmid containing the ReAR1 gene was prepared as shown in Fig. 4. Specifically, the DNA fragment obtained by PCR was double-digested with EcoRI and SpeI, and ligated with the pSQ-RET1 vector prepared in Example 1 after treating it with EcoRI and SpeI, to prepare pSQTHRA1, a coexpression plasmid carrying the ReTHL gene and ReAR1 gene.

### [Example 8] Expression of ReTHL gene and ReAR1 gene in E. coli

The plasmid pSQTHRA1 prepared in Example 7 was introduced into *E.coli* JM109 by the Hanahan method to obtain a transformant *E.coli* JM109 (pSQTHRA1). This transformant was cultured by the method described in Example 2.

The obtained culture was dispensed into a 2 ml Eppendorf tube, and centrifuged to collect the cells. Then, 50 mM potassium phosphate buffer (pH 8.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1 and for ReAR1 activity by the 3HBD activity assay, and the results were 41.3 U/mg and 6.12 U/mg, respectively.

### [Example 9] Cloning of phbB gene derived from Zoogloea ramigera

In order to clone the phbB gene present in the genomic DNA of *Zoogloea ramigera* DSM 287 prepared in Example 3 (hereinafter referred to as ZrAR1 gene; WO 9100917; amino acid sequence: SEQ ID NO: 11; nucleotide sequence: SEQ ID NO: 12), two PCR primers (ZrAR-A2 and ZrAR-T2) were designed. The nucleotide sequences of the designed sense and antisense primers are shown below:
ZrAR-A2 (SEQ ID NO: 41)
   gaggaattcatatATGAGTCGTGTAGCATTGGTAAC
ZrAR-T2 (SEQ ID NO: 42)
   cagactagtaTTAGACGAAGAACTGGCCG

The ZrAR1 gene was cloned using the two PCR primers and the genomic DNA of *Zoogloea ramigera* as a template. Specifically, a 50 µL solution containing the two PCR primers (ZrAR-A2 and ZrAR-T2), the genomic DNA, 0.2 mM dNTP, and 2.5 U PfuUltra in PfuUltra reaction buffer was prepared, and subjected to 30 cycles of 95°C for 30 seconds; 53°C for 30 seconds; and 72°C for 40 seconds. As a result, a DNA fragment of about 700 bp was amplified.

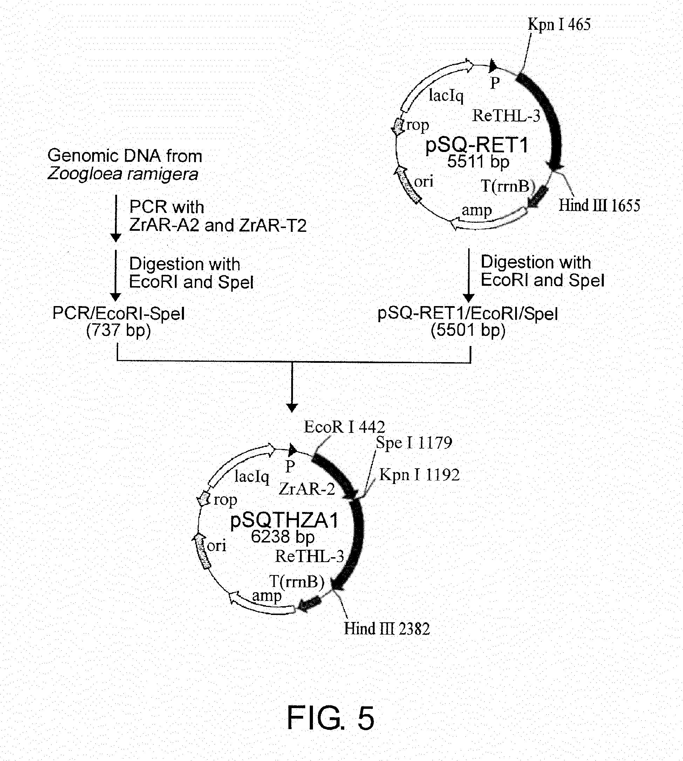

A plasmid containing the ZrAR1 gene was prepared as shown in Fig. 5. Specifically, the DNA fragment obtained by PCR was double-digested with EcoRI and SpeI, and ligated with the pSQ-RET1 vector prepared in Example 1 after treating it with EcoRI and SpeI, to prepare pSQTHZA1, a coexpression plasmid carrying the ReTHL gene and ZrAR1 gene.

### [Example 10] Expression of ReTHL gene and ZrAR1 gene in E. coli

The plasmid pSQTHZA1 prepared in Example 9 was introduced into *E. coli* JM109 by the Hanahan method to obtain a transformant *E.coli* JM109 (pSQTHZA1). This transformant was cultured by the method described in Example 2.

The obtained culture was dispensed into a 2 ml Eppendorf tube, and centrifuged to collect the cells. Then, 50 mM potassium phosphate buffer (pH 8.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1 and for ZrAR1 activity by the 3HBD activity assay, and the results were 72.9 U/mg and 0.582 U/mg, respectively.

### [Example 11] Cloning of actIII gene derived from Streptomyces violaceoruber

*Streptomyces violaceoruber* IFO 15146 was inoculated in 50 mL of liquid medium consisting of 4 g/L glucose, 4 g/L yeast extract, and 10 g/L malt extract (YM medium), which had been adjusted to pH 7.2. The cells were cultured with shaking at 28°C for 24 hours.

The cells were collected from the resulting culture by centrifugation, and genomic DNA was obtained from the cells. The genomic DNA was prepared using Genomic Tip-100/G Kit (QIAGEN).

In order to clone the actIII gene present in the obtained genomic DNA (hereinafter referred to as SvKR1 gene; DDBJ ID=M19536; amino acid sequence: SEQ ID NO: 15; nucleotide sequence: SEQ ID NO: 16), two PCR primers (SvKR-A4 and SvKR-T4) were designed. The nucleotide sequences of the designed sense and antisense primers are shown below:
SvKR-A4 (SEQ ID NO: 43)
   gaggaattcatatATGGCCACGCAGGACTCC
SvKR-T4 (SEQ ID NO: 44)
   cagactagtaTTAGTAGTTCCCCAGCCCG

The SvKR1 gene was cloned using the two PCR primers and the genomic DNA of *Streptomyces violaceoruber* as a template. Specifically, a 50 µL solution containing the two PCR primers (SvKR-A4 and SvKR-T4), the genomic DNA, 0.2 mM dNTP, and 2.5 U PfuUltra in PfuUltra reaction buffer was prepared, and subjected to 30 cycles of 95°C for 30 seconds; 55°C for 30 seconds; and 72°C for 1 minute. As a result, a DNA fragment of about 800 bp was amplified.

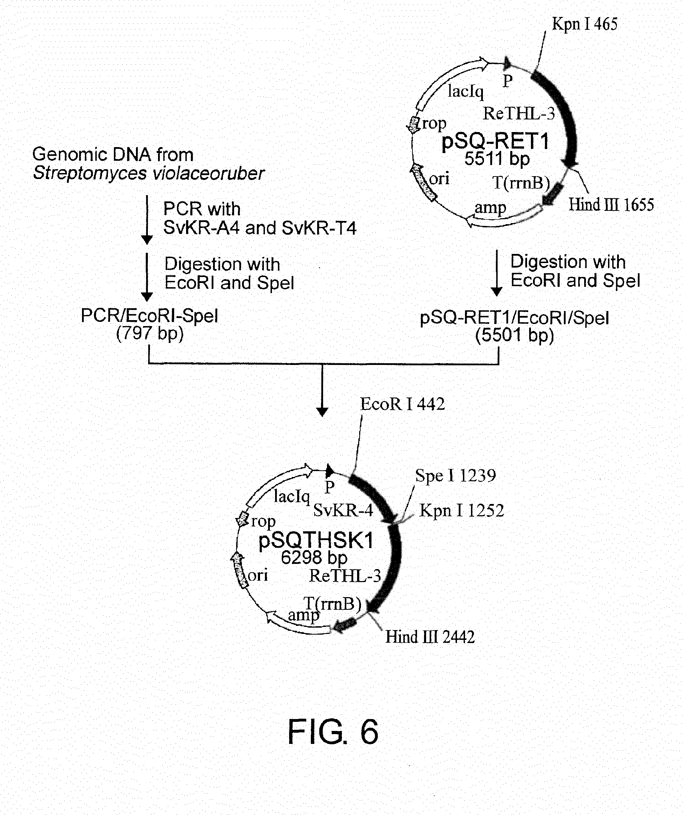

A plasmid containing the SvKR1 gene was prepared as shown in Fig. 6. Specifically, the DNA fragment obtained by PCR was double-digested with EcoRI and SpeI, and ligated with the pSQ-RET1 vector prepared in Example 1 after treating it with EcoRI and SpeI, to prepare pSQTHSK1, a coexpression plasmid carrying the ReTHL gene and the SvKR1 gene.

### [Example 12] Expression of ReTHL gene and SvKR1 gene in E. coli

The plasmid pSQTHSK1 prepared in Example 11 was introduced into *E. coli* JM109 by the Hanahan method to obtain a transformant *E. coli* JM109 (pSQTHSK1). This transformant was cultured by the method described in Example 2.

The obtained culture was dispensed into a 2 ml Eppendorf tube, and centrifuged to collect the cells. Then, 50 mM potassium phosphate buffer (pH 8.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1 and for SvKR1 activity by the 3HBD activity assay, and the results were 7.71 U/mg and 0.0547 U/mg, respectively.

### [Example 13] Cloning of β-ketoacyl-ACP reductase gene derived from Geobacillus stearothermophilus

*Geobacillus stearothermophilus* NBRC 12550 was inoculated in 50 mL of liquid medium consisting of 10 g/L polypeptone, 2 g/L yeast extract, and 1 g/L MgSO₄·H₂O, which had been adjusted to pH 7.0. The cells were cultured with shaking at 50°C for 21 hours.

The cells were collected from the resulting culture by centrifugation, and genomic DNA was obtained from the cells. The genomic DNA was prepared using Genomic Tip-100/G Kit (QIAGEN).

In order to clone the β-ketoacyl-ACP reductase gene present in the obtained genomic DNA (hereinafter referred to as BstKR1 gene; JP-A (Kokai) 2002-209592; amino acid sequence: SEQ ID NO: 13; nucleotide sequence: SEQ ID NO: 14), two PCR primers (BstKR-A3 and BstKR-T3) were designed. The nucleotide sequences of the designed sense and antisense primers are shown below:
BstKR-A3 (SEQ ID NO: 45)
   gaggaattcatatATGTCTCAACGTTTTGCAGGTC
BstKR-T3 (SEQ ID NO: 46)
   cagactagtaTTAACATTTTGGACCACCTGC

The BstKR1 gene was cloned using the two PCR primers and the genomic DNA of *Geobacillus stearothermophilus* as a template. Specifically, a 50 µL solution containing the two PCR primers (BstKR-A3 and BstKR-T3), the genomic DNA, 0.2 mM dNTP, and 2.5 U PfuUltra in PfuUltra reaction buffer was prepared, and subjected to 30 cycles of 95°C for 30 seconds; 50°C for 30 seconds; and 72°C for 1 minute. As a result, a DNA fragment of about 800 bp was amplified.

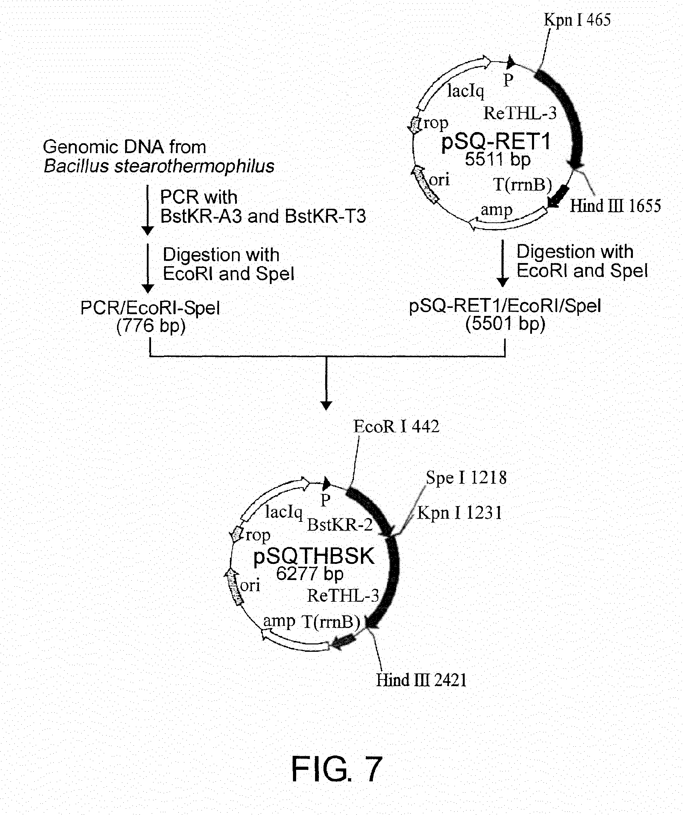

A plasmid containing the BstKR1 gene was prepared as shown in Fig. 7. Specifically, the DNA fragment obtained by PCR was double-digested with EcoRI and SpeI, and ligated with the pSQ-RET1 vector prepared in Example 1 after treating it with EcoRI and SpeI, to prepare pSQTHBSK, a coexpression plasmid carrying the ReTHL gene and the BstKR1 gene.

### [Example 14] Expression of ReTHL gene and BstKR1 gene in E. coli

The plasmid pSQTHBSK prepared in Example 13 was introduced into *E. coli* JM109 by the Hanahan method to obtain a transformant E. *coli* JM109 (pSQTHBSK). This transformant was cultured by the method described in Example 2.

The culture obtained by the above-mentioned method was dispensed into a 2 ml Eppendorf tube, and centrifuged to collect the cells. Then, 50 mM potassium phosphate buffer (pH 8.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1 and for BstKR1 activity by the 3HBD activity assay, and the results were 99.0 U/mg and 1.494 U/mg, respectively.

### [Example 15] Cloning of 3-hydroxybutyryl-CoA dehydrogenase gene derived from Clostridium acetobutylicum

In order to clone the 3-hydroxybutyryl-CoA dehydrogenase gene present in the genomic DNA purchased from ATCC (hereinafter referred to as CaHBD gene; DDBJ ID=AE001437; amino acid sequence: SEQ ID NO: 13; nucleotide sequence: SEQ ID NO: 14), two PCR primers (CaHBD-A1 and CaHBD-T1) were designed. The nucleotide sequences of the designed sense and antisense primers are shown below:
CaHBD-A1 (SEQ ID NO: 47)
   gaggaattcatatATGAAAAAGGTATGTGTTATAGGTGC
CaHBD-T1 (SEQ ID NO: 48)
   cagactagtaTTATTTTGAATAATCGTAGAAACCTTTTCC

The CaHBD gene was cloned using the two PCR primers and the genomic DNA of *Clostridium acetobutylicum* as a template. Specifically, a 50 µL solution containing the two PCR primers (CaHBD-A1 and CaHBD-T1), the genomic DNA, 0.2 mM dNTP, and 2.5 U PfuUltra in PfuUltra reaction buffer was prepared, and subjected to 30 cycles of 95°C for 30 seconds; 50°C for 30 seconds; and 72°C for 1 minute. As a result, a DNA fragment of about 900 bp was amplified.

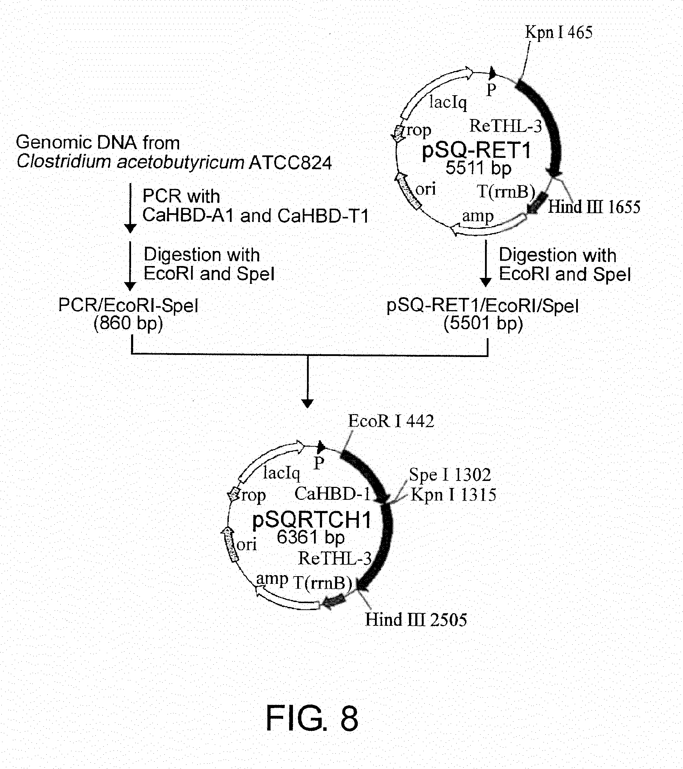

A plasmid containing the CaHBD gene was prepared as shown in Fig. 8. Specifically, the DNA fragment obtained by PCR was double-digested with EcoRI and SpeI, and ligated with the pSQ-RET1 vector prepared in Example 1 after treating it with EcoRI and SpeI, to prepare pSQRTCH1, a coexpression plasmid carrying the ReTHL gene and the CaHBD gene.

### [Example 16] Expression of ReTHL gene and CaHBD gene in E. coli

The plasmid pSQRTCH1 prepared in Example 15 was introduced into *E. coli* JM109 by the Hanahan method to obtain a transformant *E. coli* JM109 (pSQRTCH1). This transformant was cultured by the method described in Example 2.

The culture obtained by the above-mentioned method was dispensed into a 2 ml Eppendorf tube, and centrifuged to collect the cells. Then, 50 mM potassium phosphate buffer (pH 8.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1 and for ReAR1 activity by the 3HBD activity assay, and the results were 13.8 U/mg and 63.2 U/mg, respectively.

### [Example 17] Cloning of (R)-2-octanol dehydrogenase gene derived from Pichia finlandica

*Pichia finlandica* DSM 70280 was inoculated in 50 mL of liquid medium consisting of 10 g/L glucose, 5 g/L peptone (derived from soybean), 3 g/L yeast extract, and 3 g/L malt extract, which had been adjusted to pH 7.0. The cells were cultured with shaking at 25°C for 24 hours.

The cells were collected from the resulting culture by centrifugation, and genomic DNA was obtained from the cells. The genomic DNA was prepared using Genomic Tip-100/G Kit (QIAGEN).

In order to clone the (R)-2-octanol dehydrogenase gene present in the obtained genomic DNA (hereinafter referred to as PfODH gene; DDBJ ID=AB259114; amino acid sequence: SEQ ID NO: 17; nucleotide sequence: SEQ ID NO: 18), six PCR primers (PfODH-A3, PfODH-T3, PfODH-Xba-F1, PfODH-Xba-R1, PfODH-Hind-F1, and PfOHD-Hind-R1) were designed. The nucleotide sequences of the designed sense and antisense primers are shown below:
PfODH-A3 (SEQ ID NO: 49)
   gaggAATTCTAAAATGTCTTATAATTTCCATAACAAGGTTGC
PfODH-T3 (SEQ ID NO: 50)
   tcgACTAGTATTATTGTGCTGTGTACCCACCGTCAACC
PfODH-Xba-F1 (SEQ ID NO: 51)
   GGCTCTGGAGTACGCATCTCATGGTATTCGTGTAAATTC
PfODH-Xba-R1 (SEQ ID NO: 52)
   GAATTTACACGAATACCATGAGATGCGTACTCCAGAGCC
PfODH-Hind-F1 (SEQ ID NO: 53)
   GTAAGCCTGCACCCTATTGGGCGTCTGGGTCGTC
PfODH-Hind-R1 (SEQ ID NO: 54)
   GACGACCCAGACGCCCAATAGGGTGCAGGCTTAC

DNA fragments were amplified using the genomic DNA of *Pichia finlandica* DSM 70280 as a template. Specifically, a 50 µL solution containing two PCR primers (PfODH-A3 and PfODH-Xba-R1), the genomic DNA, 0.2 mM dNTP, and 2.5 U PfuUltra in PfuUltra reaction buffer was prepared, and subjected to 30 cycles of 95°C for 30 seconds; 55°C for 30 seconds; and 72°C for 30 seconds. As a result, DNA fragment 1 of about 500 bp was amplified. Another PCR was performed using two PCR primers (PfODH-Xba-F1 and PfODH-Hind-R1) to amplify DNA fragment 2. Another PCR was performed using two PCR primers (PfODH-Hind-F1 and PfODH-T3) to amplify DNA fragment 3 of about 200 bp.

These three DNA fragments were used to construct the entire ORF of the PfODH gene. Specifically, a 50 µL solution containing the three DNA fragments, two PCR primers (PfODH-A3 and PfODH-T3), 0.2 mM dNTP, and 3.0 U PfuUltra in PfuUltra reaction buffer was prepared, and subjected to 30 cycles of 95°C for 30 seconds; 55°C for 30 seconds; and 72°C for 1 minute. As a result, a DNA fragment of about 800 bp was amplified.

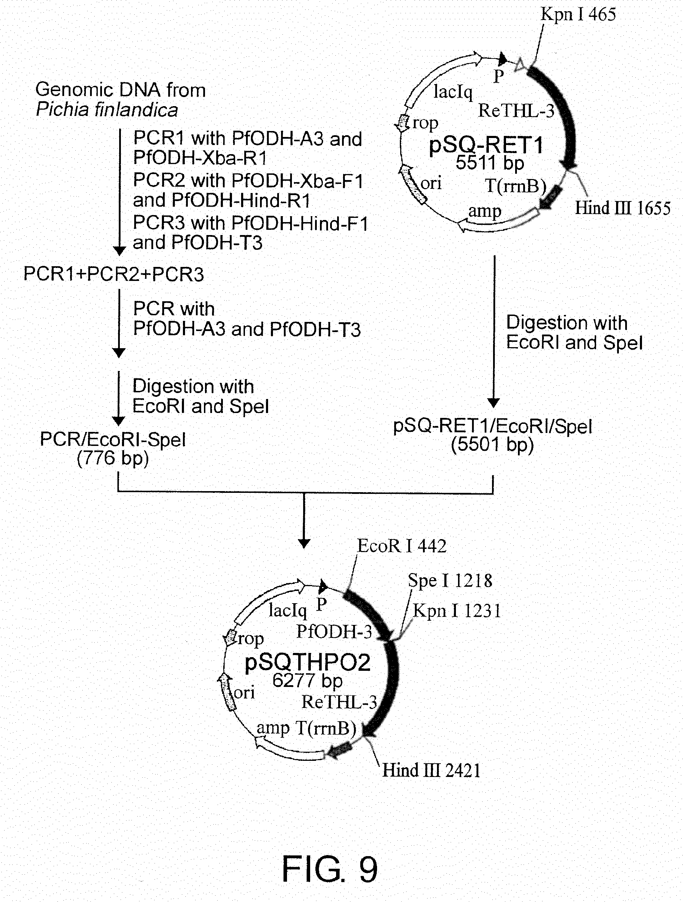

A plasmid containing the PfODH gene was prepared as shown in Fig. 9. Specifically, the DNA fragment obtained by PCR was double-digested with EcoRI and SpeI, and ligated with the pSQ-RET1 vector prepared in Example 1 after treating it with EcoRI and SpeI, to prepare pSQTHPO2, a coexpression plasmid carrying the ReTHL gene and the PfODH gene.

### [Example 18] Expression of ReTHL gene and PfODH gene in E. coli

The plasmid pSQTHPO2 prepared in Example 17 was introduced into *E. coli* JM109 by the Hanahan method to obtain a transformant *E. coli* JM109 (pSQTHPO2). This transformant was cultured by the method described in Example 2.

The culture obtained by the above-mentioned method was dispensed into a 2 ml Eppendorf tube, and centrifuged to collect the cells. Then, 50 mM potassium phosphate buffer (pH 8.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1 and for PfODH activity by the 3HBD activity assay, and the results were 74.9 U/mg and 0.0120 U/mg, respectively.

### [Example 19] Production of 3-hydroxybutyryl-CoA in enzymatic reaction using ReTHL-CaHBD

To a solution containing 100 mM Tris-HCl buffer (pH 7.4), 2.5 mM NADH, and 2.5 mM acetyl-CoA, 6.2 µL of the cell-free extract containing ReTHL and CaHBD prepared according to Example 14 was added, and reacted at 30°C for 1 hour. The reaction was terminated by adding 5 µL of 60%(v/v) perchloric acid and 5 µL of 5N aqueous sodium hydroxide solution to the reaction solution. To this reacted solution, 510 µL of 200 mM potassium phosphate buffer was added, and centrifuged to obtain a supernatant. HPLC analysis of the supernatant showed that 0.27 mM 3-hydroxybutyryl-CoA was produced.

The HPLC conditions were as follows:
- HPLC column: Wakosil-II 5C18HG (4.6mm x 150mm) manufactured by Wako Pure Chemical Industries, Ltd.
- Eluent: 50 mM phosphate buffer (pH 5.0) : acetonitrile = 95 : 5
- Column temperature: 30°C
- Flow rate: 1.0 mL/min
- Detection: UV absorption at 254 nm

Under the above conditions, 3-hydroxybutyryl-CoA was eluted at 15.3 min. The accumulated concentration was determined based on a calibration curve obtained using 3-hydroxybutyryl-CoA (Sigma).

### [Example 20] Cloning of aldehyde/alcohol dehydrogenase gene derived from Clostridium acetobutylicum

In order to clone the aldehyde/alcohol dehydrogenase gene present in the megaplasmid obtained in Example 15 (hereinafter referred to as CaAdhE gene; DDBJ ID=AE001438; amino acid sequence: SEQ ID NO: 1; nucleotide sequence: SEQ ID NO: 2), four PCR primers (CaAdhE2-A1, CaAdhE2-Tl, CaAdhE2-Nde-F1, and CaAdhE2-Nde-R1) were designed. The nucleotide sequences of the designed sense and antisense primers are shown below:
CaAdhE2-A1 (SEQ ID NO: 55)
   gaccatATGAAAGTTACAAATCAAAAAGAACTAAAAC
CaAdhE2-T1 (SEQ ID NO: 56)
   ctgttaaTTAAAATGATTTTATATAGATATCCTTAAGTTC
CaAdhE2-Nde-F1 (SEQ ID NO: 57)
   CTATAGAAGCATACGTTTCGG
CaAdhE2-Nde-R1 (SEQ ID NO: 58)
   CCGAAACGTATGCTTCTATAG

DNA fragments were amplified using the megaplasmid DNA of *Clostridium acetobutylicum* ATCC 824 as a template. Specifically, a 50 µL solution containing two PCR primers (CaAdhE2-A1 and CaAdhE2-Nde-R1), the genomic DNA, 0.2 mM dNTP, and 2.5 U PfuUltra in PfuUltra reaction buffer was prepared, and subjected to 30 cycles of 95°C for 30 seconds; 50°C for 30 seconds; and 72°C for 2 minute. As a result, DNA fragment 1 of about 2 kb was amplified. Another PCR was performed using two PCR primers (CaAdhE2-Nde-F1 and CaAdhE2-T1) to amplify DNA fragment 2 of about 600 bp.

These two DNA fragments were used to construct the entire ORF of the CaAdhE gene. Specifically, a 50 µL solution containing the two DNA fragments, two PCR primers (CaAdhE2-A1 and CaAdhE2-T1), 0.2 mM dNTP, and 3.0 U PfuUltra in PfuUltra reaction buffer was prepared, and subjected to 30 cycles of 95°C for 30 seconds; 50°C for 30 seconds; and 72°C for 2 minute 30 seconds. As a result, a DNA fragment of about 2.6 kb was amplified.

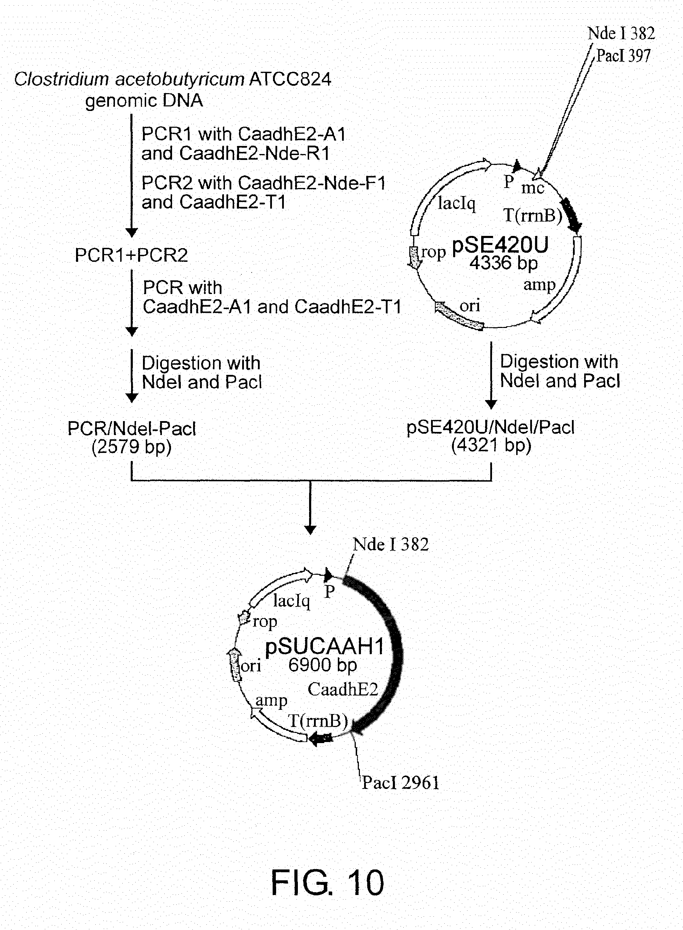

A plasmid containing the CaAdhE gene was prepared as shown in Fig. 10. Specifically, the DNA fragment obtained by PCR was double-digested with NdeI and PacI, and ligated with NdeI-PacI-treated pSE420U vector (WO 2006-132145) to prepare pSUCAAH1, an expression plasmid carrying the CaAdhE gene.

### [Example 21] Expression of CaAdhE gene in E. coli

The plasmid pSUCAAH1 prepared in Example 20 was introduced into *E. coli* JM109 by the Hanahan method to obtain a transformant *E. coli* JM109 (pSUCAAH1). This transformant was cultured by the method described in Example 2.

The culture obtained by the above-mentioned method was dispensed into a 2 ml Eppendorf tube, and centrifuged to collect the cells. Under anaerobic conditions, 50 mM MOPS buffer (pH 7.0) containing 1 mM DTT was added to the cells, and subjected to ultrasonication to disrupt the cells. Intact cells and cell debris were removed by centrifugation to obtain a cell-free extract.

The BCDH activity of the obtained cell-free extract was measured by the BCDH activity assay using butyryl-CoA as a substrate, and determined to be 0.129 U/mg.

The CaAdhE gene is known to act on acetyl-CoA and butyryl-CoA. However, its activity on 3-hydroxybutyryl-CoA has never been reported and is therefore unknown. Thus, the BCDH activity was measured using 3-hydroxybutyryl-CoA as a substrate. As a result, the activity was 0.0237 U/mg, which was found to be 18% relative to the activity on butyryl-CoA

### [Example 22] Production of 1,3-BG in enzymatic reaction using CaAdhE

To a solution containing 50 mM MES buffer (pH 6.0), 36 mM NADH, and 15 mM 3-hydroxybutyryl-CoA, 0.0938 U of the cell-free extract containing the CaAdhE enzyme prepared according to Example 21 was added, and reacted at 37°C for 24 hours. The reaction solution was centrifuged to remove precipitate, and the supernatant was analyzed by HPLC. The result showed that 3.66 mM 1,3-BG was produced.

The HPLC conditions were as follows:
- HPLC column: ULTRON PS-80H (8.0mm x 300mm) manufactured by Shinwa Chemical Industries Ltd.
- Eluent: 10 mM aqueous sulfuric acid solution
- Column temperature: 40°C
- Flow rate: 0.7 mL/min
- Detection: RI (refractive index detector)

Under the above conditions, 1,3-BG was eluted at 20.1 min. The accumulated concentration was determined based on a calibration curve obtained using 1,3-BG (Wako).

### [Example 23] Cloning of aldehyde/alcohol dehydrogenase gene derived from Thermoanaerobacter pseudethanolicus

In order to clone the aldehyde/alcohol dehydrogenase gene present in the genomic DNA purchased from DSM (hereinafter referred to as TpAdhE gene; amino acid sequence: SEQ ID NO: 65; nucleotide sequence: SEQ ID NO: 66), two PCR primers (TpadhE-A1 and TpAdhE-T1) were designed. The nucleotide sequences of the designed sense and antisense primers are shown below:
TpAdhE-A1 (SEQ ID NO: 69)
   gaccatATGCCTAACTTATTACAAGAACGCCGCGAAGTAAAAGA
TpAdhE-T1 (SEQ ID NO: 70)
   gctgttaattaaTTATTCTCCATAGGCTTTGCGATATATTTCTGC

PCR amplification of the TpAdhE gene was performed using the genomic DNA of *Thermoanaerobacter pseudethanolicus* as a template. Specifically, a 50 µL solution containing two PCR primers (TpadhE-A1 and TpAdhE-T1), the genomic DNA, 0.2 mM dNTP, and 2.5 U PfuUltra in PfuUltra reaction buffer was prepared, and subjected to 30 cycles of 95°C for 30 seconds; 50°C for 30 seconds; and 72°C for 2 minutes. As a result, a DNA fragment of about 2.6 kb was amplified.

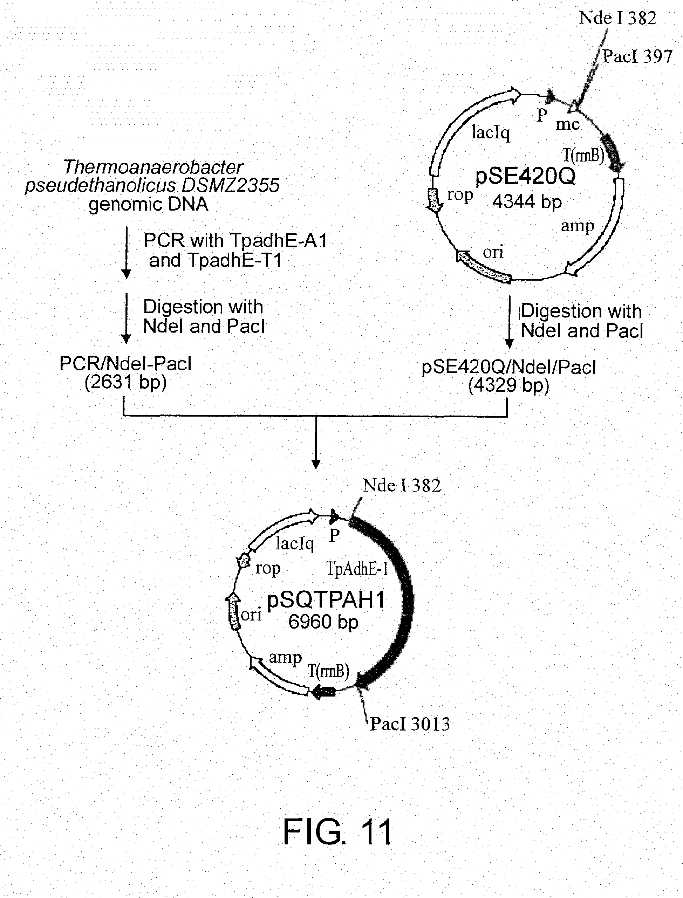

A plasmid containing the TpAdhE gene was prepared as shown in Fig. 11. Specifically, the DNA fragment obtained by PCR was double-digested with NdeI and PacI, and ligated with NdeI-PacI-treated pSE420Q (WO 2006-132145) to prepare pSQTPAH1, an expression plasmid carrying the TpAdhE gene.

### [Example 24] Expression of TpAdhE gene in E. coli

The plasmid pSQTPAH1 prepared in Example 23 was introduced into *E. coli* JM109 by the Hanahan method to obtain a transformant *E. coli* JM109 (pSQTPAH1). This transformant was cultured by the method described in Example 2.

The culture obtained by the above-mentioned method was dispensed into a 2 ml Eppendorftube, and centrifuged to collect the cells. Under anaerobic conditions, 50 mM MOPS buffer (pH 7.0) containing 1 mM DTT was added to the cells, and subjected to ultrasonication to disrupt the cells. Intact cells and cell debris were removed by centrifugation to obtain a cell-free extract.

The BCDH/BDH activity of TpAdhE in the obtained cell-free extract on 3-hydroxybutyryl-CoA was determined to be 0.00538 U/mg, and the BDH activity on prepared 3-hydroxybutylaldehyde was 0.0619 U/mg.

### [Example 25] Cloning of aldehyde/alcohol dehydrogenase gene derived from Propionibacterium freudenreichii subsp. freudenreichii

In order to clone the aldehyde/alcohol dehydrogenase gene present in the genomic DNA purchased from DSM (hereinafter referred to as PfALD gene; amino acid sequence: SEQ ID NO: 67; nucleotide sequence: SEQ ID NO: 68), two PCR primers (PfadhE-A1 and PfAdhE-T1) were designed. The nucleotide sequences of the designed sense and antisense primers are shown below:
PfAdhE-Al (SEQ ID NO: 71)
   gaccatATGGATTTCTCATTGACCGAAGACCAGCAG
PfAdhE-T1 (SEQ ID NO: 72)
   gctgttaaTTAACTACGGTAGTCGCGCAGTGCACC

PCR amplification of the PfALD gene was performed using the genomic DNA of *Propionibacterium freudenreichii* subsp. *freudenreichii* as a template. Specifically, a 50 µL solution containing two PCR primers (PfadhE-A1 and PfAdhE-T1), the genomic DNA, 0.2 mM dNTP, and 2.5 U PfuUltra in PfuUltra reaction buffer was prepared, and subjected to 30 cycles of 95°C for 30 seconds; 50°C for 30 seconds; and 72°C for 2 minutes. As a result, a DNA fragment of about 1.1 kb was amplified.

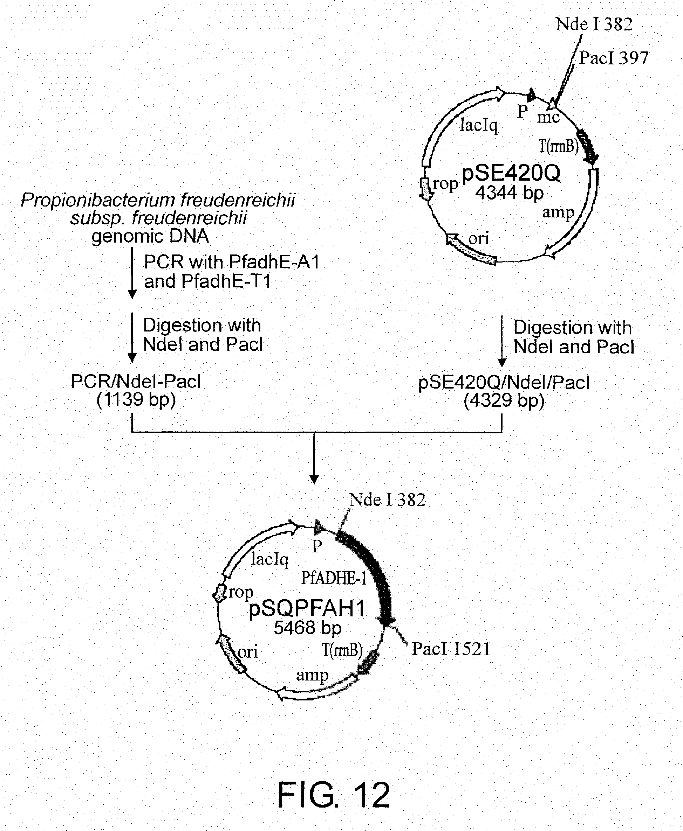

A plasmid containing the PfALD gene was prepared as shown in Fig. 12. Specifically, the DNA fragment obtained by PCR was double-digested with NdeI and PacI, and ligated with NdeI-PacI-treated pSE420Q (WO 2006-132145) to prepare pSQPFAH1, an expression plasmid carrying the PfALD gene.

### [Example 26] Expression of PfALD gene in E. coli

The plasmid pSQPFAH1 prepared in Example 25 was introduced into *E. coli* JM109 by the Hanahan method to obtain a transformant *E. coli* JM109 (pSQPFAH1). This transformant was cultured by the method described in Example 2.

The culture obtained by the above-mentioned method was dispensed into a 2 ml Eppendorf tube, and centrifuged to collect the cells. Under anaerobic conditions, 50 mM MOPS buffer (pH 7.0) containing 1 mM DTT was added to the cells, and subjected to ultrasonication to disrupt the cells. Intact cells and cell debris were removed by centrifugation to obtain a cell-free extract.

The BCDH activity of PfALD in the obtained cell-free extract on 3-hydroxybutyryl-CoA was determined to be 0.0826 U/mg.

### [Example 27] Plasmid construction for fermentative production of 1,3-BG

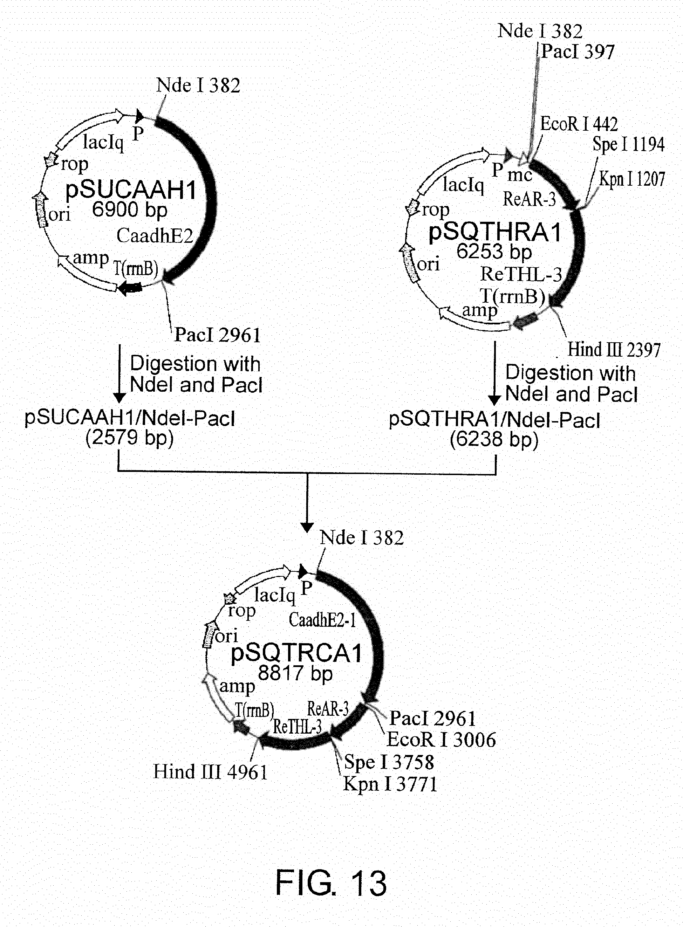

In order to construct a plasmid for fermentative production of 1,3-BG, the CaAdhE gene in the pSUCAAH1 plasmid produced in Example 20 was subcloned into pSQTHRA1 by the method shown in Fig. 13. Specifically, pSUCAAH1 was double-digested with NdeI and PacI, and ligated with NdeI-PacI-treated pSQTHRA1 vector to construct pSQTRCA1, a coexpression plasmid carrying the ReTHL gene, ReAR1 gene, and CaAdhE gene.

### [Example 28] Expression of ReTHL gene, ReAR1 gene, and CaAdhE gene in E. coli

The plasmid pSQTHRA1 prepared in Example 27 was introduced into *E. coli* JM109 by the Hanahan method to obtain a transformant *E. coli* JM109 (pSQTHRA1). This transformant was cultured by the method described in Example 2.

The culture obtained by the above-mentioned method was dispensed into a 2 ml Eppendorf tube, and centrifuged to collect the cells. Under anaerobic conditions, 50 mM MOPS buffer (pH 7.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1, for ReAR1 activity by the 3HBD activity assay, and for BCDH activity of CaAdhE by the BCDH activity assay using butyryl-CoA as a substrate, and the results were 6.46 U/mg, 0.952 U/mg, and 0.118 U/mg, respectively.

### [Example 29] Cloning of butanol dehydrogenase II gene derived from Clostridium acetobutylicum

In order to clone the bdhB gene from *C. acetobutylicum* (hereinafter referred to as CaBDHB gene; DDBJ ID=AE001437; amino acid sequence: SEQ ID NO: 3; nucleotide sequence: SEQ ID NO: 4), six PCR primers (CaBDHB-A2, CaBDHB-T2, CaBDHB-Nco-F1, CaBDHB-Nco-R1 CaBDHB-Xba-F1, and CaBDHB-Xba-R1) were designed. The nucleotide sequences of the designed sense and antisense primers are shown below:
CaBDHB-A2 (SEQ ID NO: 59)
   ggaccATGGTTGATTTCGAATATTCAATACCAACTAGAA
CaBDHB-T2 (SEQ ID NO: 60)
   cgatctagaaTTACACAGATTTTTTGAATATTTGTAGGACTTCGGAG
CaBDHB-Nco-F1 (SEQ ID NO: 61)
   GATGGAAATCCGTGGGATATTGTG
CaBDHB-Nco-R1 (SEQ ID NO: 62)
   CACAATATCCCACGGATTTCCATC
CaBDHB-Xba-F1 (SEQ ID NO: 63)
   GTTTACCATCTCGTCTGCGTGATGTTG
CaBDHB-Xba-R1 (SEQ ID NO: 64)
   CAACATCACGCAGACGAGATGGTAAAC

DNA fragments were amplified using the genomic DNA of *Clostridium acetobutylicum* as a template. Specifically, a 50 µL solution containing two PCR primers (CaBDHB-A2 and CaBDHB-Nco-R1), the genomic DNA, 0.2 mM dNTP, and 2.5 U PfuUltra in PfuUltra reaction buffer was prepared, and subjected to 30 cycles of 95°C for 30 seconds; 50°C for 30 seconds; and 72°C for 30 seconds. As a result, DNA fragment 1 of about 350 bp was amplified. Another PCR was performed using two PCR primers (CaBDHB-Nco-F1 and CaBDHB-Xba-R1) to amplify DNA fragment 2. Another PCR was performed using two PCR primers (CaBDHB-Xba-F1 and CaBDHB-T2) to amplify DNA fragment 3 of about 100 bp.

These three DNA fragments were used to construct the entire ORF of the CaBDHB gene. Specifically, a 50 µL solution containing the two DNA fragments, two PCR primers (CaBDHB-A2 and CaBDHB-T2), 0.2 mM dNTP, and 3.0 U PfuUltra in PfuUltra reaction buffer was prepared, and subjected to 30 cycles of 95°C for 30 seconds; 50°C for 30 seconds; and 72°C for 1 minute 30 seconds. As a result, a DNA fragment of about 1.2 kb was amplified.

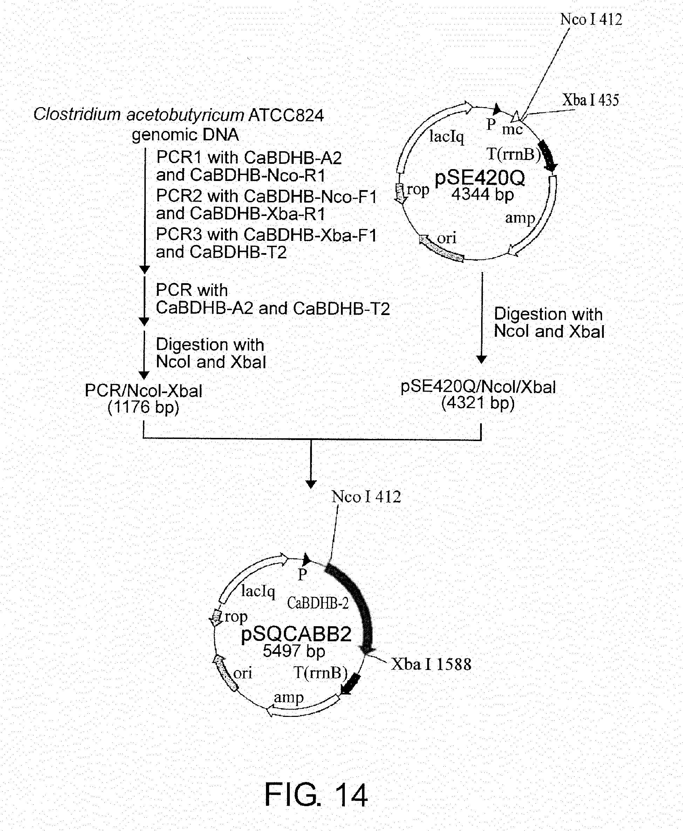

A plasmid containing the whole CaBDHB gene was prepared as shown in Fig. 14. Specifically, the DNA fragment obtained by PCR was double-digested with NcoI and XbaI, and ligated with NcoI-XbaI-treated pSE420Q vector (WO 2006-132145) to prepare pSQCABB2, an expression plasmid carrying the CaBDHB gene.

### [Example 30] Expression of CaBDHB gene in E. coli

The plasmid pSQCABB2 prepared in Example 29 was introduced into *E. coli* JM109 by the Hanahan method to obtain a transformant E. *coli* JM109 (pSQCABB2). This transformant was cultured by the method described in Example 2.

The culture obtained by the above-mentioned method was dispensed into a 2 ml Eppendorf tube, and centrifuged to collect the cells. Under anaerobic conditions, 50 mM MOPS buffer (pH 7.0) containing 1 mM DTT was added to the cells, and subjected to ultrasonication to disrupt the cells. Intact cells and cell debris were removed by centrifugation to obtain a cell-free extract.

The CaBDHB activity of the obtained cell-free extract was measured by the BDH activity assay using butylaldehyde as a substrate, and determined to be 0.0971 U/mg.

CaBDHB is known to act on acetaldehyde and butylaldehyde. However, its activity on 3-hydroxybutylaldehyde has never been reported and is therefore unknown. Thus, the CaBDHB activity was measured using prepared 3-hydroxybutylaldehyde (3-hydroxybutylaldehyde : acetaldehyde=3 : 1) as a substrate. As a result, the activity was 0.0462 U/mg, which was found to be 48% relative to the activity on butylaldehyde taken as 100%.

### [Example 31] Plasmid construction for fermentative production of 1,3-BG- 2

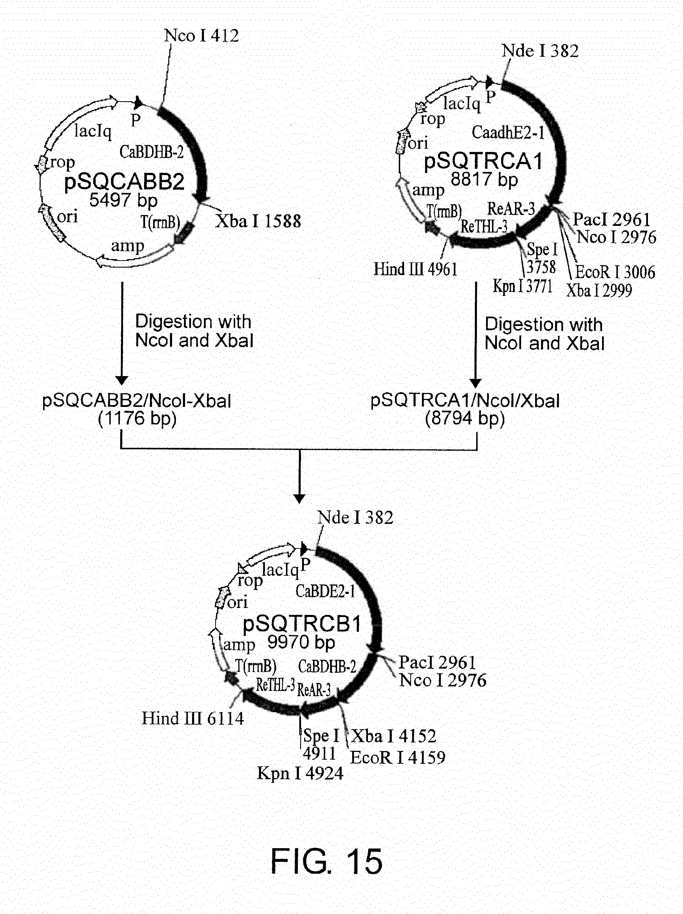

In order to construct plasmid 2 for fermentative production of 1,3-BG, the CaBDHB gene in the pSQCABB plasmid produced in Example 29 was subcloned into pSQTRCA1 by the method shown in Fig. 15. Specifically, pSQCABB2 was double-digested with NcoI and XbaI, and ligated with NcoI-XbaI-treated pSQTRCA1 vector to construct pSQTRCB1, a coexpression plasmid carrying the ReTHL gene, ReAR1 gene, CaAdhE gene, and CaBDHB gene.

### [Example 32] Expression of ReTHL gene, ReAR1 gene, CaAdhE gene, and CaBDHB gene in E. coli

The plasmid pSQTRCB1 prepared in Example 31 was introduced into *E. coli* W3110 by electroporation to obtain a transformant *E. coli* W3110 (pSQTRCB1). This transformant was cultured by the method described in Example 2.

The culture obtained by the above-mentioned method was dispensed into a 2 ml Eppendorf tube, and centrifuged to collect the cells. Under anaerobic conditions, 50 mM MOPS buffer (pH 7.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1, for ReAR1 activity by the 3HBD activity assay, for BCDH activity of CaAdhE by the BCDH activity assay (substrate: butyryl-CoA), and for CaBDHB activity by the BDH activity assay (substrate: butylaldehyde), and the results were 10.0 U/mg, 2.08 U/mg, 0.0298 U/mg, and 0.124 U/mg, respectively.

### [Example 33] Expression of ReTHL gene, ReAR1 gene, and CaAdhE gene in E. coli - 2

The plasmid pSQTHRA1 prepared in Example 27 was introduced into E. *coli* HB101 by electroporation to obtain a transformant *E. coli* HB101 (pSQTHRA1). This transformant was cultured by the method described in Example 2.

The culture obtained by the above-mentioned method was dispensed into a 2 ml Eppendorf tube, and centrifuged to collect the cells. Under anaerobic conditions, 50 mM MOPS buffer (pH 7.0) containing 1 mM DTT was added to the cells, and subjected to ultrasonication to disrupt the cells. Intact cells and cell debris were removed by centrifugation to obtain a cell-free extract.

The obtained cell-free extract was assayed for ReTHL activity by THL activity assay-1, for ReAR1 activity by the 3HBD activity assay, and for BCDH activity of CaAdhE by the BCDH activity assay (substrate: butyryl-CoA) and the BDH activity assay (butylaldehyde), and the results were 7.36 U/mg, 1.18 U/mg, 0.0434 U/mg and 0.151 U/mg, respectively.

### [Example 34] Production of 1,3-BG by fermentation

The objective of this Example is to produce 1,3-BG from glucose by fermentation using E. *coli* JM109 (pSQTRCA1).

Transformant *E. coli* JM109 (pSQTRCA1) was inoculated into a 21 mm diameter test tube containing 7 mL of LB medium consisting of 10 g/L tryptone, 5 g/L yeast extract, 10 g/L NaCl, and 50 mg/L ampicillin, which had been adjusted to pH 7.2. The cells were pre-cultured under aerobic conditions at 30°C while shaking at 250 rpm for 18 hours.

The pre-culture was inoculated into a 500-mL baffled flask containing 50 L of liquid medium consisting of 20 g/L tryptone, 10 g/L yeast extract, 10 g/L NaCl, and 50 mg/L ampicillin, which had been adjusted to pH 7.0. The cells were cultured at 30.0°C while shaking at 140 rpm for 18 hours. The induction of expression was performed using 0.02 mM IPTG.

Fermentation liquid 1: Washed cells obtained from the above main culture of *E. coli* JM109 (pSQTRCA1) were added to a 100-mL baffled flask with 10 mL of a solution containing 56 g/L glucose and liquid medium consisting of 20 g/L tryptone, 10 g/L yeast extract, and 10 g/L NaCl, which had been adjusted to pH 7.0, such that the inoculation ratio was 100%. The flask was sealed with a silicone plug, and fermentation was performed under non-aerated conditions at 30°C while shaking at 100 rpm for 72 hours.

Fermentation liquid 2: 10-fold concentrated washed cells from the above main culture of *E. coli* JM109 (pSQTRCA1) were added to a 100-mL baffled flask with 10 mL of a solution containing 56 g/L glucose and MES buffer (pH 6.0) such that the inoculation ratio was 100%. The flask was sealed with a silicone plug, and fermentation was performed under non-aerated conditions at 30°C while shaking at 100 rpm for 72 hours.

After 72 hours of the beginning of fermentation, 2 ml of the fermentation liquid was sampled and centrifuged to remove the cells and insoluble materials. The supernatant was filtered through Millex-LH (Millipore), and the filtrate was analyzed by HPLC under the same conditions as in Example 22. The concentration of produced 1,3-BG is shown in Table 1. *E. coli* JM109 carrying no plasmid was also used for fermentation under the same conditions, but found to produce no detectable 1,3-BG.

**[Table 1] 1,3-BG production by E. coli JM109 (pSQTRCA1)**

| No. | Fermentation liquid | 1,3-BG (g/L)¹⁾ | Molar yield (%)²⁾ |
|---|---|---|---|
| Fermentation liquid 1 | 2 x YT | 0.560 | 2.0 |
| Fermentation liquid 2 | MES (pH 6.0) | 0.819 | 2.9 |

| | | | |
|---|---|---|---|
| 1) calculated by HPLC 2) molar yield (%) = 100 x ((produced 1,3-BG [mM]) / (initial glucose concentration [mM])) | | | |

### [Example 35] Production of 1,3-BG by fermentation - 2

The pre-culture and main culture of *E. coli* HB101 (pSQTRCA1) and *E. coli* HB101 (pSQTRCB1) were carried out by the methods described in Example 34.

Fermentation liquid 3: 10-fold concentrated washed cells from the main culture of *E. coli* HB101 (pSQTRCA1) were added to a 500-mL baffled flask with 100 mL of a solution containing 30 g/L glucose, 0.02 mM IPTG, 100 mM HEPES buffer, and M9 medium consisting of 6.8 g/L Na₂HPO₄, 3.0 g/L KH₂PO₄, 0.5 g/L NaCl, 1.0 g/L NH₄Cl, 493 mg/L MgSO₄·7H₂O, and 14.7 mg/L CaCl₂·2H₂O, which had been adjusted to pH 7.5, such that the inoculation ratio was 20%. The flask was sealed with a silicone plug, and fermentation was performed at 30°C while shaking at 140 rpm for 48 hours.

Fermentation liquid 4: Fermentation was performed in the same manner as fermentation liquid 3 except that the fermentation temperature was 37°C.

Fermentation liquid 5: Fermentation was performed in the same manner as fermentation liquid 4 except that the inoculated transformant was *E. coli* HB101 (pSQTRCB 1).

After 48 hours of the beginning of fermentation, 2 ml of the fermentation liquid was sampled and centrifuged to remove the cells and insoluble materials. The supernatant was filtered through Millex-LH (Millipore), and the filtrate was analyzed by HPLC under the same conditions as in Example 22. The concentration of produced 1,3-BG is shown in Table 2.

**[Table 2] 1,3-BG production by E. coli HB101 (pSQTRCA1) and E. coli HB101 (pSQTRCB1)**

| No. | Strain | 1.3-BG (g/L)¹⁾ | Molar yield (%)²⁾ |
|---|---|---|---|
| Fermentation liquid 3 | W3110(pSQTRCA1) | 0.435 | 2.9 |
| Fermentation liquid 4 | W3110(pSQTRCA1) | 1.028 | 6.9 |
| Fermentation liquid 5 | W3110(pSQTRCB1) | 0.075 | 0.5 |

| | | | |
|---|---|---|---|
| 1) calculated by HPLC 2) molar yield (%) = 100 x ((produced 1,3-BG [mM]) / (initial glucose concentration [mM])) | | | |

### [Example 36] Determination of optical purity of produced 1,3-BG

After 48 hours of fermentation, an organic layer was extracted from a 1-mL sample of fermentation liquid 4 with ethyl acetate, and salted out using sodium chloride. After concentration of the obtained extract, 0.1 mL acetyl chloride was added and reacted at 25°C for 10 minutes. The reacted solution was neutralized using saturated sodium bicarbonate, and then an organic layer was extracted with 1 mL hexane. HPLC analysis of this extract showed that the optical purity of (R)-1,3-BG was 86.6%.

The HPLC conditions were as follows:
- HPLC column: CHIRALCEL (4.6 mm x 250 mm) manufactured by Daicel Chemical Industries, Ltd.
- Eluent: hexane : isopropanol =19:1 1
- Column temperature: 40°C
- Flow rate: 1.0 mL/min
- Detection: UV absorption at 220 nm

Under the above conditions, (S)-1,3-BG and (R)-1,3-BG was eluted at 6.8 min and 8.5 min, respectively. The optical purity was determined based on the 220-nm UV absorption of each fraction.

### [Industrial Applicability]

The present invention provides recombinant microorganisms capable of producing 1,3-butanediol efficiently from carbohydrate materials such as glucose, which are derived from renewable resources. In addition, methods for producing 1,3-butanediol using such microorganisms are provided. The methods of the present invention are industrially advantageous in that cheaper materials can be used to produce 1,3-butanediol. The present invention enables the production of 1,3-butanediol from renewable resources.

## Claims

1. A recombinant microorganism in which the enzymatic activity of (1) shown below is enhanced, wherein the microorganism produces 1,3-alkyldiol represented by Formula 2 from a fermentation substrate:
(1) activity of an enzyme that catalyzes production of 3-hydroxyalkylaldehyde by reducing 3-hydroxyacyl-CoA using NADH and/or NADPH as a coenzyme, as shown by Formula 1: 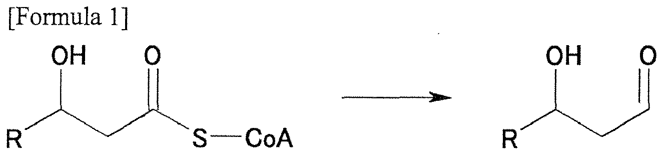 (wherein R represents a C₁₋₃ alkyl group or hydrogen; and CoA represents coenzyme A) 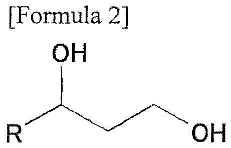 (wherein R represents a C₁₋₃ alkyl group or hydrogen).

2. The recombinant microorganism of claim 1, wherein R is methyl in Formulas 1 and 2 recited in claim 1, and the microorganism produces 1,3-butanediol from a fermentation substrate.

3. The recombinant microorganism of claim 1 or 2, wherein 1,3-butanediol produced from the fermentation substrate recited in claim 2 is (R)-1,3-butanediol.

4. The recombinant microorganism of any one of claims 1 to 3, wherein the enzyme catalyzing the reaction of Formula 1 recited in claim 1(1) is classified as EC 1.2.1.10 under the international classification of enzyme.

5. The recombinant microorganism of any one of claims 1 to 4, wherein the enzyme catalyzing the reaction of Formula 1 recited in claim 1(1) is any one of:
(a) a protein comprising the amino acid sequence of SEQ ID NO: 1, 65, or 67;
(b) an enzyme comprising an amino acid sequence in which one or more amino acids are substituted, deleted, inserted, or added in the amino acid sequence of SEQ ID NO: 1, 65, or 67;
(c) an enzyme comprising an amino acid sequence encoded by the nucleotide sequence of SEQ ID NO: 2, 66, or 68;
(d) an enzyme comprising an amino acid sequence encoded by a DNA which hybridizes under stringent conditions with a DNA comprising the nucleotide sequence of SEQ ID NO: 2, 66, or 68; and
(e) a protein having an identity of 85% or higher with the amino acid sequence of SEQ ID NO: 1, 65, or 67.

6. The recombinant microorganism of any one of claims 1 to 5, in which the enzymatic activity of (2) shown below is enhanced in addition to the enzymatic activity of (1) recited in claim 1, wherein the microorganism produces 1,3-alkyldiol represented by Formula 2 from a fermentation substrate:
(1) activity of an enzyme that catalyzes production of 3-hydroxyalkylaldehyde by reducing 3-hydroxyacyl-CoA using NADH and/or NADPH as a coenzyme, as shown by Formula 1; and
(2) activity of an enzyme that catalyzes production of 1,3-alkyldiol represented by Formula 2 by reducing 3-hydroxyalkylaldehyde using NADH and/or NADPH as a coenzyme, as shown by Formula 3 (Formula 3 does not show two reactions, but only shows the production of alcohol from aldehyde): 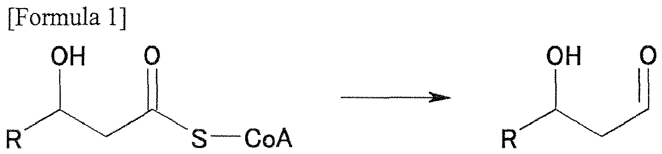 (wherein R represents a C₁₋₃ alkyl group or hydrogen; and CoA represents coenzyme A) 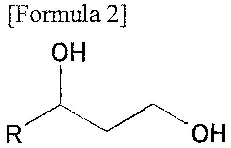 (wherein R represents a C₁₋₃ alkyl group or hydrogen) 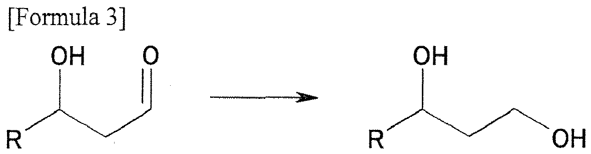 (wherein R represents a C₁₋₃ alkyl group or hydrogen).

7. The recombinant microorganism of claim 6, wherein R is methyl in Formulas 1 to 3 recited in claim 6, and the microorganism produces 1,3-butanediol from a fermentation substrate.

8. The recombinant microorganism of claim 6 or 7, wherein 1,3-butanediol produced from the fermentation substrate recited in claim 7 is (R)-1,3-butanediol.

9. The recombinant microorganism of any one of claims 6 to 8, wherein the enzyme catalyzing the reaction of Formula 3 recited in claim 6 is any one of:
(a) a protein comprising the amino acid sequence of SEQ ID NO: 3, 5, or 7;
(b) an enzyme comprising an amino acid sequence in which one or more amino acids are substituted, deleted, inserted, or added in the amino acid sequence of SEQ ID NO: 3, 5, or 7;
(c) an enzyme comprising an amino acid sequence encoded by the nucleotide sequence of SEQ ID NO: 4, 6, or 8;
(d) an enzyme comprising an amino acid sequence encoded by a DNA which hybridizes under stringent conditions with a DNA comprising the nucleotide sequence of SEQ ID NO: 4, 6, or 8; and
(e) an enzyme having an identity of 85% or higher with the amino acid sequence of SEQ ID NO: 3, 5, or 7.

10. The recombinant microorganism of any one of claims 1 to 5, in which the enzymatic activity of (3) shown below is enhanced in addition to the enzymatic activity of (1) recited in claim 1, wherein the microorganism produces 1,3-alkyldiol represented by Formula 2 from a fermentation substrate:
(1) activity of an enzyme that catalyzes production of 3-hydroxyalkylaldehyde by reducing 3-hydroxyacyl-CoA using NADH and/or NADPH as a coenzyme, as shown by Formula 1; and (3) activity of an enzyme that produces 3-hydroxyacyl-CoA by reducing 3-oxoacyl-CoA in an NADH- and/or NADPH-dependent manner as shown by Formula 4: 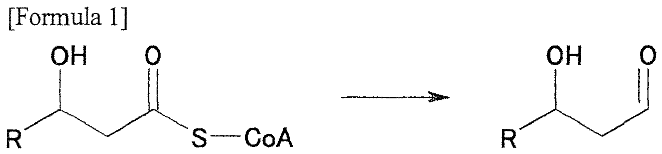 (wherein R represents a C₁₋₃ alkyl group or hydrogen; and CoA represents coenzyme A) 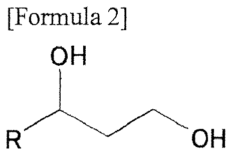 (wherein R represents a C₁₋₃ alkyl group or hydrogen) 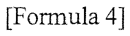 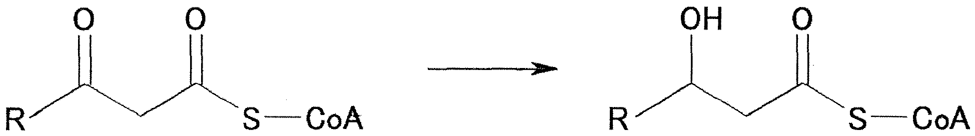 (wherein R represents a C₁₋₃ alkyl group or hydrogen; and CoA represents coenzyme A).

11. The recombinant microorganism of claim 10, wherein R is methyl in Formulas 1 and 2 recited in claim 10, and the microorganism produces (R)-1,3-butanediol represented by Formula 5 from a fermentation substrate:

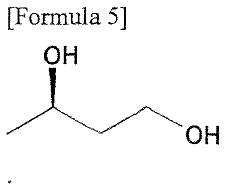

12. The recombinant microorganism of claim 10 or 11, wherein the enzyme catalyzing the reaction of Formula 4 recited in claim 10 is an R-form-specific reductase, and is any one of:
(a) a protein comprising the amino acid sequence of SEQ ID NO: 9, 11, 13, 15, or 17;
(b) an enzyme comprising an amino acid sequence in which one or more amino acids are substituted, deleted, inserted, or added in the amino acid sequence of SEQ ID NO: 9, 11, 13, 15, or 17;
(c) an enzyme comprising an amino acid sequence encoded by the nucleotide sequence of SEQ ID NO: 10, 12, 14, 16, or 18;
(d) an enzyme comprising an amino acid sequence encoded by a DNA which hybridizes under stringent conditions with a DNA comprising the nucleotide sequence of SEQ ID NO: 10, 12, 14, 16, or 18; and
(e) an enzyme having an identity of 85% or higher with the amino acid sequence of SEQ ID NO: 9, 11, 13, 15, or 17.

13. A method for producing a diol compound represented by Formula 2, comprising the steps of:
contacting a fermentation substrate with at least one active material selected from the group consisting of a culture of the recombinant microorganism of any one of claims 1 to 12, a cell of the recombinant microorganism, and a processed product thereof; and
collecting 1,3-alkyodiol represented by Formula 2: 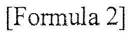 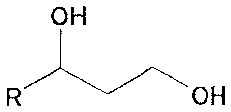 (wherein R represents a C₁₋₃ alkyl group or hydrogen).

14. The method of claim 13, wherein the diol compound is (R)-1,3 -butanediol represented by Formula 5

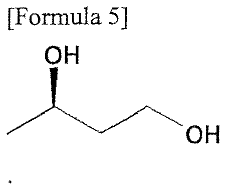

15. The method of claim 13 or 14, wherein the culturing of the recombinant microorganism and the production of the diol compound are carried out separately.


SEQUENCE LISTING

<110>  DAICEL CHEMICAL INDUSTRIES, LTD.

<120>  Recombinant microorganism provided producing function of 1, 3-
butanediol and use thereof

<130>  D1-A0902P

<150>  JP 2009-251276
<151>  2009-10-30

<160>  72

<170>  PatentIn version 3.4

<210>  1
<211>  858
<212>  PRT
<213>  Clostridium acetobutylicum

<400>  1

Met Lys Val Thr Asn Gln Lys Glu Leu Lys Gln Lys Leu Asn Glu Leu
1               5                   10                  15

Arg Glu Ala Gln Lys Lys Phe Ala Thr Tyr Thr Gln Glu Gln Val Asp
            20                  25                  30

Lys Ile Phe Lys Gln Cys Ala Ile Ala Ala Ala Lys Glu Arg Ile Asn
        35                  40                  45

Leu Ala Lys Leu Ala Val Glu Glu Thr Gly Ile Gly Leu Val Glu Asp
    50                  55                  60

Lys Ile Ile Lys Asn His Phe Ala Ala Glu Tyr Ile Tyr Asn Lys Tyr
65                  70                  75                  80

Lys Asn Glu Lys Thr Cys Gly Ile Ile Asp His Asp Asp Ser Leu Gly
                85                  90                  95

Ile Thr Lys Val Ala Glu Pro Ile Gly Ile Val Ala Ala Ile Val Pro
            100                 105                 110

Thr Thr Asn Pro Thr Ser Thr Ala Ile Phe Lys Ser Leu Ile Ser Leu
        115                 120                 125

Lys Thr Arg Asn Ala Ile Phe Phe Ser Pro His Pro Arg Ala Lys Lys
    130                 135                 140

Ser Thr Ile Ala Ala Ala Lys Leu Ile Leu Asp Ala Ala Val Lys Ala
145                 150                 155                 160

Gly Ala Pro Lys Asn Ile Ile Gly Trp Ile Asp Glu Pro Ser Ile Glu
                165                 170                 175

Leu Ser Gln Asp Leu Met Ser Glu Ala Asp Ile Ile Leu Ala Thr Gly
            180                 185                 190

Gly Pro Ser Met Val Lys Ala Ala Tyr Ser Ser Gly Lys Pro Ala Ile
        195                 200                 205

Gly Val Gly Ala Gly Asn Thr Pro Ala Ile Ile Asp Glu Ser Ala Asp
    210                 215                 220

Ile Asp Met Ala Val Ser Ser Ile Ile Leu Ser Lys Thr Tyr Asp Asn
225                 230                 235                 240

Gly Val Ile Cys Ala Ser Glu Gln Ser Ile Leu Val Met Asn Ser Ile
                245                 250                 255

Tyr Glu Lys Val Lys Glu Glu Phe Val Lys Arg Gly Ser Tyr Ile Leu
            260                 265                 270

Asn Gln Asn Glu Ile Ala Lys Ile Lys Glu Thr Met Phe Lys Asn Gly
        275                 280                 285

Ala Ile Asn Ala Asp Ile Val Gly Lys Ser Ala Tyr Ile Ile Ala Lys
    290                 295                 300

Met Ala Gly Ile Glu Val Pro Gln Thr Thr Lys Ile Leu Ile Gly Glu
305                 310                 315                 320

Val Gln Ser Val Glu Lys Ser Glu Leu Phe Ser His Glu Lys Leu Ser
                325                 330                 335

Pro Val Leu Ala Met Tyr Lys Val Lys Asp Phe Asp Glu Ala Leu Lys
            340                 345                 350

Lys Ala Gln Arg Leu Ile Glu Leu Gly Gly Ser Gly His Thr Ser Ser
        355                 360                 365

Leu Tyr Ile Asp Ser Gln Asn Asn Lys Asp Lys Val Lys Glu Phe Gly
    370                 375                 380

Leu Ala Met Lys Thr Ser Arg Thr Phe Ile Asn Met Pro Ser Ser Gln
385                 390                 395                 400

Gly Ala Ser Gly Asp Leu Tyr Asn Phe Ala Ile Ala Pro Ser Phe Thr
                405                 410                 415

Leu Gly Cys Gly Thr Trp Gly Gly Asn Ser Val Ser Gln Asn Val Glu
            420                 425                 430

Pro Lys His Leu Leu Asn Ile Lys Ser Val Ala Glu Arg Arg Glu Asn
        435                 440                 445

Met Leu Trp Phe Lys Val Pro Gln Lys Ile Tyr Phe Lys Tyr Gly Cys
    450                 455                 460

Leu Arg Phe Ala Leu Lys Glu Leu Lys Asp Met Asn Lys Lys Arg Ala
465                 470                 475                 480

Phe Ile Val Thr Asp Lys Asp Leu Phe Lys Leu Gly Tyr Val Asn Lys
                485                 490                 495

Ile Thr Lys Val Leu Asp Glu Ile Asp Ile Lys Tyr Ser Ile Phe Thr
            500                 505                 510

Asp Ile Lys Ser Asp Pro Thr Ile Asp Ser Val Lys Lys Gly Ala Lys
        515                 520                 525

Glu Met Leu Asn Phe Glu Pro Asp Thr Ile Ile Ser Ile Gly Gly Gly
    530                 535                 540

Ser Pro Met Asp Ala Ala Lys Val Met His Leu Leu Tyr Glu Tyr Pro
545                 550                 555                 560

Glu Ala Glu Ile Glu Asn Leu Ala Ile Asn Phe Met Asp Ile Arg Lys
                565                 570                 575

Arg Ile Cys Asn Phe Pro Lys Leu Gly Thr Lys Ala Ile Ser Val Ala
            580                 585                 590

Ile Pro Thr Thr Ala Gly Thr Gly Ser Glu Ala Thr Pro Phe Ala Val
        595                 600                 605

Ile Thr Asn Asp Glu Thr Gly Met Lys Tyr Pro Leu Thr Ser Tyr Glu
    610                 615                 620

Leu Thr Pro Asn Met Ala Ile Ile Asp Thr Glu Leu Met Leu Asn Met
625                 630                 635                 640

Pro Arg Lys Leu Thr Ala Ala Thr Gly Ile Asp Ala Leu Val His Ala
                645                 650                 655

Ile Glu Ala Tyr Val Ser Val Met Ala Thr Asp Tyr Thr Asp Glu Leu
            660                 665                 670

Ala Leu Arg Ala Ile Lys Met Ile Phe Lys Tyr Leu Pro Arg Ala Tyr
        675                 680                 685

Lys Asn Gly Thr Asn Asp Ile Glu Ala Arg Glu Lys Met Ala His Ala
    690                 695                 700

Ser Asn Ile Ala Gly Met Ala Phe Ala Asn Ala Phe Leu Gly Val Cys
705                 710                 715                 720

His Ser Met Ala His Lys Leu Gly Ala Met His His Val Pro His Gly
                725                 730                 735

Ile Ala Cys Ala Val Leu Ile Glu Glu Val Ile Lys Tyr Asn Ala Thr
            740                 745                 750

Asp Cys Pro Thr Lys Gln Thr Ala Phe Pro Gln Tyr Lys Ser Pro Asn
        755                 760                 765

Ala Lys Arg Lys Tyr Ala Glu Ile Ala Glu Tyr Leu Asn Leu Lys Gly
    770                 775                 780

Thr Ser Asp Thr Glu Lys Val Thr Ala Leu Ile Glu Ala Ile Ser Lys
785                 790                 795                 800

Leu Lys Ile Asp Leu Ser Ile Pro Gln Asn Ile Ser Ala Ala Gly Ile
                805                 810                 815

Asn Lys Lys Asp Phe Tyr Asn Thr Leu Asp Lys Met Ser Glu Leu Ala
            820                 825                 830

Phe Asp Asp Gln Cys Thr Thr Ala Asn Pro Arg Tyr Pro Leu Ile Ser
        835                 840                 845

Glu Leu Lys Asp Ile Tyr Ile Lys Ser Phe
    850                 855

<210>  2
<211>  2577
<212>  DNA
<213>  Clostridium acetobutylicum

<400>  2
atgaaagtta caaatcaaaa agaactaaaa caaaagctaa atgaattgag agaagcgcaa      60

aagaagtttg caacctatac tcaagagcaa gttgataaaa tttttaaaca atgtgccata     120

gccgcagcta aagaaagaat aaacttagct aaattagcag tagaagaaac aggaataggt     180

cttgtagaag ataaaattat aaaaaatcat tttgcagcag aatatatata caataaatat     240

aaaaatgaaa aaacttgtgg cataatagac catgacgatt ctttaggcat aacaaaggtt     300

gctgaaccaa ttggaattgt tgcagccata gttcctacta ctaatccaac ttccacagca     360

attttcaaat cattaatttc tttaaaaaca agaaacgcaa tattcttttc accacatcca     420

cgtgcaaaaa aatctacaat tgctgcagca aaattaattt tagatgcagc tgttaaagca     480

ggagcaccta aaaatataat aggctggata gatgagccat caatagaact ttctcaagat 540

ttgatgagtg aagctgatat aatattagca acaggaggtc cttcaatggt taaagcggcc 600

tattcatctg gaaaacctgc aattggtgtt ggagcaggaa atacaccagc aataatagat 660

gagagtgcag atatagatat ggcagtaagc tccataattt tatcaaagac ttatgacaat 720

ggagtaatat gcgcttctga acaatcaata ttagttatga attcaatata cgaaaaagtt 780

aaagaggaat ttgtaaaacg aggatcatat atactcaatc aaaatgaaat agctaaaata 840

aaagaaacta tgtttaaaaa tggagctatt aatgctgaca tagttggaaa atctgcttat 900

ataattgcta aaatggcagg aattgaagtt cctcaaacta caaagatact tataggcgaa 960

gtacaatctg ttgaaaaaag cgagctgttc tcacatgaaa aactatcacc agtacttgca 1020

atgtataaag ttaaggattt tgatgaagct ctaaaaaagg cacaaaggct aatagaatta 1080

ggtggaagtg gacacacgtc atctttatat atagattcac aaaacaataa ggataaagtt 1140

aaagaatttg gattagcaat gaaaacttca aggacattta ttaacatgcc ttcttcacag 1200

ggagcaagcg gagatttata caattttgcg atagcaccat catttactct tggatgcggc 1260

acttggggag gaaactctgt atcgcaaaat gtagagccta aacatttatt aaatattaaa 1320

agtgttgctg aaagaaggga aaatatgctt tggtttaaag tgccacaaaa aatatatttt 1380

aaatatggat gtcttagatt tgcattaaaa gaattaaaag atatgaataa gaaaagagcc 1440

tttatagtaa cagataaaga tctttttaaa cttggatatg ttaataaaat aacaaaggta 1500

ctagatgaga tagatattaa atacagtata tttacagata ttaaatctga tccaactatt 1560

gattcagtaa aaaaaggtgc taaagaaatg cttaactttg aacctgatac tataatctct 1620

attggtggtg gatcgccaat ggatgcagca aaggttatgc acttgttata tgaatatcca 1680

gaagcagaaa ttgaaaatct agctataaac tttatggata taagaaagag aatatgcaat 1740

ttccctaaat taggtacaaa ggcgatttca gtagctattc ctacaactgc tggtaccggt 1800

tcagaggcaa caccttttgc agttataact aatgatgaaa caggaatgaa atacccttta 1860

acttcttatg aattgacccc aaacatggca ataatagata ctgaattaat gttaaatatg 1920

cctagaaaat taacagcagc aactggaata gatgcattag ttcatgctat agaagcatat 1980

gtttcggtta tggctacgga ttatactgat gaattagcct taagagcaat aaaaatgata 2040

tttaaatatt tgcctagagc ctataaaaat gggactaacg acattgaagc aagagaaaaa 2100

atggcacatg cctctaatat tgcggggatg gcatttgcaa atgctttctt aggtgtatgc 2160

cattcaatgg ctcataaact tggggcaatg catcacgttc cacatggaat tgcttgtgct 2220

gtattaatag aagaagttat taaatataac gctacagact gtccaacaaa gcaaacagca 2280

ttccctcaat ataaatctcc taatgctaag agaaaatatg ctgaaattgc agagtatttg 2340

aatttaaagg gtactagcga taccgaaaag gtaacagcct taatagaagc tatttcaaag 2400

ttaaagatag atttgagtat tccacaaaat ataagtgccg ctggaataaa taaaaaagat 2460

ttttataata cgctagataa aatgtcagag cttgcttttg atgaccaatg tacaacagct 2520

aatcctaggt atccacttat aagtgaactt aaggatatct atataaaatc attttaa 2577

<210> 3
<211> 390
<212> PRT
<213> Clostridium acetobutylicum

<400> 3

Val Val Asp Phe Glu Tyr Ser Ile Pro Thr Arg Ile Phe Phe Gly Lys
1               5                   10                  15

Asp Lys Ile Asn Val Leu Gly Arg Glu Leu Lys Lys Tyr Gly Ser Lys
            20                  25                  30

Val Leu Ile Val Tyr Gly Gly Gly Ser Ile Lys Arg Asn Gly Ile Tyr
        35                  40                  45

Asp Lys Ala Val Ser Ile Leu Glu Lys Asn Ser Ile Lys Phe Tyr Glu
    50                  55                  60

Leu Ala Gly Val Glu Pro Asn Pro Arg Val Thr Thr Val Glu Lys Gly
65                  70                  75                  80

Val Lys Ile Cys Arg Glu Asn Gly Val Glu Val Val Leu Ala Ile Gly
                85                  90                  95

Gly Gly Ser Ala Ile Asp Cys Ala Lys Val Ile Ala Ala Ala Cys Glu
            100                 105                 110

Tyr Asp Gly Asn Pro Trp Asp Ile Val Leu Asp Gly Ser Lys Ile Lys
        115                 120                 125

Arg Val Leu Pro Ile Ala Ser Ile Leu Thr Ile Ala Ala Thr Gly Ser
    130                 135                 140

Glu Met Asp Thr Trp Ala Val Ile Asn Asn Met Asp Thr Asn Glu Lys
145                 150                 155                 160

Leu Ile Ala Ala His Pro Asp Met Ala Pro Lys Phe Ser Ile Leu Asp
                165                 170                 175

Pro Thr Tyr Thr Tyr Thr Val Pro Thr Asn Gln Thr Ala Ala Gly Thr
            180                 185                 190

Ala Asp Ile Met Ser His Ile Phe Glu Val Tyr Phe Ser Asn Thr Lys
        195                 200                 205

Thr Ala Tyr Leu Gln Asp Arg Met Ala Glu Ala Leu Leu Arg Thr Cys
    210                 215                 220

Ile Lys Tyr Gly Gly Ile Ala Leu Glu Lys Pro Asp Asp Tyr Glu Ala
225                 230                 235                 240

Arg Ala Asn Leu Met Trp Ala Ser Ser Leu Ala Ile Asn Gly Leu Leu
                245                 250                 255

Thr Tyr Gly Lys Asp Thr Asn Trp Ser Val His Leu Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asn Asp Thr Val Tyr Lys
    290                 295                 300

Phe Val Glu Tyr Gly Val Asn Val Trp Gly Ile Asp Lys Glu Lys Asn
305                 310                 315                 320

His Tyr Asp Ile Ala His Gln Ala Ile Gln Lys Thr Arg Asp Tyr Phe
                325                 330                 335

Val Asn Val Leu Gly Leu Pro Ser Arg Leu Arg Asp Val Gly Ile Glu
            340                 345                 350

Glu Glu Lys Leu Asp Ile Met Ala Lys Glu Ser Val Lys Leu Thr Gly
        355                 360                 365

Gly Thr Ile Gly Asn Leu Arg Pro Val Asn Ala Ser Glu Val Leu Gln
    370                 375                 380

Ile Phe Lys Lys Ser Val
385                 390

<210>  4
<211>  1173
<212>  DNA
<213>  Clostridium acetobutylicum

<400>  4
gtggttgatt tcgaatattc aataccaact agaatttttt tcggtaaaga taagataaat      60

gtacttggaa gagagcttaa aaaatatggt tctaaagtgc ttatagttta tggtggagga     120

agtataaaga gaaatggaat atatgataaa gctgtaagta tacttgaaaa aaacagtatt     180

aaattttatg aacttgcagg agtagagcca aatccaagag taactacagt tgaaaaagga     240

gttaaaatat gtagagaaaa tggagttgaa gtagtactag ctataggtgg aggaagtgca     300

atagattgcg caaaggttat agcagcagca tgtgaatatg atggaaatcc atgggatatt 360

gtgttagatg gctcaaaaat aaaaagggtg cttcctatag ctagtatatt aaccattgct 420

gcaacaggat cagaaatgga tacgtgggca gtaataaata atatggatac aaacgaaaaa 480

ctaattgcgg cacatccaga tatggctcct aagttttcta tattagatcc aacgtatacg 540

tataccgtac ctaccaatca aacagcagca ggaacagctg atattatgag tcatatattt 600

gaggtgtatt ttagtaatac aaaaacagca tatttgcagg atagaatggc agaagcgtta 660

ttaagaactt gtattaaata tggaggaata gctcttgaga agccggatga ttatgaggca 720

agagccaatc taatgtgggc ttcaagtctt gcgataaatg gacttttaac atatggtaaa 780

gacactaatt ggagtgtaca cttaatggaa catgaattaa gtgcttatta cgacataaca 840

cacggcgtag ggcttgcaat tttaacacct aattggatgg agtatatttt aaataatgat 900

acagtgtaca agtttgttga atatggtgta aatgtttggg gaatagacaa agaaaaaaat 960

cactatgaca tagcacatca agcaatacaa aaaacaagag attactttgt aaatgtacta 1020

ggtttaccat ctagactgag agatgttgga attgaagaag aaaaattgga cataatggca 1080

aaggaatcag taaagcttac aggaggaacc ataggaaacc taagaccagt aaacgcctcc 1140

gaagtcctac aaatattcaa aaaatctgtg taa 1173

<210> 5
<211> 337
<212> PRT
<213> Bacillus\201@stearothermophilus

<400> 5

Met Lys Ala Ala Val Val Glu Gln Phe Lys Lys Pro Leu Gln Val Lys
1               5                   10                  15

Glu Val Glu Lys Pro Lys Ile Ser Tyr Gly Glu Val Leu Val Arg Ile
            20                  25                  30

Lys Ala Cys Gly Val Cys His Thr Asp Leu His Ala Ala His Gly Asp
        35                  40                  45

Trp Pro Val Lys Pro Lys Leu Pro Leu Ile Pro Gly His Glu Gly Val
    50                  55                  60

Gly Val Ile Glu Glu Val Gly Pro Gly Val Thr His Leu Lys Val Gly
65                  70                  75                  80

Asp Arg Val Gly Ile Pro Trp Leu Tyr Ser Ala Cys Gly His Cys Asp
                85                  90                  95

Tyr Cys Leu Ser Gly Gln Glu Thr Leu Cys Glu Arg Gln Gln Asn Ala
            100                 105                 110

Gly Tyr Ser Val Asp Gly Gly Tyr Ala Glu Tyr Cys Arg Ala Ala Ala
        115                 120                 125

Asp Tyr Val Val Lys Ile Pro Asp Asn Leu Ser Phe Glu Glu Ala Ala
    130                 135                 140

Pro Ile Phe Cys Ala Gly Val Thr Thr Tyr Lys Ala Leu Lys Val Thr
145                 150                 155                 160

Gly Ala Lys Pro Gly Glu Trp Val Ala Ile Tyr Gly Ile Gly Gly Leu
                165                 170                 175

Gly His Val Ala Val Gln Tyr Ala Lys Ala Met Gly Leu Asn Val Val
            180                 185                 190

Ala Val Asp Leu Gly Asp Glu Lys Leu Glu Leu Ala Lys Gln Leu Gly
        195                 200                 205

Ala Asp Leu Val Val Asn Pro Lys His Asp Asp Ala Ala Gln Trp Ile
    210                 215                 220

Lys Glu Lys Val Gly Gly Val His Ala Thr Val Val Thr Ala Val Ser
225                 230                 235                 240

Lys Ala Ala Phe Glu Ser Ala Tyr Lys Ser Ile Arg Arg Gly Gly Ala
                245                 250                 255

Cys Val Leu Val Gly Leu Pro Pro Glu Glu Ile Pro Ile Pro Ile Phe
            260                 265                 270

Asp Thr Val Leu Asn Gly Val Lys Ile Ile Gly Ser Ile Val Gly Thr
        275                 280                 285

Arg Lys Asp Leu Gln Glu Ala Leu Gln Phe Ala Ala Glu Gly Lys Val
    290                 295                 300

Lys Thr Ile Val Glu Val Gln Pro Leu Glu Asn Ile Asn Asp Val Phe
305                 310                 315                 320

Asp Arg Met Leu Lys Gly Gln Ile Asn Gly Arg Val Val Leu Lys Val
                325                 330                 335

Asp

<210>  6
<211>  1014
<212>  DNA
<213>  Bacillus\201@stearothermophilus

<400>  6
atgaaagctg cagttgtgga acaatttaaa aagccgttac aagtgaaaga agtggaaaaa      60

cctaagatct catacgggga agtattagtg cgcatcaaag cgtgtggggt atgccataca     120

gacttgcatg ccgcacatgg cgactggcct gtaaagccta aactgcctct cattcctggc     180

catgaaggcg tcggtgtaat tgaagaagta ggtcctgggg taacacattt aaaagttgga     240

gatcgcgtag gtatcccttg gctttattcg gcgtgcggtc attgtgacta ttgcttaagc     300

ggacaagaaa cattatgcga acgtcaacaa aacgctggct attccgtcga tggtggttat     360

gctgaatatt gccgtgctgc agccgattat gtcgtaaaaa ttcctgataa cttatcgttt     420

gaagaagccg ctccaatctt ttgcgctggt gtaacaacat ataaagcgct caaagtaaca     480

ggcgcaaaac caggtgaatg ggtagccatt tacggtatcg gcgggcttgg acatgtcgca     540

gtccaatacg caaaggcgat ggggttaaac gtcgttgctg tcgatttagg tgatgaaaaa     600

cttgagcttg ctaaacaact tggtgcagat cttgtcgtca atccgaaaca tgatgatgca     660

gcacaatgga taaaagaaaa agtgggcggt gtgcatgcga ctgtcgtcac agctgtttca     720

aaagccgcgt tcgaatcagc ctacaaatcc attcgtcgcg gtggtgcttg cgtactcgtc     780

ggattaccgc cggaagaaat acctattcca attttcgata cagtattaaa tggagtaaaa     840

attattggtt ctatcgttgg tacgcgcaaa gacttacaag aggcacttca atttgcagca     900

gaaggaaaag taaaaacaat tgtcgaagtg caaccgcttg aaaacattaa cgacgtattc     960

gatcgtatgt taaaagggca aattaacggc cgcgtcgtgt taaaagtaga ttaa          1014

<210>  7
<211>  348
<212>  PRT
<213>  Saccharomyces\201@cerevisiae

<400>  7

Met Ser Ile Pro Glu Thr Gln Lys Ala Ile Ile Phe Tyr Glu Ser Asn
1               5                   10                  15

Gly Lys Leu Glu His Lys Asp Ile Pro Val Pro Lys Pro Lys Pro Asn
            20                  25                  30

Glu Leu Leu Ile Asn Val Lys Tyr Ser Gly Val Cys His Thr Asp Leu
        35                  40                  45

His Ala Trp His Gly Asp Trp Pro Leu Pro Thr Lys Leu Pro Leu Val
    50                  55                  60

Gly Gly His Glu Gly Ala Gly Val Val Val Gly Met Gly Glu Asn Val
65                  70                  75                  80

Lys Gly Trp Lys Ile Gly Asp Tyr Ala Gly Ile Lys Trp Leu Asn Gly

                85                  90                  95

Ser Cys Met Ala Cys Glu Tyr Cys Glu Leu Gly Asn Glu Ser Asn Cys
            100                 105                 110

Pro His Ala Asp Leu Ser Gly Tyr Thr His Asp Gly Ser Phe Gln Glu
        115                 120                 125

Tyr Ala Thr Ala Asp Ala Val Gln Ala Ala His Ile Pro Gln Gly Thr
    130                 135                 140

Asp Leu Ala Glu Val Ala Pro Ile Leu Cys Ala Gly Ile Thr Val Tyr
145                 150                 155                 160

Lys Ala Leu Lys Ser Ala Asn Leu Arg Ala Gly His Trp Ala Ala Ile
                165                 170                 175

Ser Gly Ala Ala Gly Gly Leu Gly Ser Leu Ala Val Gln Tyr Ala Lys
            180                 185                 190

Ala Met Gly Tyr Arg Val Leu Gly Ile Asp Gly Gly Pro Gly Lys Glu
        195                 200                 205

Glu Leu Phe Thr Ser Leu Gly Gly Glu Val Phe Ile Asp Phe Thr Lys
    210                 215                 220

Glu Lys Asp Ile Val Ser Ala Val Val Lys Ala Thr Asn Gly Gly Ala
225                 230                 235                 240

His Gly Ile Ile Asn Val Ser Val Ser Glu Ala Ala Ile Glu Ala Ser
                245                 250                 255

Thr Arg Tyr Cys Arg Ala Asn Gly Thr Val Val Leu Val Gly Leu Pro
            260                 265                 270

Ala Gly Ala Lys Cys Ser Ser Asp Val Phe Asn His Val Val Lys Ser
        275                 280                 285

Ile Ser Ile Val Gly Ser Tyr Val Gly Asn Arg Ala Asp Thr Arg Glu
    290                 295                 300

Ala Leu Asp Phe Phe Ala Arg Gly Leu Val Lys Ser Pro Ile Lys Val
305                 310                 315                 320

Val Gly Leu Ser Ser Leu Pro Glu Ile Tyr Glu Lys Met Glu Lys Gly
                325                 330                 335

Gln Ile Ala Gly Arg Tyr Val Val Asp Thr Ser Lys
            340                 345

<210>  8
<211>  1047
<212>  DNA
<213>  Saccharomyces\201@cerevisiae

<400>  8
atgtctattc cagaaactca aaaagccatt atcttctacg aatccaacgg caagttggag      60

cataaggata tcccagttcc aaagccaaag cccaacgaat tgttaatcaa cgtcaagtac     120

tctggtgtct gccacaccga tttgcacgct tggcatggtg actggccatt gccaactaag     180

ttaccattag ttggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca agaatacgct accgctgacg ctgttcaagc cgctcacatt     420

cctcaaggta ctgacttggc tgaagtcgcg ccaatcttgt gtgctggtat caccgtatac     480

aaggctttga agtctgccaa cttgagagca ggccactggg cggccatttc tggtgctgct     540

ggtggtctag gttctttggc tgttcaatat gctaaggcga tgggttacag agtcttaggt     600

attgatggtg gtccaggaaa ggaagaattg tttacctcgc tcggtggtga agtattcatc     660

gacttcacca aagagaagga cattgttagc gcagtcgtta aggctaccaa cggcggtgcc     720

cacggtatca tcaatgtttc cgtttccgaa gccgctatcg aagcttctac cagatactgt     780

agggcgaacg gtactgttgt cttggttggt ttgccagccg gtgcaaagtg ctcctctgat     840

gtcttcaacc acgttgtcaa gtctatctcc attgtcggct cttacgtggg gaacagagct     900

gataccagag aagccttaga tttctttgcc agaggtctag tcaagtctcc aataaaggta     960

gttggcttat ccagtttacc agaaatttac gaaaagatgg agaagggcca aattgctggt    1020

cgttacgttg ttgacacttc taaataa                                        1047


<210>  9
<211>  246
<212>  PRT
<213>  Ralstonia eutropha

<400>  9

Met Thr Gln Arg Ile Ala Tyr Val Thr Gly Gly Met Gly Gly Ile Gly
1               5                   10                  15


Thr Ala Ile Cys Gln Arg Leu Ala Lys Asp Gly Phe Arg Val Val Ala
            20                  25                  30


Gly Cys Gly Pro Asn Ser Pro Arg Arg Glu Lys Trp Leu Glu Gln Gln
        35                  40                  45


Lys Ala Leu Gly Phe Asp Phe Ile Ala Ser Glu Gly Asn Val Ala Asp

50                  55                  60

Trp Asp Ser Thr Lys Thr Ala Phe Asp Lys Val Lys Ser Glu Val Gly
65                  70                  75                  80

Glu Val Asp Val Leu Ile Asn Asn Ala Gly Ile Thr Arg Asp Val Val
                85                  90                  95

Phe Arg Lys Met Thr Arg Ala Asp Trp Asp Ala Val Ile Asp Thr Asn
            100                 105                 110

Leu Thr Ser Leu Phe Asn Val Thr Lys Gln Val Ile Asp Gly Met Ala
        115                 120                 125

Asp Arg Gly Trp Gly Arg Ile Val Asn Ile Ser Ser Val Asn Gly Gln
    130                 135                 140

Lys Gly Gln Phe Gly Gln Thr Asn Tyr Ser Thr Ala Lys Ala Gly Leu
145                 150                 155                 160

His Gly Phe Thr Met Ala Leu Ala Gln Glu Val Ala Thr Lys Gly Val
                165                 170                 175

Thr Val Asn Thr Val Ser Pro Gly Tyr Ile Ala Thr Asp Met Val Lys
            180                 185                 190

Ala Ile Arg Gln Asp Val Leu Asp Lys Ile Val Ala Thr Ile Pro Val
        195                 200                 205

Lys Arg Leu Gly Leu Pro Glu Glu Ile Ala Ser Ile Cys Ala Trp Leu
    210                 215                 220

Ser Ser Glu Glu Ser Gly Phe Ser Thr Gly Ala Asp Phe Ser Leu Asn
225                 230                 235                 240

Gly Gly Leu His Met Gly
                245

<210>  10
<211>  741
<212>  DNA
<213>  Ralstonia eutropha

<400>  10
atgactcagc gcattgcgta tgtgaccggc ggcatgggtg gtatcggaac cgccatttgc      60

cagcggctgg ccaaggatgg ctttcgtgtg gtggccggtt gcggccccaa ctcgccgcgc     120

cgcgaaaagt ggctggagca gcagaaggcc ctgggcttcg atttcattgc ctcggaaggc     180

aatgtggctg actgggactc gaccaagacc gcattcgaca aggtcaagtc cgaggtcggc     240

gaggttgatg tgctgatcaa caacgccggt atcacccgcg acgtggtgtt ccgcaagatg 300

acccgcgccg actgggatgc ggtgatcgac accaacctga cctcgctgtt caacgtcacc 360

aagcaggtga tcgacggcat ggccgaccgt ggctggggcc gcatcgtcaa catctcgtcg 420

gtgaacgggc agaagggcca gttcggccag accaactact ccaccgccaa ggccggcctg 480

catggcttca ccatggcact ggcgcaggaa gtggcgacca agggcgtgac cgtcaacacg 540

gtctctccgg gctatatcgc caccgacatg gtcaaggcga tccgccagga cgtgctcgac 600

aagatcgtcg cgacgatccc ggtcaagcgc ctgggcctgc cggaagagat cgcctcgatc 660

tgcgcctggt tgtcgtcgga ggagtccggt ttctcgaccg gcgccgactt ctcgctcaac 720

ggcggcctgc atatgggctg a 741

<210> 11
<211> 241
<212> PRT
<213> Zoogloea ramigera

<400> 11

Met Ser Arg Val Ala Leu Val Thr Gly Gly Ser Arg Gly Ile Gly Ala
1               5                   10                  15

Ala Ile Ser Ile Ala Leu Lys Ala Ala Gly Tyr Lys Val Ala Ala Ser
            20                  25                  30

Tyr Ala Gly Asn Asp Asp Ala Ala Asn Ala Phe Lys Ala Glu Thr Gly
        35                  40                  45

Ile Ala Val Tyr Lys Trp Asp Val Ser Ser Tyr Glu Ala Cys Val Glu
    50                  55                  60

Gly Ile Ala Lys Val Glu Ala Asp Leu Gly Pro Ile Asp Val Leu Val
65                  70                  75                  80

Asn Asn Ala Gly Ile Thr Lys Asp Ala Met Phe His Lys Met Thr Pro
                85                  90                  95

Asp Gln Trp Asn Ala Val Ile Asn Thr Asn Leu Thr Gly Leu Phe Asn
            100                 105                 110

Met Thr His Pro Val Trp Ser Gly Met Arg Asp Arg Ser Phe Gly Arg
        115                 120                 125

Ile Val Asn Ile Ser Ser Ile Asn Gly Gln Lys Gly Gln Met Gly Gln
    130                 135                 140

Ala Asn Tyr Ser Ala Ala Lys Ala Gly Asp Leu Gly Phe Thr Lys Ala
145                 150                 155                 160

Leu Ala Gln Glu Gly Ala Ala Lys Gly Ile Thr Val Asn Ala Ile Cys
                165                 170                 175

Pro Gly Tyr Ile Gly Thr Glu Met Val Arg Ala Ile Pro Glu Lys Val
            180                 185                 190

Leu Asn Glu Arg Ile Ile Pro Gln Ile Pro Val Gly Arg Leu Gly Glu
        195                 200                 205

Pro Asp Glu Ile Ala Arg Ile Val Val Phe Leu Ala Ser Asp Glu Ala
    210                 215                 220

Gly Phe Ile Thr Gly Ser Thr Ile Ser Ala Asn Gly Gly Gln Phe Phe
225                 230                 235                 240

Val

<210>  12
<211>  726
<212>  DNA
<213>  Zoogloea ramigera

<400>  12
atgagtcgtg tagcattggt aacgggggga tcgcggggca tcggcgcagc catttcgatt      60

gcgctgaagg cggcgggata caaggtggct gccagctatg ccggcaatga cgatgcggcc     120

aacgccttca aggcggaaac gggcatcgcc gtctacaagt gggacgtgtc gagctacgag     180

gcctgcgtcg aggggatcgc caaggtggag gccgatctcg gcccgattga cgttctcgtc     240

aacaatgcgg gcatcaccaa ggacgcgatg ttccacaaga tgacgcccga ccagtggaat     300

gcggtcatca acaccaacct cacgggtctc ttcaacatga cccatccggt ctggtccggc     360

atgcgcgacc gcagcttcgg ccgcatcgtc aacatctcct cgatcaacgg ccagaagggc     420

cagatgggtc aggcgaacta ttccgccgcc aaggccggcg acctcggctt caccaaggcg     480

ctggcgcagg aaggcgcggc caagggcatc acggtcaacg ccatctgccc cggctatatc     540

ggtacggaaa tggtgcgcgc cattccggaa aaggtgctga acgagcggat catcccgcag     600

atcccggtcg gccgcctcgg cgagccggac gagatcgccc gcatcgtcgt cttcctcgcc     660

tcggacgagg ccggcttcat caccggctcg accatctcgg cgaacggcgg ccagttcttc     720

gtctaa                                                                726

<210>  13
<211>  254
<212>  PRT
<213>  Bacillus stearothermophilus

<400>  13

Met Ser Gln Arg Phe Ala Gly Arg Val Ala Phe Val Thr Gly Gly Ser
1               5                   10                  15

Arg Gly Ile Gly Lys Ala Ile Val Thr Arg Phe Ala Glu Glu Gly Ala
            20                  25                  30

Lys Val Ala Phe Ile Asp Leu Asn Glu Glu Ala Leu Glu Ala Thr Ala
        35                  40                  45

Ala Glu Leu Arg Glu Lys Gly Tyr Asp Val Tyr Ala Lys Val Ala Ser
    50                  55                  60

Val Thr Asp Arg Glu Gln Val Glu Thr Thr Met Gln Glu Val Val Asp
65                  70                  75                  80

Arg Phe Gly Ser Leu Asp Ile Leu Val Asn Asn Ala Gly Val Ile Arg
                85                  90                  95

Asp Asn Leu Leu Phe Lys Met Thr Asp Asp Asp Trp Gln Thr Val Met
            100                 105                 110

Asp Val His Leu Lys Gly Ala Phe Tyr Cys Ala Arg Ala Ala Gln Lys
        115                 120                 125

Tyr Met Val Glu Lys Gly Tyr Gly Arg Ile Ile Asn Ile Ser Ser Thr
    130                 135                 140

Ser Ala Leu Gly Asn Arg Gly Gln Ala Asn Tyr Ser Ala Ala Lys Ala
145                 150                 155                 160

Gly Ile Gln Gly Phe Thr Lys Thr Leu Ala Ile Glu Leu Gly Lys Phe
                165                 170                 175

Gly Ile Thr Thr Asn Ala Ile Ala Pro Gly Phe Ile Glu Thr Asp Met
            180                 185                 190

Thr Lys Ala Thr Ala Glu Arg Leu Gly Ile Ser Phe Glu Gln Leu Ile
        195                 200                 205

Gln Ala Ser Val Ala Asn Ile Pro Val Gly Arg Ser Gly Arg Pro Glu
    210                 215                 220

Asp Ile Ala His Ala Val Ala Phe Phe Ala Asp Glu Arg Ser Ser Phe
225                 230                 235                 240

Val Asn Gly Gln Val Leu Tyr Val Ala Gly Gly Pro Lys Cys
                245                 250

<210>  14

<211>  765
<212>  DNA
<213>  Bacillus stearothermophilus

<400>  14
atgtctcaac gttttgcagg tcgtgtggcg tttgtgaccg gaggaagccg cggcatcggc      60

aaggcgattg tcacccgctt cgcggaagaa ggggcgaaag tggcattcat cgatttaaat     120

gaagaagcgc ttgaagcgac ggctgccgag ctgcgggaga aagggtatga cgtgtacgcc     180

aaagtggcga gcgtcaccga ccgcgaacaa gtggaaacga cgatgcaaga ggtcgtcgac     240

cggttcggct cgcttgatat tcttgtcaac aacgccggcg tcatccgcga caacttgctc     300

tttaaaatga cggacgacga ctggcaaacg gtcatggacg ttcatttgaa aggggcgttt     360

tattgcgccc gcgccgcgca aaaatatatg gtcgaaaaag ggtacgggcg catcatcaac     420

atttcgtcga cctccgcgct cggaaaccgc ggccaggcga actattcggc ggcgaaagcc     480

ggcattcaag ggtttacgaa aacgttggcg atcgagctcg gcaagttcgg cattacgacg     540

aacgccatcg ctccaggatt cattgaaacc gatatgacaa aggcaaccgc tgagcggctt     600

ggcatttcgt ttgagcagct cattcaggcg agcgtcgcca acatcccggt cggacgcagt     660

ggccgtccgg aagacatcgc ccatgcagtc gcgtttttcg ccgacgaacg gtcgtcgttt     720

gtcaacggcc aagtgctgta tgtggcaggt ggtccaaaat gttaa                     765


<210>  15
<211>  261
<212>  PRT
<213>  Streptomyces violaceoruber

<400>  15

Met Ala Thr Gln Asp Ser Glu Val Ala Leu Val Thr Gly Ala Thr Ser
1               5                   10                  15


Gly Ile Gly Leu Glu Ile Ala Arg Arg Leu Gly Lys Glu Gly Leu Arg
            20                  25                  30


Val Phe Val Cys Ala Arg Gly Glu Glu Gly Leu Arg Thr Thr Leu Lys
        35                  40                  45


Glu Leu Arg Glu Ala Gly Val Glu Ala Asp Gly Arg Thr Cys Asp Val
    50                  55                  60


Arg Ser Val Pro Glu Ile Glu Ala Leu Val Ala Ala Val Val Glu Arg
65                  70                  75                  80


Tyr Gly Pro Val Asp Val Leu Val Asn Asn Ala Gly Arg Pro Gly Gly
                85                  90                  95


Gly Ala Thr Ala Glu Leu Ala Asp Glu Leu Trp Leu Asp Val Val Glu
            100                 105                 110

Thr Asn Leu Thr Gly Val Phe Arg Val Thr Lys Gln Val Leu Lys Ala
        115                 120                 125

Gly Gly Met Leu Glu Arg Gly Thr Gly Arg Ile Val Asn Ile Ala Ser
    130                 135                 140

Thr Gly Gly Lys Gln Gly Val Val His Ala Ala Pro Tyr Ser Ala Ser
145                 150                 155                 160

Lys His Gly Val Val Gly Phe Thr Lys Ala Leu Gly Leu Glu Leu Ala
                165                 170                 175

Arg Thr Gly Ile Thr Val Asn Ala Val Cys Pro Gly Phe Val Glu Thr
            180                 185                 190

Pro Met Ala Ala Ser Val Arg Glu His Tyr Ser Asp Ile Trp Glu Val
        195                 200                 205

Ser Thr Glu Glu Ala Phe Asp Arg Ile Thr Ala Arg Val Pro Ile Gly
    210                 215                 220

Arg Tyr Val Gln Pro Ser Glu Val Ala Glu Met Val Ala Tyr Leu Ile
225                 230                 235                 240

Gly Pro Gly Ala Ala Ala Val Thr Ala Gln Ala Leu Asn Val Cys Gly
                245                 250                 255

Gly Leu Gly Asn Tyr
            260

<210>  16
<211>  786
<212>  DNA
<213>  Streptomyces violaceoruber

<400>  16
atggccacgc aggactccga agtcgcactg gtgacgggtg cgaccagcgg aatcgggctg      60

gagatcgccc gcaggctcgg taaggagggg ttgcgcgtgt tcgtctgcgc gcgcggcgaa     120

gaaggactgc ggacgacgct gaaggagttg cgggaggcgg gcgtggaggc ggacgggcgg      180

acctgcgacg tccgctcggt cccggagatc gaggctctcg tggcggcggt ggtcgagcgt     240

tacggtccgg tcgacgtgct ggtgaacaac gcgggccggc ccggcggcgg cgccacggcc     300

gaactcgccg acgaactctg gctcgatgtc gtggagacca acctcaccgg cgtgttccgg     360

gtgaccaaac aggtcctcaa ggcgggcggc atgctcgaac ggggcacggg ccgaatcgtc     420

aacatcgcct cgaccggcgg aaagcagggt gtggtgcacg ccgcgcccta ctccgcctcg     480

aagcacggcg tggtcggctt caccaaggcg ctcggtctcg aactggcgag gaccggcatc     540

acggtgaacg ccgtctgccc cggattcgtc gagacgccga tggccgcgtc cgtgcgcgag 600

cactactcgg acatctggga ggtgtcgacc gaggaggcct tcgaccggat caccgcgcgc 660

gtgccgatcg gccggtacgt gcagccgtcc gaggtggcgg agatggtggc gtacctgatc 720

gggcccggtg cggccgcggt caccgcgcag gcgctgaacg tctgcggcgg gctggggaac 780

tactaa 786

<210> 17
<211> 254
<212> PRT
<213> Pichia finlandica

<400> 17

Met Ser Tyr Asn Phe His Asn Lys Val Ala Val Val Thr Gly Ala Leu
1               5                   10                  15

Ser Gly Ile Gly Leu Ser Val Ala Lys Lys Phe Leu Gln Leu Gly Ala
            20                  25                  30

Lys Val Thr Ile Ser Asp Val Ser Gly Glu Lys Lys Tyr His Glu Thr
        35                  40                  45

Val Val Ala Leu Lys Ala Gln Asn Leu Asn Thr Asp Asn Leu His Tyr
    50                  55                  60

Val Gln Ala Asp Ser Ser Lys Glu Glu Asp Asn Lys Lys Leu Ile Ser
65                  70                  75                  80

Glu Thr Leu Ala Thr Phe Gly Gly Leu Asp Ile Val Cys Ala Asn Ala
                85                  90                  95

Gly Ile Gly Lys Phe Ala Pro Thr His Glu Thr Pro Phe Asp Val Trp
            100                 105                 110

Lys Lys Val Ile Ala Val Asn Leu Asn Gly Val Phe Leu Leu Asp Lys
        115                 120                 125

Leu Ala Ile Asn Tyr Trp Leu Glu Lys Ser Lys Pro Gly Val Ile Val
    130                 135                 140

Asn Met Gly Ser Val His Ser Phe Val Ala Ala Pro Gly Leu Ala His
145                 150                 155                 160

Tyr Gly Ala Ala Lys Gly Gly Val Lys Leu Leu Thr Gln Thr Leu Ala
                165                 170                 175

Leu Glu Tyr Ala Ser His Gly Ile Arg Val Asn Ser Val Asn Pro Gly
            180                 185                 190

Tyr Ile Ser Thr Pro Leu Ile Asp Glu Val Pro Lys Glu Arg Leu Asp
        195                 200                 205

Lys Leu Val Ser Leu His Pro Ile Gly Arg Leu Gly Arg Pro Glu Glu
    210                 215                 220

Val Ala Asp Ala Val Ala Phe Leu Cys Ser Gln Glu Ala Thr Phe Ile
225                 230                 235                 240

Asn Gly Val Ser Leu Pro Val Asp Gly Gly Tyr Thr Ala Gln
                245                 250

<210> 18
<211> 765
<212> DNA
<213> Pichia finlandica

<400> 18
atgtcttata atttccataa caaggttgca gttgttactg gagctctatc aggaatcggc 60

ttaagcgtcg caaaaaagtt ccttcagctc ggcgccaaag taacgatctc tgatgtcagt 120

ggagagaaaa aatatcacga gactgttgtt gctctgaaag cccaaaatct caacactgac 180

aacctccatt atgtacaggc agattccagc aaagaagaag ataacaagaa attgatttcg 240

gaaactctgg caacctttgg gggcctggat attgtttgtg ctaatgcagg aattggaaag 300

ttcgctccca cccatgaaac acccttcgac gtatggaaga aggtgattgc tgtgaatttg 360

aatggagtat tcttactgga taagctagcc atcaattact ggctagagaa aagcaaaccc 420

ggcgtaattg tcaacatggg atcagtccac tcttttgtag cagctcctgg ccttgcgcat 480

tatggagctg caaaaggcgg tgtcaaactg ttaacacaaa cattggctct ggagtacgca 540

tctcatggta ttcgtgtaaa ttctgtcaat ccggggtaca tttcgactcc tttgatagat 600

gaggttccga aagagcggtt ggataaactt gtaagcctgc accctattgg gcgtctgggt 660

cgtccagagg aagttgctga tgcagtcgca tttctgtgtt cccaggaggc cactttcatc 720

aacggcgttt ctttgccggt tgacggtggg tacacagcac aataa 765

<210> 19
<211> 282
<212> PRT
<213> Clostridium acetobutylicum

<400> 19

Met Lys Lys Val Cys Val Ile Gly Ala Gly Thr Met Gly Ser Gly Ile
1               5                   10                  15

Ala Gln Ala Phe Ala Ala Lys Gly Phe Glu Val Val Leu Arg Asp Ile
            20                  25                  30

Lys Asp Glu Phe Val Asp Arg Gly Leu Asp Phe Ile Asn Lys Asn Leu
        35                  40                  45

Ser Lys Leu Val Lys Lys Gly Lys Ile Glu Glu Ala Thr Lys Val Glu
    50                  55                  60

Ile Leu Thr Arg Ile Ser Gly Thr Val Asp Leu Asn Met Ala Ala Asp
65                  70                  75                  80

Cys Asp Leu Val Ile Glu Ala Ala Val Glu Arg Met Asp Ile Lys Lys
                85                  90                  95

Gln Ile Phe Ala Asp Leu Asp Asn Ile Cys Lys Pro Glu Thr Ile Leu
            100                 105                 110

Ala Ser Asn Thr Ser Ser Leu Ser Ile Thr Glu Val Ala Ser Ala Thr
        115                 120                 125

Lys Arg Pro Asp Lys Val Ile Gly Met His Phe Phe Asn Pro Ala Pro
    130                 135                 140

Val Met Lys Leu Val Glu Val Ile Arg Gly Ile Ala Thr Ser Gln Glu
145                 150                 155                 160

Thr Phe Asp Ala Val Lys Glu Thr Ser Ile Ala Ile Gly Lys Asp Pro
                165                 170                 175

Val Glu Val Ala Glu Ala Pro Gly Phe Val Val Asn Arg Ile Leu Ile
            180                 185                 190

Pro Met Ile Asn Glu Ala Val Gly Ile Leu Ala Glu Gly Ile Ala Ser
        195                 200                 205

Val Glu Asp Ile Asp Lys Ala Met Lys Leu Gly Ala Asn His Pro Met
    210                 215                 220

Gly Pro Leu Glu Leu Gly Asp Phe Ile Gly Leu Asp Ile Cys Leu Ala
225                 230                 235                 240

Ile Met Asp Val Leu Tyr Ser Glu Thr Gly Asp Ser Lys Tyr Arg Pro
                245                 250                 255

His Thr Leu Leu Lys Lys Tyr Val Arg Ala Gly Trp Leu Gly Arg Lys
            260                 265                 270

Ser Gly Lys Gly Phe Tyr Asp Tyr Ser Lys
        275                 280

<210> 20
<211> 849
<212> DNA
<213> Clostridium acetobutylicum

<400> 20
atgaaaaagg tatgtgttat aggtgcaggt actatgggtt caggaattgc tcaggcattt 60

gcagctaaag gatttgaagt agtattaaga gatattaaag atgaatttgt tgatagagga 120

ttagatttta tcaataaaaa tctttctaaa ttagttaaaa aaggaaagat agaagaagct 180

actaaagttg aaatcttaac tagaatttcc ggaacagttg accttaatat ggcagctgat 240

tgcgatttag ttatagaagc agctgttgaa agaatggata ttaaaaagca gatttttgct 300

gacttagaca atatatgcaa gccagaaaca attcttgcat caaatacatc atcactttca 360

ataacagaag tggcatcagc aactaaaaga cctgataagg ttataggtat gcatttcttt 420

aatccagctc ctgttatgaa gcttgtagag gtaataagag gaatagctac atcacaagaa 480

acttttgatg cagttaaaga gacatctata gcaataggaa aagatcctgt agaagtagca 540

gaagcaccag gatttgttgt aaatagaata ttaataccaa tgattaatga agcagttggt 600

atattagcag aaggaatagc ttcagtagaa gacatagata aagctatgaa acttggagct 660

aatcacccaa tgggaccatt agaattaggt gattttatag gtcttgatat atgtcttgct 720

ataatggatg ttttatactc agaaactgga gattctaagt atagaccaca tacattactt 780

aagaagtatg taagagcagg atggcttgga agaaaatcag gaaaaggttt ctacgattat 840

tcaaaataa 849

<210> 21
<211> 393
<212> PRT
<213> Ralstonia eutropha

<400> 21

Met Thr Asp Val Val Ile Val Ser Ala Ala Arg Thr Ala Val Gly Lys
1               5                   10                  15

Phe Gly Gly Ser Leu Ala Lys Ile Pro Ala Pro Glu Leu Gly Ala Val
            20                  25                  30

Val Ile Lys Ala Ala Leu Glu Arg Ala Gly Val Lys Pro Glu Gln Val
        35                  40                  45

Ser Glu Val Ile Met Gly Gln Val Leu Thr Ala Gly Ser Gly Gln Asn
    50                  55                  60

Pro Ala Arg Gln Ala Ala Ile Lys Ala Gly Leu Pro Ala Met Val Pro
65                  70                  75                  80

Ala Met Thr Ile Asn Lys Val Cys Gly Ser Gly Leu Lys Ala Val Met

                85                  90                  95

Leu Ala Ala Asn Ala Ile Met Ala Gly Asp Ala Glu Ile Val Val Ala
            100                 105                 110

Gly Gly Gln Glu Asn Met Ser Ala Ala Pro His Val Leu Pro Gly Ser
        115                 120                 125

Arg Asp Gly Phe Arg Met Gly Asp Ala Lys Leu Val Asp Thr Met Ile
    130                 135                 140

Val Asp Gly Leu Trp Asp Val Tyr Asn Gln Tyr His Met Gly Ile Thr
145                 150                 155                 160

Ala Glu Asn Val Ala Lys Glu Tyr Gly Ile Thr Arg Glu Ala Gln Asp
                165                 170                 175

Glu Phe Ala Val Gly Ser Gln Asn Lys Ala Glu Ala Ala Gln Lys Ala
            180                 185                 190

Gly Lys Phe Asp Glu Glu Ile Val Pro Val Leu Ile Pro Gln Arg Lys
        195                 200                 205

Gly Asp Pro Val Ala Phe Lys Thr Asp Glu Phe Val Arg Gln Gly Ala
    210                 215                 220

Thr Leu Asp Ser Met Ser Gly Leu Lys Pro Ala Phe Asp Lys Ala Gly
225                 230                 235                 240

Thr Val Thr Ala Ala Asn Ala Ser Gly Leu Asn Asp Gly Ala Ala Ala
                245                 250                 255

Val Val Val Met Ser Ala Ala Lys Ala Lys Glu Leu Gly Leu Thr Pro
            260                 265                 270

Leu Ala Thr Ile Lys Ser Tyr Ala Asn Ala Gly Val Asp Pro Lys Val
        275                 280                 285

Met Gly Met Gly Pro Val Pro Ala Ser Lys Arg Ala Leu Ser Arg Ala
    290                 295                 300

Glu Trp Thr Pro Gln Asp Leu Asp Leu Met Glu Ile Asn Glu Ala Phe
305                 310                 315                 320

Ala Ala Gln Ala Leu Ala Val His Gln Gln Met Gly Trp Asp Thr Ser
                325                 330                 335

Lys Val Asn Val Asn Gly Gly Ala Ile Ala Ile Gly His Pro Ile Gly
            340                 345                 350

Ala Ser Gly Cys Arg Ile Leu Val Thr Leu Leu His Glu Met Lys Arg
        355                 360                 365

Arg Asp Ala Lys Lys Gly Leu Ala Ser Leu Cys Ile Gly Gly Gly Met
    370                 375                 380

Gly Val Ala Leu Ala Val Glu Arg Lys
385                 390

<210>  22
<211>  1182
<212>  DNA
<213>  Ralstonia eutropha

<400>  22
atgactgacg ttgtcatcgt atccgccgcc cgcaccgcgg tcggcaagtt tggcggctcg      60

ctggccaaga tcccggcacc ggaactgggt gccgtggtca tcaaggccgc gctggagcgc     120

gccggcgtca agccggagca ggtgagcgaa gtcatcatgg gccaggtgct gaccgccggt     180

tcgggccaga accccgcacg ccaggccgcg atcaaggccg gcctgccggc gatggtgccg     240

gccatgacca tcaacaaggt gtgcggctcg ggcctgaagg ccgtgatgct ggccgccaac     300

gcgatcatgg cgggcgacgc cgagatcgtg gtggccggcg gccaggaaaa catgagcgcc     360

gccccgcacg tgctgccggg ctcgcgcgat ggtttccgca tgggcgatgc caagctggtc     420

gacaccatga tcgtcgacgg cctgtgggac gtgtacaacc agtaccacat gggcatcacc     480

gccgagaacg tggccaagga atacggcatc acacgcgagg cgcaggatga gttcgccgtc     540

ggctcgcaga acaaggccga agccgcgcag aaggccggca agtttgacga agagatcgtc     600

ccggtgctga tcccgcagcg caagggcgac ccggtggcct tcaagaccga cgagttcgtg     660

cgccagggcg ccacgctgga cagcatgtcc ggcctcaagc ccgccttcga caaggccggc     720

acggtgaccg cggccaacgc ctcgggcctg aacgacggcg ccgccgcggt ggtggtgatg     780

tcggcggcca aggccaagga actgggcctg accccgctgg ccacgatcaa gagctatgcc     840

aacgccggtg tcgatcccaa ggtgatgggc atgggcccgg tgccggcctc caagcgcgcc     900

ctgtcgcgcg ccgagtggac cccgcaagac ctggacctga tggagatcaa cgaggccttt     960

gccgcgcagg cgctggcggt gcaccagcag atgggctggg acacctccaa ggtcaatgtg    1020

aacggcggcg ccatcgccat cggccacccg atcggcgcgt cgggctgccg tatcctggtg    1080

acgctgctgc acgagatgaa gcgccgtgac gcgaagaagg gcctggcctc gctgtgcatc    1140

ggcggcggca tgggcgtggc gctggcagtc gagcgcaaat aa                       1182

<210>  23
<211>  393
<212>  PRT
<213>  Zoogloea ramigera

<400>  23

Met Ser Thr Pro Ser Ile Val Ile Ala Ser Ala Ala Arg Thr Ala Val
1               5                   10                  15

Gly Ser Phe Asn Gly Ala Phe Ala Asn Thr Pro Ala His Glu Leu Gly
            20                  25                  30

Ala Thr Val Ile Ser Ala Val Leu Glu Arg Ala Gly Val Ala Ala Gly
        35                  40                  45

Glu Val Asn Glu Val Ile Leu Gly Gln Val Leu Pro Ala Gly Glu Gly
    50                  55                  60

Gln Asn Pro Ala Arg Gln Ala Ala Met Lys Ala Gly Val Pro Gln Glu
65                  70                  75                  80

Ala Thr Ala Trp Gly Met Asn Gln Leu Cys Gly Ser Gly Leu Arg Ala
                85                  90                  95

Val Ala Leu Gly Met Gln Gln Ile Ala Thr Gly Asp Ala Ser Ile Ile
            100                 105                 110

Val Ala Gly Gly Met Glu Ser Met Ser Met Ala Pro His Cys Ala His
        115                 120                 125

Leu Arg Gly Gly Val Lys Met Gly Asp Phe Lys Met Ile Asp Thr Met
    130                 135                 140

Ile Lys Asp Gly Leu Thr Asp Ala Phe Tyr Gly Tyr His Met Gly Thr
145                 150                 155                 160

Thr Ala Glu Asn Val Ala Lys Gln Trp Gln Leu Ser Arg Asp Glu Gln
                165                 170                 175

Asp Ala Phe Ala Val Ala Ser Gln Asn Lys Ala Glu Ala Ala Gln Lys
            180                 185                 190

Asp Gly Arg Phe Lys Asp Glu Ile Val Pro Phe Ile Val Lys Gly Arg
        195                 200                 205

Lys Gly Asp Ile Thr Val Asp Ala Asp Glu Tyr Ile Arg His Gly Ala
    210                 215                 220

Thr Leu Asp Ser Met Ala Lys Leu Arg Pro Ala Phe Asp Lys Glu Gly
225                 230                 235                 240

Thr Val Thr Ala Gly Asn Ala Ser Gly Leu Asn Asp Gly Ala Ala Ala
                245                 250                 255

Ala Leu Leu Met Ser Glu Ala Glu Ala Ser Arg Arg Gly Ile Gln Pro
            260                 265                 270

Leu Gly Arg Ile Val Ser Trp Ala Thr Val Gly Val Asp Pro Lys Val
        275                 280                 285

Met Gly Thr Gly Pro Ile Pro Ala Ser Arg Lys Ala Leu Glu Arg Ala
    290                 295                 300

Gly Trp Lys Ile Gly Asp Leu Asp Leu Val Glu Ala Asn Glu Ala Phe
305                 310                 315                 320

Ala Ala Gln Ala Cys Ala Val Asn Lys Asp Leu Gly Trp Asp Pro Ser
                325                 330                 335

Ile Val Asn Val Asn Gly Gly Ala Ile Ala Ile Gly His Pro Ile Gly
            340                 345                 350

Ala Ser Gly Ala Arg Ile Leu Asn Thr Leu Leu Phe Glu Met Lys Arg
        355                 360                 365

Arg Gly Ala Arg Lys Gly Leu Ala Thr Leu Cys Ile Gly Gly Gly Met
    370                 375                 380

Gly Val Ala Met Cys Ile Glu Ser Leu
385                 390

<210>  24
<211>  1182
<212>  DNA
<213>  Zoogloea ramigera

<400>  24
atgagtactc catcaatcgt catcgccagc gccgcccgca ccgcggtcgg ttccttcaac      60

ggcgctttcg ccaacacgcc cgcccatgaa ctcggggcga ccgtgatttc ggcggttctc     120

gagcgcgcgg gcgttgcggc gggcgaggtg aacgaggtga ttctcggcca ggtgctgccg     180

gccggcgaag gccagaaccc ggcccgccag gccgccatga aggccggcgt gccgcaggag     240

gcgaccgcct ggggcatgaa ccagctttgc ggctcgggcc tgcgcgccgt cgcgctcggc     300

atgcagcaga tcgccacggg cgatgcgagc atcatcgtcg ccggcggcat ggaatccatg     360

tcaatggccc cgcattgcgc gcatctgcgc ggcggcgtga agatgggcga tttcaagatg     420

atcgacacga tgatcaagga cggcctgacc gacgccttct acggctacca catgggcacg     480

accgccgaga atgtcgccaa gcagtggcag ctttcccgcg acgagcagga cgccttcgcc     540

gtcgcctcgc agaacaaggc cgaggccgcc cagaaggacg gccgcttcaa ggacgagatc     600

gttcccttca tcgtcaaggg ccgcaagggc gacatcacgg tcgatgccga cgaatatatc     660

cgccacggcg cgacgctcga ttcaatggcg aagctccgcc cggccttcga caaggaaggc 720

acggtgacgg ccggcaacgc ctccggcctc aatgacggcg cggccgcggc cctcctgatg 780

agcgaagcgg aagcctcgcg ccgcggcatc cagccgctcg gccgcatcgt ttcctgggcg 840

acggtcggcg tcgatcccaa ggtcatgggc accggcccga tcccggcctc ccgcaaggcg 900

ctcgagcgcg ccggctggaa gatcggcgat ctcgacctcg tggaagccaa cgaagccttc 960

gcggcgcagg cctgcgcggt caacaaggac ctcggctggg atccgtccat cgtcaacgtc 1020

aacggcggtg ccatcgccat cggccacccg atcggcgcgt ccggcgcccg catcctcaac 1080

acgctcctct tcgagatgaa gcgtcgcggc gcccgcaagg gtctcgccac gctctgcatc 1140

ggcggcggca tgggcgtggc gatgtgcatc gaaagtcttt aa 1182

<210> 25
<211> 394
<212> PRT
<213> Escherichia coli

<400> 25

Met Lys Asn Cys Val Ile Val Ser Ala Val Arg Thr Ala Ile Gly Ser
1               5                   10                  15

Phe Asn Gly Ser Leu Ala Ser Thr Ser Ala Ile Asp Leu Gly Ala Thr
            20                  25                  30

Val Ile Lys Ala Ala Ile Glu Arg Ala Lys Ile Asp Ser Gln His Val
        35                  40                  45

Asp Glu Val Ile Met Gly Asn Val Leu Gln Ala Gly Leu Gly Gln Asn
    50                  55                  60

Pro Ala Arg Gln Ala Leu Leu Lys Ser Gly Leu Ala Glu Thr Val Cys
65                  70                  75                  80

Gly Phe Thr Val Asn Lys Val Cys Gly Ser Gly Leu Lys Ser Val Ala
                85                  90                  95

Leu Ala Ala Gln Ala Ile Gln Ala Gly Gln Ala Gln Ser Ile Val Ala
            100                 105                 110

Gly Gly Met Glu Asn Met Ser Leu Ala Pro Tyr Leu Leu Asp Ala Lys
        115                 120                 125

Ala Arg Ser Gly Tyr Arg Leu Gly Asp Gly Gln Val Tyr Asp Val Ile
    130                 135                 140

Leu Arg Asp Gly Leu Met Cys Ala Thr His Gly Tyr His Met Gly Ile
145                 150                 155                 160

Thr Ala Glu Asn Val Ala Lys Glu Tyr Gly Ile Thr Arg Glu Met Gln
                165                 170                 175

Asp Glu Leu Ala Leu His Ser Gln Arg Lys Ala Ala Ala Ala Ile Glu
            180                 185                 190

Ser Gly Ala Phe Thr Ala Glu Ile Val Pro Val Asn Val Val Thr Arg
        195                 200                 205

Lys Lys Thr Phe Val Phe Ser Gln Asp Glu Phe Pro Lys Ala Asn Ser
    210                 215                 220

Thr Ala Glu Ala Leu Gly Ala Leu Arg Pro Ala Phe Asp Lys Ala Gly
225                 230                 235                 240

Thr Val Thr Ala Gly Asn Ala Ser Gly Ile Asn Asp Gly Ala Ala Ala
                245                 250                 255

Leu Val Ile Met Glu Glu Ser Ala Ala Leu Ala Ala Gly Leu Thr Pro
            260                 265                 270

Leu Ala Arg Ile Lys Ser Tyr Ala Ser Gly Gly Val Pro Pro Ala Leu
        275                 280                 285

Met Gly Met Gly Pro Val Pro Ala Thr Gln Lys Ala Leu Gln Leu Ala
    290                 295                 300

Gly Leu Gln Leu Ala Asp Ile Asp Leu Ile Glu Ala Asn Glu Ala Phe
305                 310                 315                 320

Ala Ala Gln Phe Leu Ala Val Gly Lys Asn Leu Gly Phe Asp Ser Glu
                325                 330                 335

Lys Val Asn Val Asn Gly Gly Ala Ile Ala Leu Gly His Pro Ile Gly
            340                 345                 350

Ala Ser Gly Ala Arg Ile Leu Val Thr Leu Leu His Ala Met Gln Ala
        355                 360                 365

Arg Asp Lys Thr Leu Gly Leu Ala Thr Leu Cys Ile Gly Gly Gly Gln
    370                 375                 380

Gly Ile Ala Met Val Ile Glu Arg Leu Asn
385                 390

<210>  26
<211>  1185
<212>  DNA

<213>  Escherichia coli

<400>  26
   atgaaaaatt gtgtcatcgt cagtgcggta cgtactgcta tcggtagttt taacggttca      60
   ctcgcttcca ccagcgccat cgacctgggg gcgacagtaa ttaaagccgc cattgaacgt     120
   gcaaaaatcg attcacaaca cgttgatgaa gtgattatgg gtaacgtgtt acaagccggg     180
   ctggggcaaa atccggcgcg tcaggcactg ttaaaaagcg ggctggcaga aacggtgtgc     240
   ggattcacgg tcaataaagt atgtggttcg ggtcttaaaa gtgtggcgct tgccgcccag     300
   gccattcagg caggtcaggc gcagagcatt gtggcggggg gtatggaaaa tatgagttta     360
   gccccctact tactcgatgc aaaagcacgc tctggttatc gtcttggaga cggacaggtt     420
   tatgacgtaa tcctgcgcga tggcctgatg tgcgccaccc atggttatca tatggggatt     480
   accgccgaaa acgtggctaa agagtacgga attacccgtg aaatgcagga tgaactggcg     540
   ctacattcac agcgtaaagc ggcagccgca attgagtccg gtgcttttac agccgaaatc     600
   gtcccggtaa atgttgtcac tcgaaagaaa accttcgtct tcagtcaaga cgaattcccg     660
   aaagcgaatt caacggctga agcgttaggt gcattgcgcc cggccttcga taaagcagga     720
   acagtcaccg ctgggaacgc gtctggtatt aacgacggtg ctgccgctct ggtgattatg     780
   gaagaatctg cggcgctggc agcaggcctt acccccctgg ctcgcattaa aagttatgcc     840
   agcggtggcg tgccccccgc attgatgggt atggggccag tacctgccac gcaaaaagcg     900
   ttacaactgg cggggctgca actggcggat attgatctca ttgaggctaa tgaagcattt     960
   gctgcacagt tccttgccgt tgggaaaaac ctgggctttg attctgagaa agtgaatgtc    1020
   aacggcgggg ccatcgcgct cgggcatcct atcggtgcca gtggtgctcg tattctggtc    1080
   acactattac atgccatgca ggcacgcgat aaaacgctgg ggctggcaac actgtgcatt    1140
   ggcggcggtc agggaattgc gatggtgatt gaacgcttga attaa                    1185


<210>  27
<211>  38
<212>  DNA
<213>  Artificial

<220>
<223>  An artificially synthesized primer sequence

<400>  27
gacggtacct atatatgact gatgttgtca tcgtatcc                              38


<210>  28
<211>  34
<212>  DNA
<213>  Artificial

<220>
<223>  An artificially synthesized primer sequence

<400>  28

cacaagctta ttatttacgt tcaactgcca gcgc                                    34

<210>  29
<211>  35
<212>  DNA
<213>  Artificial

<220>
<223>  An artificially synthesized primer sequence

<400>  29
gacggtacct atatatgagt actccatcaa tcgtc                                   35

<210>  30
<211>  34
<212>  DNA
<213>  Artificial

<220>
<223>  An artificially synthesized primer sequence

<400>  30
cacaagctta ttaaagactt tcgatgcaca tcgc                                    34

<210>  31
<211>  22
<212>  DNA
<213>  Artificial

<220>
<223>  An artificially synthesized primer sequence

<400>  31
ggaatccatg tcaatggccc cg                                                 22

<210>  32
<211>  20
<212>  DNA
<213>  Artificial

<220>
<223>  An artificially synthesized primer sequence

<400>  32
gctcgattca atggcgaagc                                                    20

<210>  33
<211>  22
<212>  DNA
<213>  Artificial

<220>
<223>  An artificially synthesized primer sequence

<400>  33
caatgcgggg ccattgacat gg                                                 22

<210>  34
<211>  23

<212>  DNA
<213>  Artificial

<220>
<223>  An artificially synthesized primer sequence

<400>  34
cggagcttcg ccattgaatc gag                                              23


<210>  35
<211>  37
<212>  DNA
<213>  Artificial

<220>
<223>  An artificially synthesized primer sequence

<400>  35
gacggtacct atatatgaaa aattgtgtca tcgtcag                               37


<210>  36
<211>  35
<212>  DNA
<213>  Artificial

<220>
<223>  An artificially synthesized primer sequence

<400>  36
cacaagctta ttaattcaag cgttcaatca ccatc                                 35


<210>  37
<211>  37
<212>  DNA
<213>  Artificial

<220>
<223>  An artificially synthesized primer sequence

<400>  37
gaggaattca tatatgactc aacgtattgc gtatgtg                               37


<210>  38
<211>  29
<212>  DNA
<213>  Artificial

<220>
<223>  An artificially synthesized primer sequence

<400>  38
cagactagta ttagcccatg tgcaggccg                                        29


<210>  39
<211>  23
<212>  DNA
<213>  Artificial

<220>
<223>  An artificially synthesized primer sequence

<400>  39
catggcttca ctatggcact ggc                                              23


<210>  40
<211>  23
<212>  DNA
<213>  Artificial

<220>
<223>  An artificially synthesized primer sequence

<400>  40
gccagtgcca tagtgaagcc atg                                              23


<210>  41
<211>  36
<212>  DNA
<213>  Artificial

<220>
<223>  An artificially synthesized primer sequence

<400>  41
gaggaattca tatatgagtc gtgtagcatt ggtaac                                36


<210>  42
<211>  29
<212>  DNA
<213>  Artificial

<220>
<223>  An artificially synthesized primer sequence

<400>  42
cagactagta ttagacgaag aactggccg                                        29


<210>  43
<211>  31
<212>  DNA
<213>  Artificial

<220>
<223>  An artificially synthesized primer sequence

<400>  43
gaggaattca tatatggcca cgcaggactc c                                     31


<210>  44
<211>  29
<212>  DNA
<213>  Artificial

<220>
<223>  An artificially synthesized primer sequence

<400>  44
cagactagta ttagtagttc cccagcccg                                        29

<210>  45
<211>  35
<212>  DNA
<213>  Artificial

<220>
<223>  An artificially synthesized primer sequence

<400>  45
gaggaattca tatatgtctc aacgttttgc aggtc                                35


<210>  46
<211>  31
<212>  DNA
<213>  Artificial

<220>
<223>  An artificially synthesized primer sequence

<400>  46
cagactagta ttaacatttt ggaccacctg c                                    31


<210>  47
<211>  39
<212>  DNA
<213>  Artificial

<220>
<223>  An artificially synthesized primer sequence

<400>  47
gaggaattca tatatgaaaa aggtatgtgt tataggtgc                            39


<210>  48
<211>  40
<212>  DNA
<213>  Artificial

<220>
<223>  An artificially synthesized primer sequence

<400>  48
cagactagta ttattttgaa taatcgtaga aaccttttcc                          40


<210>  49
<211>  42
<212>  DNA
<213>  Artificial

<220>
<223>  An artificially synthesized primer sequence

<400>  49
gaggaattct aaaatgtctt ataatttcca taacaaggtt gc                        42


<210>  50
<211>  38
<212>  DNA
<213>  Artificial

<220>
<223>  An artificially synthesized primer sequence

<400>  50
tcgactagta ttattgtgct gtgtacccac cgtcaacc                              38


<210>  51
<211>  39
<212>  DNA
<213>  Artificial

<220>
<223>  An artificially synthesized primer sequence

<400>  51
ggctctggag tacgcatctc atggtattcg tgtaaattc                             39


<210>  52
<211>  39
<212>  DNA
<213>  Artificial

<220>
<223>  An artificially synthesized primer sequence

<400>  52
gaatttacac gaataccatg agatgcgtac tccagagcc                             39


<210>  53
<211>  34
<212>  DNA
<213>  Artificial

<220>
<223>  An artificially synthesized primer sequence

<400>  53
gtaagcctgc accctattgg gcgtctgggt cgtc                                  34


<210>  54
<211>  34
<212>  DNA
<213>  Artificial

<220>
<223>  An artificially synthesized primer sequence

<400>  54
gacgacccag acgcccaata gggtgcaggc ttac                                  34


<210>  55
<211>  37
<212>  DNA
<213>  Artificial

<220>
<223>  An artificially synthesized primer sequence

<400>  55
gaccatatga aagttacaaa tcaaaaagaa ctaaaac                               37

<210>  56
<211>  40
<212>  DNA
<213>  Artificial

<220>
<223>  An artificially synthesized primer sequence

<400>  56
ctgttaatta aaatgatttt atatagatat ccttaagttc                              40


<210>  57
<211>  21
<212>  DNA
<213>  Artificial

<220>
<223>  An artificially synthesized primer sequence

<400>  57
ctatagaagc atacgtttcg g                                                  21


<210>  58
<211>  21
<212>  DNA
<213>  Artificial

<220>
<223>  An artificially synthesized primer sequence

<400>  58
ccgaaacgta tgcttctata g                                                  21


<210>  59
<211>  39
<212>  DNA
<213>  Artificial

<220>
<223>  An artificially synthesized primer sequence

<400>  59
ggaccatggt tgatttcgaa tattcaatac caactagaa                               39


<210>  60
<211>  47
<212>  DNA
<213>  Artificial

<220>
<223>  An artificially synthesized primer sequence

<400>  60
cgatctagaa ttacacagat tttttgaata tttgtaggac ttcggag                      47


<210>  61
<211>  24
<212>  DNA

<213>  Artificial

<220>
<223>  An artificially synthesized primer sequence

<400>  61
gatggaaatc cgtgggatat tgtg                                              24


<210>  62
<211>  24
<212>  DNA
<213>  Artificial

<220>
<223>  An artificially synthesized primer sequence

<400>  62
cacaatatcc cacggatttc catc                                              24


<210>  63
<211>  27
<212>  DNA
<213>  Artificial

<220>
<223>  An artificially synthesized primer sequence

<400>  63
gtttaccatc tcgtctgcgt gatgttg                                           27


<210>  64
<211>  27
<212>  DNA
<213>  Artificial

<220>
<223>  An artificially synthesized primer sequence

<400>  64
caacatcacg cagacgagat ggtaaac                                           27


<210>  65
<211>  874
<212>  PRT
<213>  Thermoanaerobacter pseudethanolicus

<400>  65

Met Pro Asn Leu Leu Gln Glu Arg Arg Glu Val Lys Glu Lys Thr Glu
1               5                   10                  15


Val Lys Glu Thr Leu Asp Val Lys Arg Gln Ile Asp Gln Leu Val Glu
            20                  25                  30


Lys Ala Gln Arg Ala Gln Glu Lys Phe Met Ser Tyr Thr Gln Glu Gln
        35                  40                  45


Ile Asp Lys Ile Val Lys Ala Met Ala Leu Ala Gly Ile Glu Asn His

50                  55                  60

Val Arg Leu Ala Lys Leu Ala His Glu Glu Thr Lys Met Gly Val Tyr
65                  70                  75                  80

Glu Asp Lys Ile Thr Lys Asn Leu Phe Ala Val Glu Tyr Val Tyr Asn
                85                  90                  95

Tyr Ile Lys Asp Lys Lys Thr Val Gly Val Leu Ser Glu Asn Leu Glu
            100                 105                 110

Glu Asn Tyr Met Glu Val Ala Glu Pro Val Gly Val Ile Ala Gly Val
        115                 120                 125

Thr Pro Val Thr Asn Pro Thr Ser Thr Thr Met Phe Lys Ser Leu Ile
    130                 135                 140

Ala Ile Lys Thr Arg Asn Pro Ile Ile Phe Ser Phe His Pro Lys Ala
145                 150                 155                 160

Leu Lys Cys Ser Ile Glu Thr Ala Lys Thr Met Tyr Glu Ala Ala Leu
                165                 170                 175

Lys Ala Gly Ala Pro Glu Gly Cys Ile Gly Trp Ile Glu Thr Pro Ser
            180                 185                 190

Ile Glu Ala Thr Gln Leu Leu Met Thr His Pro Gly Val Ser Leu Ile
        195                 200                 205

Leu Ala Thr Gly Gly Ala Gly Met Val Lys Ala Ala Tyr Ser Ser Gly
    210                 215                 220

Lys Pro Ala Leu Gly Val Gly Pro Gly Asn Val Pro Cys Tyr Ile Glu
225                 230                 235                 240

Lys Thr Ala Asn Ile Lys Arg Ala Val Ser Asp Leu Ile Leu Ser Lys
                245                 250                 255

Thr Phe Asp Asn Gly Thr Val Cys Ala Ser Glu Gln Ala Val Ile Ile
            260                 265                 270

Asp Glu Glu Ile Ala Asp Glu Val Lys Lys Leu Met Lys Glu Tyr Gly
        275                 280                 285

Cys Tyr Phe Leu Asn Lys Glu Glu Thr Lys Met Leu Glu Glu Phe Ala
    290                 295                 300

Ile Asp Lys Asn Thr Gly Leu Met Asn Pro Ala Val Val Gly Gln Pro
305                 310                 315                 320

Ala Thr Lys Ile Ala Glu Met Ala Gly Phe Lys Val Pro Glu Asn Thr
                325                 330                 335

Lys Ile Leu Val Ala Glu Tyr Pro Ala Val Gly Pro Glu Tyr Pro Leu
            340                 345                 350

Ser Arg Glu Lys Leu Ser Pro Ile Leu Ala Leu Tyr Thr Val Lys Asp
        355                 360                 365

Tyr Asn Glu Gly Ile Lys Arg Cys Glu Glu Met Thr Glu Phe Gly Gly
    370                 375                 380

Leu Gly His Ser Ala Val Ile His Ser Glu Asn Gln Gln Ile Ile Asn
385                 390                 395                 400

Glu Phe Ala Lys Arg Val Arg Ala Gly Arg Ile Leu Val Asn Ala Pro
                405                 410                 415

Ala Ser Gln Gly Ala Ile Gly Asp Ile Tyr Asn Thr Ala Ile Pro Ser
            420                 425                 430

Leu Thr Leu Gly Cys Gly Thr Met Gly Arg Asn Ser Thr Thr Asp Asn
        435                 440                 445

Val Ser Val Tyr Asn Leu Ile Asn Ile Lys Arg Val Phe Ile Arg Lys
    450                 455                 460

Glu Arg Met Lys Trp Phe Arg Val Pro Pro Gln Ile Tyr Phe Glu Arg
465                 470                 475                 480

Gly Ser Leu Gln Tyr Leu Ser Gln Val Lys Gly Lys Lys Ala Phe Ile
                485                 490                 495

Val Thr Asp Pro Val Met Val Lys Leu Gly Phe Val Asp Lys Val Thr
            500                 505                 510

Tyr Gln Leu Asp Lys Ala Asn Ile Lys Tyr Glu Ile Phe Ser Glu Val
        515                 520                 525

Glu Pro Asp Pro Ser Val Asp Thr Val Glu Lys Gly Val Lys Ile Met
    530                 535                 540

Arg Gly Phe Glu Pro Asp Leu Leu Ile Ala Val Gly Gly Gly Ser Ala
545                 550                 555                 560

Ile Asp Ala Ala Lys Gly Met Trp Leu Phe Tyr Glu Tyr Pro Asp Thr
                565                 570                 575

Lys Phe Glu Asp Leu Arg Leu Lys Phe Met Asp Ile Arg Lys Arg Thr
            580                 585                 590

Tyr Arg Phe Pro Glu Leu Gly Lys Lys Ala Leu Phe Ile Ala Ile Pro
        595                 600                 605

Thr Thr Ser Gly Ser Gly Thr Gly Ser Glu Val Thr Ala Phe Ala Val
    610                 615                 620

Ile Thr Asp Lys Lys Lys Asn Ile Lys Tyr Pro Leu Thr Asp Tyr Glu
625                 630                 635                 640

Leu Thr Pro Asp Ile Ala Ile Ile Asp Pro Asp Leu Thr Met Thr Ile
                645                 650                 655

Pro Pro Ser Val Thr Ala Asp Thr Gly Met Asp Ala Leu Thr His Ala
            660                 665                 670

Ile Glu Ala Tyr Val Ser Val Met Ala Ser Asp Tyr Thr Asp Ala Leu
        675                 680                 685

Ala Glu Lys Ala Ile Lys Leu Ile Phe Glu Tyr Leu Pro Lys Ala Tyr
    690                 695                 700

Lys Asn Gly Gln Asp Lys Val Ala Arg Glu Lys Met His Asn Ala Ser
705                 710                 715                 720

Cys Ile Ala Gly Met Ala Phe Thr Asn Ala Phe Leu Gly Ile Asn His
                725                 730                 735

Ser Met Ala His Ile Leu Gly Ala Lys Phe His Leu Pro His Gly Arg
            740                 745                 750

Ala Asn Ala Ile Leu Leu Pro Tyr Val Ile Glu Tyr Asn Ala Glu Leu
        755                 760                 765

Pro Lys Lys Phe Ala Ser Phe Pro Gln Tyr Glu Tyr Pro Lys Ala Ala
    770                 775                 780

Glu Lys Tyr Ala Glu Ile Ala Lys Phe Leu Gly Leu Pro Ala Ser Thr
785                 790                 795                 800

Ile Glu Glu Gly Val Lys Ser Leu Ile Glu Ala Ile Lys Asn Leu Met
                805                 810                 815

Lys Glu Leu Asn Leu Pro Leu Thr Leu Lys Glu Ala Gly Ile Asn Lys
            820                 825                 830

Glu Glu Phe Glu Lys Gln Ile Met Glu Met Ser Asp Ile Ala Phe Asn
        835                 840                 845

Asp Gln Cys Thr Gly Ser Asn Pro Arg Met Pro Leu Val Ser Glu Ile
    850                 855                 860

Ala Glu Ile Tyr Arg Lys Ala Tyr Gly Glu
865                 870

<210>  66
<211>  2625
<212>  DNA
<213>  Thermoanaerobacter pseudethanolicus

<400>  66
atgcctaact tattacaaga aagaagagaa gtaaaagaaa aaacagaagt gaaagaaacg      60

ctggatgtta aaagacaaat agaccaatta gtagaaaaag cacaaagagc acaagaaaaa     120

ttcatgtctt atacccaaga acaaatagat aaaatagtta aagctatggc tttggctggt     180

attgaaaatc atgtgagatt ggcaaaatta gcccatgaag aaacaaaaat gggtgtttat     240

gaagacaaaa taacgaaaaa tctttttgca gtagaatatg tgtacaatta tataaaagac     300

aaaaagactg tcggcgtcct tagtgaaaac ttagaagaaa attatatgga agtggcagaa     360

cctgtaggag ttatagccgg cgtaacaccc gttacaaacc ctacttctac aacaatgttc     420

aaaagcttaa tcgctattaa aacaagaaat ccaataattt ttagctttca cccaaaagca     480

ttaaaatgta gtatagaaac agcaaagacc atgtacgagg cagcattaaa agctggtgca     540

ccagaaggat gcataggttg gattgaaacg ccttcaattg aagcaacgca acttctcatg     600

acacatcccg gcgtctccct tatcctcgca acaggcggcg ctggaatggt aaaagccgct     660

tacagctcag gaaaacccgc ccttggcgta ggtcctggca atgtcccctg ctacattgaa     720

aaaactgcaa atattaaaag agcagtctca gacctaatac ttagcaaaac ttttgacaat     780

ggcacagtat gcgcatcaga acaagctgtg ataatagacg aagaaatagc agatgaagtg     840

aaaaaattaa tgaaagaata tggttgctat tttctcaata aagaagagac taaaatgcta     900

gaagaattcg ctatagacaa aaacaccggt ttaatgaatc cagcagttgt aggtcaaccg     960

gcaacaaaaa ttgctgaaat ggctggattt aaagtacctg aaaacacaaa aatacttgta    1020

gctgaatatc ctgctgtagg accagaatat cctctctcca gagaaaagct aagtccaata    1080

cttgctttat acacagtcaa agattacaac gaaggcataa aaagatgtga agaaatgaca    1140

gaattcggcg gacttggtca ctctgcagta atacattctg aaaaccaaca aattattaat    1200

gaatttgcaa aaagagtacg agcaggaaga attcttgtaa acgcccctgc ttctcaagga    1260

gctattggag atatttataa cacggctatc ccatctttga ctttgggttg cggtacaatg    1320

ggtagaaatt caacaacaga taatgtaagt gtttataacc ttataaacat aaaacgagtt    1380

tttataagaa aagagagaat gaaatggttc agagttccac cacaaattta ttttgaaaga    1440

gggtcattac aatatctgtc acaagtaaaa ggcaaaaaag cctttattgt aacagaccct 1500

gtgatggtta aattaggttt tgtagataaa gtaacgtatc aattagataa ggcaaatatt 1560

aaatatgaaa tattctctga agttgaacca gatccttcag ttgacactgt agaaaaaggg 1620

gtaaaaataa tgagagggtt tgagcctgac ttactaatag ctgtaggtgg tggttctgca 1680

atagatgcag cgaagggaat gtggcttttc tacgaatacc ctgatacaaa attcgaagac 1740

ttaaggttaa aatttatgga cataagaaaa agaacttata gatttcctga actggggaaa 1800

aaagctttat ttatcgcaat accaaccacc agcggtagcg gtacaggttc tgaagttact 1860

gcctttgcag ttataacaga caaaaagaaa aatataaagt accctctcac agattacgaa 1920

ttaacaccag atatcgctat aatagaccct gacctcacaa tgacaatacc accctctgtc 1980

acagcagaca ctggtatgga cgctttaacc catgctattg aagcctatgt atctgtaatg 2040

gcatcagact atacagacgc tctcgcagaa aaagctataa aattgatatt tgaatatcta 2100

cctaaagcat acaaaaacgg gcaagataaa gtcgcccgcg aaaagatgca caatgcttca 2160

tgcatagctg gaatggcctt cacaaatgca tttttaggaa taaaccatag tatggctcat 2220

atacttggtg ctaaattcca tcttccacac ggaagagcaa atgcaatact tctgccctat 2280

gtcatagaat acaatgctga gttacctaaa aaatttgctt cattcccaca atatgaatat 2340

cccaaagcag ccgagaaata tgcagaaatt gcaaaattct tgggacttcc tgcatcaacc 2400

attgaagaag gagtaaaaag cctcattgaa gcaataaaaa atcttatgaa agagcttaat 2460

cttcctttga cacttaaaga agcaggcata aataaagaag aattcgaaaa acaaataatg 2520

gaaatgtctg atatagcctt taatgaccag tgcaccggtt caaacccaag aatgccattg 2580

gtatccgaaa ttgcagaaat atatagaaaa gcctatggag aataa 2625

<210> 67
<211> 378
<212> PRT
<213> Propionibacterium freudenreichii subsp. freudenreichii

<400> 67

Met Asp Phe Ser Leu Thr Glu Asp Gln Gln Leu Met Val Asp Gly Phe
1               5                   10                  15

Thr Glu Leu Met Asn Ser Glu Ala Trp Glu Lys Tyr Phe His Glu Cys
            20                  25                  30

Asp Glu Asn Ser Glu Tyr Pro Glu Arg Trp Val Lys Ala Ile Cys Asp
        35                  40                  45

Leu Gly Phe Asp Arg Ile Leu Leu Pro Glu Glu Tyr Asp Gly Leu Gly
    50                  55                  60

Leu Gly Trp Gln Thr Leu Ala Ala Ala Tyr Glu Ala Leu Gly Arg Ala
65                  70                  75                  80

Gly Gly Pro Thr Tyr Val Leu Tyr Gln Leu Pro Gly Trp Asp Thr Val
                85                  90                  95

Ile Arg Glu Gly Thr Glu Glu Gln Lys Lys Asp Ile Leu Lys Phe Val
            100                 105                 110

Gly Ser Gly Lys Gln Met Leu Asn Tyr Ala Met Thr Glu Pro Ser Ala
        115                 120                 125

Gly Ser Ser Trp Asp Asp Met Ser Thr Thr Tyr Ser Arg Lys Gly Gly
    130                 135                 140

Lys Val Tyr Leu Asn Gly His Lys Thr Phe Ile Thr Ser Ser Met Lys
145                 150                 155                 160

Val Pro Tyr Leu Val Val Met Ala Arg Asp Ala Asp Asn Met Gly Thr
                165                 170                 175

Tyr Thr Glu Trp Phe Val Asp Met Ser Leu Pro Gly Ile Thr Lys Glu
            180                 185                 190

Pro Leu His Lys Leu Gly Leu Arg Met Asp Ser Cys Ala Asp Ile Tyr
        195                 200                 205

Phe Asp Asn Val Glu Leu Arg Glu Glu Asp Leu Phe Gly Thr Glu Gly
    210                 215                 220

Asn Gly Phe Lys Arg Gly Val Lys Asp Phe Asp Leu Glu Arg Phe Glu
225                 230                 235                 240

Val Ala Met Thr Asn Tyr Gly Thr Ala Tyr Cys Ala Phe Glu Asp Ala
                245                 250                 255

Ala Lys Tyr Ala Asn Gln Arg Val Gln Gly Gly Glu Ala Ile Ala Arg
            260                 265                 270

Lys Gln Leu Ile Gln Leu Lys Phe Ala Asp Met Lys Val Asp Ile Thr
        275                 280                 285

Asn Met Arg Asn Met Leu Tyr Glu Ile Ala Trp Lys Ala Asp Asn Asp
    290                 295                 300

Gln Leu Gly Arg Gly Asp Cys Ser Met Ala Lys Tyr Tyr Cys Ser His
305                 310                 315                 320

Ala Ala Ala Arg Val Val Asp Asn Ala Leu Gln Thr Leu Ala Gly Val

325                 330                 335

Gly Val Thr Gly Glu His Arg Val Gln Arg Phe Tyr Arg Asp Leu Arg
            340                 345                 350

Val Asp Arg Val Ser Gly Gly Thr Asp Glu Met Met Ile Leu Ala Ala
        355                 360                 365

Gly Arg Gly Ala Leu Arg Asp Tyr Arg Ser
    370                 375

<210>  68
<211>  1137
<212>  DNA
<213>  Propionibacterium freudenreichii subsp. freudenreichii

<400>  68
atggatttct cattgaccga agaccagcag ctcatggtgg acggcttcac cgagctgatg      60

aacagtgagg catgggagaa gtacttccac gagtgtgacg agaactcgga gtatcccgag     120

cgctgggtca aggcgatctg cgacctcggc ttcgaccgca tcctgctgcc cgaggaatac     180

gacggcctgg gcctgggctg gcagacgttg gccgccgcct atgaggcgct cggccgcgct     240

ggtggcccca cctatgtgct ctaccaactg cccggctggg acacggtgat ccgcgaaggc     300

accgaggaac agaagaagga catcctcaag ttcgtgggca gcgggaagca gatgctcaac     360

tacgccatga ccgagcccag cgccgggtcg agctgggacg acatgtccac cacctacagc     420

cgcaagggcg gcaaggtgta cctcaacgga cacaagacct tcatcacctc atccatgaag     480

gtgccctacc tcgtggtgat ggcccgcgac gccgacaaca tgggcaccta caccgagtgg     540

ttcgtcgaca tgagccttcc gggcatcacc aaggagccgc tgcacaagct cggcctgcgc     600

atggattcgt gcgccgacat ctacttcgac aacgttgaac tgcgcgagga agacctgttc     660

ggcaccgagg gcaacggctt caagcgcggc gtgaaggact tcgacctgga gcgcttcgag     720

gtggccatga ccaactacgg caccgcctac tgcgccttcg aggatgcagc gaagtacgcc     780

aaccagcgcg tgcagggagg cgaggcgatt gcccgcaagc agctgatcca gctgaagttc     840

gccgacatga aggtggacat caccaatatg cgcaacatgc tctacgagat tgcctggaag     900

gccgacaacg accagctggg ccgcggcgac tgctccatgg cgaagtacta ctgctcccac     960

gccgcggccc gcgtggtcga caacgcgctg cagaccctcg ccggcgtggg cgtcaccggt    1020

gaacaccgcg tgcagcgctt ctaccgcgac ctccgcgtgg accgcgtctc cggtggaacc    1080

gacgaaatga tgatcctcgc cgccggccgt ggtgcactgc gcgactaccg cagctga       1137

<210>  69
<211>  44
<212>  DNA
<213>  Artificial

<220>
<223>  An artificially synthesized primer sequence

<400>  69
gaccatatgc ctaacttatt acaagaacgc cgcgaagtaa aaga                        44


<210>  70
<211>  45
<212>  DNA
<213>  Artificial

<220>
<223>  An artificially synthesized primer sequence

<400>  70
gctgttaatt aattattctc cataggcttt gcgatatatt tctgc                       45


<210>  71
<211>  36
<212>  DNA
<213>  Artificial

<220>
<223>  An artificially synthesized primer sequence

<400>  71
gaccatatgg atttctcatt gaccgaagac cagcag                                 36


<210>  72
<211>  35
<212>  DNA
<213>  Artificial

<220>
<223>  An artificially synthesized primer sequence

<400>  72
gctgttaatt aactacggta gtcgcgcagt gcacc                                  35

SEQUENCE LISTING

<110> DAICEL CHEMICAL INDUSTRIES, LTD.

<120> Recombinant microorganisms with 1,3-butanediol-producing
      function and uses thereof

<130> EP81739FZ240pau

<140> EP 10 826 848.3
<141> 2010-10-29

<150> PCT/JP2010/069274
<151> 2010-10-29

<150> JP 2009-251276
<151> 2009-10-30

<160> 72

<170> BiSSAP 1.0

<210> 1
<211> 858
<212> PRT
<213> Clostridium acetobutylicum

<220>
<221> SOURCE
<222> 1..858
<223> /mol_type="protein"
      /organism="Clostridium acetobutylicum"

<400> 1
Met Lys Val Thr Asn Gln Lys Glu Leu Lys Gln Lys Leu Asn Glu Leu
1               5                   10                  15
Arg Glu Ala Gln Lys Lys Phe Ala Thr Tyr Thr Gln Glu Gln Val Asp
            20                  25                  30
Lys Ile Phe Lys Gln Cys Ala Ile Ala Ala Ala Lys Glu Arg Ile Asn
        35                  40                  45
Leu Ala Lys Leu Ala Val Glu Glu Thr Gly Ile Gly Leu Val Glu Asp
    50                  55                  60
Lys Ile Ile Lys Asn His Phe Ala Ala Glu Tyr Ile Tyr Asn Lys Tyr
65                  70                  75                  80
Lys Asn Glu Lys Thr Cys Gly Ile Ile Asp His Asp Asp Ser Leu Gly
                85                  90                  95
Ile Thr Lys Val Ala Glu Pro Ile Gly Ile Val Ala Ala Ile Val Pro
            100                 105                 110
Thr Thr Asn Pro Thr Ser Thr Ala Ile Phe Lys Ser Leu Ile Ser Leu
        115                 120                 125
Lys Thr Arg Asn Ala Ile Phe Phe Ser Pro His Pro Arg Ala Lys Lys
    130                 135                 140
Ser Thr Ile Ala Ala Ala Lys Leu Ile Leu Asp Ala Ala Val Lys Ala
145                 150                 155                 160
Gly Ala Pro Lys Asn Ile Ile Gly Trp Ile Asp Glu Pro Ser Ile Glu
                165                 170                 175
Leu Ser Gln Asp Leu Met Ser Glu Ala Asp Ile Ile Leu Ala Thr Gly
            180                 185                 190
Gly Pro Ser Met Val Lys Ala Ala Tyr Ser Ser Gly Lys Pro Ala Ile
        195                 200                 205
Gly Val Gly Ala Gly Asn Thr Pro Ala Ile Ile Asp Glu Ser Ala Asp
    210                 215                 220
Ile Asp Met Ala Val Ser Ser Ile Ile Leu Ser Lys Thr Tyr Asp Asn
225                 230                 235                 240
Gly Val Ile Cys Ala Ser Glu Gln Ser Ile Leu Val Met Asn Ser Ile

245                 250                 255
Tyr Glu Lys Val Lys Glu Glu Phe Val Lys Arg Gly Ser Tyr Ile Leu
            260                 265                 270
Asn Gln Asn Glu Ile Ala Lys Ile Lys Glu Thr Met Phe Lys Asn Gly
        275                 280                 285
Ala Ile Asn Ala Asp Ile Val Gly Lys Ser Ala Tyr Ile Ile Ala Lys
    290                 295                 300
Met Ala Gly Ile Glu Val Pro Gln Thr Thr Lys Ile Leu Ile Gly Glu
305                 310                 315                 320
Val Gln Ser Val Glu Lys Ser Glu Leu Phe Ser His Glu Lys Leu Ser
                325                 330                 335
Pro Val Leu Ala Met Tyr Lys Val Lys Asp Phe Asp Glu Ala Leu Lys
            340                 345                 350
Lys Ala Gln Arg Leu Ile Glu Leu Gly Gly Ser Gly His Thr Ser Ser
        355                 360                 365
Leu Tyr Ile Asp Ser Gln Asn Asn Lys Asp Lys Val Lys Glu Phe Gly
    370                 375                 380
Leu Ala Met Lys Thr Ser Arg Thr Phe Ile Asn Met Pro Ser Ser Gln
385                 390                 395                 400
Gly Ala Ser Gly Asp Leu Tyr Asn Phe Ala Ile Ala Pro Ser Phe Thr
                405                 410                 415
Leu Gly Cys Gly Thr Trp Gly Gly Asn Ser Val Ser Gln Asn Val Glu
            420                 425                 430
Pro Lys His Leu Leu Asn Ile Lys Ser Val Ala Glu Arg Arg Glu Asn
        435                 440                 445
Met Leu Trp Phe Lys Val Pro Gln Lys Ile Tyr Phe Lys Tyr Gly Cys
    450                 455                 460
Leu Arg Phe Ala Leu Lys Glu Leu Lys Asp Met Asn Lys Lys Arg Ala
465                 470                 475                 480
Phe Ile Val Thr Asp Lys Asp Leu Phe Lys Leu Gly Tyr Val Asn Lys
                485                 490                 495
Ile Thr Lys Val Leu Asp Glu Ile Asp Ile Lys Tyr Ser Ile Phe Thr
            500                 505                 510
Asp Ile Lys Ser Asp Pro Thr Ile Asp Ser Val Lys Lys Gly Ala Lys
        515                 520                 525
Glu Met Leu Asn Phe Glu Pro Asp Thr Ile Ile Ser Ile Gly Gly Gly
    530                 535                 540
Ser Pro Met Asp Ala Ala Lys Val Met His Leu Leu Tyr Glu Tyr Pro
545                 550                 555                 560
Glu Ala Glu Ile Glu Asn Leu Ala Ile Asn Phe Met Asp Ile Arg Lys
                565                 570                 575
Arg Ile Cys Asn Phe Pro Lys Leu Gly Thr Lys Ala Ile Ser Val Ala
            580                 585                 590
Ile Pro Thr Thr Ala Gly Thr Gly Ser Glu Ala Thr Pro Phe Ala Val
        595                 600                 605
Ile Thr Asn Asp Glu Thr Gly Met Lys Tyr Pro Leu Thr Ser Tyr Glu
    610                 615                 620
Leu Thr Pro Asn Met Ala Ile Ile Asp Thr Glu Leu Met Leu Asn Met
625                 630                 635                 640
Pro Arg Lys Leu Thr Ala Ala Thr Gly Ile Asp Ala Leu Val His Ala
                645                 650                 655
Ile Glu Ala Tyr Val Ser Val Met Ala Thr Asp Tyr Thr Asp Glu Leu
            660                 665                 670
Ala Leu Arg Ala Ile Lys Met Ile Phe Lys Tyr Leu Pro Arg Ala Tyr
        675                 680                 685
Lys Asn Gly Thr Asn Asp Ile Glu Ala Arg Glu Lys Met Ala His Ala
    690                 695                 700
Ser Asn Ile Ala Gly Met Ala Phe Ala Asn Ala Phe Leu Gly Val Cys
705                 710                 715                 720
His Ser Met Ala His Lys Leu Gly Ala Met His His Val Pro His Gly
                725                 730                 735
Ile Ala Cys Ala Val Leu Ile Glu Glu Val Ile Lys Tyr Asn Ala Thr
            740                 745                 750
Asp Cys Pro Thr Lys Gln Thr Ala Phe Pro Gln Tyr Lys Ser Pro Asn
        755                 760                 765

Ala Lys Arg Lys Tyr Ala Glu Ile Ala Glu Tyr Leu Asn Leu Lys Gly
    770                 775                 780
Thr Ser Asp Thr Glu Lys Val Thr Ala Leu Ile Glu Ala Ile Ser Lys
785                 790                 795                 800
Leu Lys Ile Asp Leu Ser Ile Pro Gln Asn Ile Ser Ala Ala Gly Ile
                805                 810                 815
Asn Lys Lys Asp Phe Tyr Asn Thr Leu Asp Lys Met Ser Glu Leu Ala
            820                 825                 830
Phe Asp Asp Gln Cys Thr Thr Ala Asn Pro Arg Tyr Pro Leu Ile Ser
        835                 840                 845
Glu Leu Lys Asp Ile Tyr Ile Lys Ser Phe
    850                 855

<210> 2
<211> 2577
<212> DNA
<213> Clostridium acetobutylicum

<220>
<221> source
<222> 1..2577
<223> /mol_type="DNA"
      /organism="Clostridium acetobutylicum"

<400> 2
atgaaagtta caaatcaaaa agaactaaaa caaaagctaa atgaattgag agaagcgcaa      60

aagaagtttg caacctatac tcaagagcaa gttgataaaa tttttaaaca atgtgccata     120

gccgcagcta aagaaagaat aaacttagct aaattagcag tagaagaaac aggaataggt     180

cttgtagaag ataaaattat aaaaaatcat tttgcagcag aatatatata caataaatat     240

aaaaatgaaa aaacttgtgg cataatagac catgacgatt ctttaggcat aacaaaggtt     300

gctgaaccaa ttggaattgt tgcagccata gttcctacta ctaatccaac ttccacagca     360

attttcaaat cattaatttc tttaaaaaca agaaacgcaa tattcttttc accacatcca     420

cgtgcaaaaa aatctacaat tgctgcagca aaattaattt tagatgcagc tgttaaagca     480

ggagcaccta aaaatataat aggctggata gatgagccat caatagaact ttctcaagat     540

ttgatgagtg aagctgatat aatattagca acaggaggtc cttcaatggt taaagcggcc     600

tattcatctg gaaaacctgc aattggtgtt ggagcaggaa atacaccagc aataatagat     660

gagagtgcag atatagatat ggcagtaagc tccataattt tatcaaagac ttatgacaat     720

ggagtaatat gcgcttctga acaatcaata ttagttatga attcaatata cgaaaaagtt     780

aaagaggaat ttgtaaaacg aggatcatat atactcaatc aaaatgaaat agctaaaata     840

aaagaaacta tgtttaaaaa tggagctatt aatgctgaca tagttggaaa atctgcttat     900

ataattgcta aaatggcagg aattgaagtt cctcaaacta caaagatact tataggcgaa     960

gtacaatctg ttgaaaaaag cgagctgttc tcacatgaaa aactatcacc agtacttgca    1020

atgtataaag ttaaggattt tgatgaagct ctaaaaaagg cacaaaggct aatagaatta    1080

ggtggaagtg gacacacgtc atctttatat atagattcac aaaacaataa ggataaagtt    1140

aaagaatttg gattagcaat gaaaacttca aggacattta ttaacatgcc ttcttcacag    1200

ggagcaagcg gagatttata caattttgcg atagcaccat catttactct tggatgcggc 1260

acttggggag gaaactctgt atcgcaaaat gtagagccta aacatttatt aaatattaaa 1320

agtgttgctg aaagaaggga aaatatgctt tggtttaaag tgccacaaaa aatatatttt 1380

aaatatggat gtcttagatt tgcattaaaa gaattaaaag atatgaataa gaaaagagcc 1440

tttatagtaa cagataaaga tctttttaaa cttggatatg ttaataaaat aacaaaggta 1500

ctagatgaga tagatattaa atacagtata tttacagata ttaaatctga tccaactatt 1560

gattcagtaa aaaaaggtgc taaagaaatg cttaactttg aacctgatac tataatctct 1620

attggtggtg gatcgccaat ggatgcagca aaggttatgc acttgttata tgaatatcca 1680

gaagcagaaa ttgaaaatct agctataaac tttatggata taagaaagag aatatgcaat 1740

ttccctaaat taggtacaaa ggcgatttca gtagctattc ctacaactgc tggtaccggt 1800

tcagaggcaa caccttttgc agttataact aatgatgaaa caggaatgaa atacccttta 1860

acttcttatg aattgacccc aaacatggca ataatagata ctgaattaat gttaaatatg 1920

cctagaaaat taacagcagc aactggaata gatgcattag ttcatgctat agaagcatat 1980

gtttcggtta tggctacgga ttatactgat gaattagcct taagagcaat aaaaatgata 2040

tttaaatatt tgcctagagc ctataaaaat gggactaacg acattgaagc aagagaaaaa 2100

atggcacatg cctctaatat tgcggggatg gcatttgcaa atgctttctt aggtgtatgc 2160

cattcaatgg ctcataaact tggggcaatg catcacgttc cacatggaat tgcttgtgct 2220

gtattaatag aagaagttat taaatataac gctacagact gtccaacaaa gcaaacagca 2280

ttccctcaat ataaatctcc taatgctaag agaaaatatg ctgaaattgc agagtatttg 2340

aatttaaagg gtactagcga taccgaaaag gtaacagcct taatagaagc tatttcaaag 2400

ttaaagatag atttgagtat tccacaaaat ataagtgccg ctggaataaa taaaaaagat 2460

ttttataata cgctagataa aatgtcagag cttgcttttg atgaccaatg tacaacagct 2520

aatcctaggt atccacttat aagtgaactt aaggatatct atataaaatc attttaa 2577

<210> 3
<211> 390
<212> PRT
<213> Clostridium acetobutylicum

<220>
<221> SOURCE
<222> 1..390
<223> /mol_type="protein"
/organism="Clostridium acetobutylicum"

<400> 3
Val Val Asp Phe Glu Tyr Ser Ile Pro Thr Arg Ile Phe Phe Gly Lys
1               5                   10                  15
Asp Lys Ile Asn Val Leu Gly Arg Glu Leu Lys Lys Tyr Gly Ser Lys
            20                  25                  30
Val Leu Ile Val Tyr Gly Gly Gly Ser Ile Lys Arg Asn Gly Ile Tyr
        35                  40                  45

Asp Lys Ala Val Ser Ile Leu Glu Lys Asn Ser Ile Lys Phe Tyr Glu
    50                  55                  60
Leu Ala Gly Val Glu Pro Asn Pro Arg Val Thr Thr Val Glu Lys Gly
65                  70                  75                  80
Val Lys Ile Cys Arg Glu Asn Gly Val Glu Val Val Leu Ala Ile Gly
                85                  90                  95
Gly Gly Ser Ala Ile Asp Cys Ala Lys Val Ile Ala Ala Ala Cys Glu
            100                 105                 110
Tyr Asp Gly Asn Pro Trp Asp Ile Val Leu Asp Gly Ser Lys Ile Lys
        115                 120                 125
Arg Val Leu Pro Ile Ala Ser Ile Leu Thr Ile Ala Ala Thr Gly Ser
    130                 135                 140
Glu Met Asp Thr Trp Ala Val Ile Asn Asn Met Asp Thr Asn Glu Lys
145                 150                 155                 160
Leu Ile Ala Ala His Pro Asp Met Ala Pro Lys Phe Ser Ile Leu Asp
                165                 170                 175
Pro Thr Tyr Thr Tyr Thr Val Pro Thr Asn Gln Thr Ala Ala Gly Thr
            180                 185                 190
Ala Asp Ile Met Ser His Ile Phe Glu Val Tyr Phe Ser Asn Thr Lys
        195                 200                 205
Thr Ala Tyr Leu Gln Asp Arg Met Ala Glu Ala Leu Leu Arg Thr Cys
    210                 215                 220
Ile Lys Tyr Gly Gly Ile Ala Leu Glu Lys Pro Asp Asp Tyr Glu Ala
225                 230                 235                 240
Arg Ala Asn Leu Met Trp Ala Ser Ser Leu Ala Ile Asn Gly Leu Leu
                245                 250                 255
Thr Tyr Gly Lys Asp Thr Asn Trp Ser Val His Leu Met Glu His Glu
            260                 265                 270
Leu Ser Ala Tyr Tyr Asp Ile Thr His Gly Val Gly Leu Ala Ile Leu
        275                 280                 285
Thr Pro Asn Trp Met Glu Tyr Ile Leu Asn Asn Asp Thr Val Tyr Lys
    290                 295                 300
Phe Val Glu Tyr Gly Val Asn Val Trp Gly Ile Asp Lys Glu Lys Asn
305                 310                 315                 320
His Tyr Asp Ile Ala His Gln Ala Ile Gln Lys Thr Arg Asp Tyr Phe
                325                 330                 335
Val Asn Val Leu Gly Leu Pro Ser Arg Leu Arg Asp Val Gly Ile Glu
            340                 345                 350
Glu Glu Lys Leu Asp Ile Met Ala Lys Glu Ser Val Lys Leu Thr Gly
        355                 360                 365
Gly Thr Ile Gly Asn Leu Arg Pro Val Asn Ala Ser Glu Val Leu Gln
    370                 375                 380
Ile Phe Lys Lys Ser Val
385                 390

<210> 4
<211> 1173
<212> DNA
<213> Clostridium acetobutylicum

<220>
<221> source
<222> 1..1173
<223> /mol_type="DNA"
      /organism="Clostridium acetobutylicum"

<400> 4
gtggttgatt tcgaatattc aataccaact agaatttttt tcggtaaaga taagataaat      60

gtacttggaa gagagcttaa aaaatatggt tctaaagtgc ttatagttta tggtggagga     120

agtataaaga gaaatggaat atatgataaa gctgtaagta tacttgaaaa aaacagtatt     180

aaattttatg aacttgcagg agtagagcca aatccaagag taactacagt tgaaaaagga     240

gttaaaatat gtagagaaaa tggagttgaa gtagtactag ctataggtgg aggaagtgca 300

atagattgcg caaaggttat agcagcagca tgtgaatatg atggaaatcc atgggatatt 360

gtgttagatg gctcaaaaat aaaaagggtg cttcctatag ctagtatatt aaccattgct 420

gcaacaggat cagaaatgga tacgtgggca gtaataaata atatggatac aaacgaaaaa 480

ctaattgcgg cacatccaga tatggctcct aagttttcta tattagatcc aacgtatacg 540

tataccgtac ctaccaatca aacagcagca ggaacagctg atattatgag tcatatattt 600

gaggtgtatt ttagtaatac aaaaacagca tatttgcagg atagaatggc agaagcgtta 660

ttaagaactt gtattaaata tggaggaata gctcttgaga agccggatga ttatgaggca 720

agagccaatc taatgtgggc ttcaagtctt gcgataaatg gacttttaac atatggtaaa 780

gacactaatt ggagtgtaca cttaatggaa catgaattaa gtgcttatta cgacataaca 840

cacggcgtag ggcttgcaat tttaacacct aattggatgg agtatatttt aaataatgat 900

acagtgtaca agtttgttga atatggtgta aatgtttggg gaatagacaa agaaaaaaat 960

cactatgaca tagcacatca agcaatacaa aaaacaagag attactttgt aaatgtacta 1020

ggtttaccat ctagactgag agatgttgga attgaagaag aaaaattgga cataatggca 1080

aaggaatcag taaagcttac aggaggaacc ataggaaacc taagaccagt aaacgcctcc 1140

gaagtcctac aaatattcaa aaaatctgtg taa 1173

<210> 5
<211> 337
<212> PRT
<213> Geobacillus stearothermophilus

<220>
<221> SOURCE
<222> 1..337
<223> /mol_type="protein"
/organism="Geobacillus stearothermophilus"

<400> 5
Met Lys Ala Ala Val Val Glu Gln Phe Lys Lys Pro Leu Gln Val Lys
1               5                   10                  15
Glu Val Glu Lys Pro Lys Ile Ser Tyr Gly Glu Val Leu Val Arg Ile
            20                  25                  30
Lys Ala Cys Gly Val Cys His Thr Asp Leu His Ala Ala His Gly Asp
        35                  40                  45
Trp Pro Val Lys Pro Lys Leu Pro Leu Ile Pro Gly His Glu Gly Val
    50                  55                  60
Gly Val Ile Glu Glu Val Gly Pro Gly Val Thr His Leu Lys Val Gly
65                  70                  75                  80
Asp Arg Val Gly Ile Pro Trp Leu Tyr Ser Ala Cys Gly His Cys Asp
                85                  90                  95
Tyr Cys Leu Ser Gly Gln Glu Thr Leu Cys Glu Arg Gln Gln Asn Ala
            100                 105                 110
Gly Tyr Ser Val Asp Gly Gly Tyr Ala Glu Tyr Cys Arg Ala Ala Ala
        115                 120                 125
Asp Tyr Val Val Lys Ile Pro Asp Asn Leu Ser Phe Glu Glu Ala Ala
    130                 135                 140
Pro Ile Phe Cys Ala Gly Val Thr Thr Tyr Lys Ala Leu Lys Val Thr
145                 150                 155                 160

Gly Ala Lys Pro Gly Glu Trp Val Ala Ile Tyr Gly Ile Gly Gly Leu
                165                 170                 175
Gly His Val Ala Val Gln Tyr Ala Lys Ala Met Gly Leu Asn Val Val
            180                 185                 190
Ala Val Asp Leu Gly Asp Glu Lys Leu Glu Leu Ala Lys Gln Leu Gly
        195                 200                 205
Ala Asp Leu Val Val Asn Pro Lys His Asp Asp Ala Ala Gln Trp Ile
    210                 215                 220
Lys Glu Lys Val Gly Gly Val His Ala Thr Val Val Thr Ala Val Ser
225                 230                 235                 240
Lys Ala Ala Phe Glu Ser Ala Tyr Lys Ser Ile Arg Arg Gly Gly Ala
                245                 250                 255
Cys Val Leu Val Gly Leu Pro Pro Glu Glu Ile Pro Ile Pro Ile Phe
            260                 265                 270
Asp Thr Val Leu Asn Gly Val Lys Ile Ile Gly Ser Ile Val Gly Thr
        275                 280                 285
Arg Lys Asp Leu Gln Glu Ala Leu Gln Phe Ala Ala Glu Gly Lys Val
    290                 295                 300
Lys Thr Ile Val Glu Val Gln Pro Leu Glu Asn Ile Asn Asp Val Phe
305                 310                 315                 320
Asp Arg Met Leu Lys Gly Gln Ile Asn Gly Arg Val Val Leu Lys Val
                325                 330                 335
Asp

<210> 6
<211> 1014
<212> DNA
<213> Geobacillus stearothermophilus

<220>
<221> source
<222> 1..1014
<223> /mol_type="DNA"
      /organism="Geobacillus stearothermophilus"

<400> 6
atgaaagctg cagttgtgga acaatttaaa aagccgttac aagtgaaaga agtggaaaaa      60

cctaagatct catacgggga agtattagtg cgcatcaaag cgtgtggggt atgccataca     120

gacttgcatg ccgcacatgg cgactggcct gtaaagccta aactgcctct cattcctggc     180

catgaaggcg tcggtgtaat tgaagaagta ggtcctgggg taacacattt aaaagttgga     240

gatcgcgtag gtatcccttg gctttattcg gcgtgcggtc attgtgacta ttgcttaagc     300

ggacaagaaa cattatgcga acgtcaacaa aacgctggct attccgtcga tggtggttat     360

gctgaatatt gccgtgctgc agccgattat gtcgtaaaaa ttcctgataa cttatcgttt     420

gaagaagccg ctccaatctt ttgcgctggt gtaacaacat ataaagcgct caaagtaaca     480

ggcgcaaaac caggtgaatg ggtagccatt tacggtatcg gcgggcttgg acatgtcgca     540

gtccaatacg caaaggcgat ggggttaaac gtcgttgctg tcgatttagg tgatgaaaaa     600

cttgagcttg ctaaacaact tggtgcagat cttgtcgtca atccgaaaca tgatgatgca     660

gcacaatgga taaaagaaaa agtgggcggt gtgcatgcga ctgtcgtcac agctgtttca     720

aaagccgcgt tcgaatcagc ctacaaatcc attcgtcgcg gtggtgcttg cgtactcgtc     780

ggattaccgc cggaagaaat acctattcca attttcgata cagtattaaa tggagtaaaa     840

attattggtt ctatcgttgg tacgcgcaaa gacttacaag aggcacttca atttgcagca 900

gaaggaaaag taaaaacaat tgtcgaagtg caaccgcttg aaaacattaa cgacgtattc 960

gatcgtatgt taaaagggca aattaacggc cgcgtcgtgt taaaagtaga ttaa 1014

<210> 7
<211> 348
<212> PRT
<213> Saccharomyces cerevisiae

<220>
<221> SOURCE
<222> 1..348
<223> /mol_type="protein"
/organism="Saccharomyces cerevisiae"

<400> 7
Met Ser Ile Pro Glu Thr Gln Lys Ala Ile Ile Phe Tyr Glu Ser Asn
1               5                   10                  15
Gly Lys Leu Glu His Lys Asp Ile Pro Val Pro Lys Pro Lys Pro Asn
            20                  25                  30
Glu Leu Leu Ile Asn Val Lys Tyr Ser Gly Val Cys His Thr Asp Leu
        35                  40                  45
His Ala Trp His Gly Asp Trp Pro Leu Pro Thr Lys Leu Pro Leu Val
    50                  55                  60
Gly Gly His Glu Gly Ala Gly Val Val Val Gly Met Gly Glu Asn Val
65                  70                  75                  80
Lys Gly Trp Lys Ile Gly Asp Tyr Ala Gly Ile Lys Trp Leu Asn Gly
                85                  90                  95
Ser Cys Met Ala Cys Glu Tyr Cys Glu Leu Gly Asn Glu Ser Asn Cys
            100                 105                 110
Pro His Ala Asp Leu Ser Gly Tyr Thr His Asp Gly Ser Phe Gln Glu
        115                 120                 125
Tyr Ala Thr Ala Asp Ala Val Gln Ala Ala His Ile Pro Gln Gly Thr
    130                 135                 140
Asp Leu Ala Glu Val Ala Pro Ile Leu Cys Ala Gly Ile Thr Val Tyr
145                 150                 155                 160
Lys Ala Leu Lys Ser Ala Asn Leu Arg Ala Gly His Trp Ala Ala Ile
                165                 170                 175
Ser Gly Ala Ala Gly Gly Leu Gly Ser Leu Ala Val Gln Tyr Ala Lys
            180                 185                 190
Ala Met Gly Tyr Arg Val Leu Gly Ile Asp Gly Gly Pro Gly Lys Glu
        195                 200                 205
Glu Leu Phe Thr Ser Leu Gly Gly Glu Val Phe Ile Asp Phe Thr Lys
    210                 215                 220
Glu Lys Asp Ile Val Ser Ala Val Val Lys Ala Thr Asn Gly Gly Ala
225                 230                 235                 240
His Gly Ile Ile Asn Val Ser Val Ser Glu Ala Ala Ile Glu Ala Ser
                245                 250                 255
Thr Arg Tyr Cys Arg Ala Asn Gly Thr Val Val Leu Val Gly Leu Pro
            260                 265                 270
Ala Gly Ala Lys Cys Ser Ser Asp Val Phe Asn His Val Val Lys Ser
        275                 280                 285
Ile Ser Ile Val Gly Ser Tyr Val Gly Asn Arg Ala Asp Thr Arg Glu
    290                 295                 300
Ala Leu Asp Phe Phe Ala Arg Gly Leu Val Lys Ser Pro Ile Lys Val
305                 310                 315                 320
Val Gly Leu Ser Ser Leu Pro Glu Ile Tyr Glu Lys Met Glu Lys Gly
                325                 330                 335
Gln Ile Ala Gly Arg Tyr Val Val Asp Thr Ser Lys
            340                 345

<210> 8

<211> 1047
<212> DNA
<213> Saccharomyces cerevisiae

<220>
<221> source
<222> 1..1047
<223> /mol_type="DNA"
      /organism="Saccharomyces cerevisiae"

<400> 8
atgtctattc cagaaactca aaaagccatt atcttctacg aatccaacgg caagttggag      60

cataaggata tcccagttcc aaagccaaag cccaacgaat tgttaatcaa cgtcaagtac     120

tctggtgtct gccacaccga tttgcacgct tggcatggtg actggccatt gccaactaag     180

ttaccattag ttggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca agaatacgct accgctgacg ctgttcaagc cgctcacatt     420

cctcaaggta ctgacttggc tgaagtcgcg ccaatcttgt gtgctggtat caccgtatac     480

aaggctttga agtctgccaa cttgagagca ggccactggg cggccatttc tggtgctgct     540

ggtggtctag gttctttggc tgttcaatat gctaaggcga tgggttacag agtcttaggt     600

attgatggtg gtccaggaaa ggaagaattg tttacctcgc tcggtggtga agtattcatc     660

gacttcacca aagagaagga cattgttagc gcagtcgtta aggctaccaa cggcggtgcc     720

cacggtatca tcaatgtttc cgtttccgaa gccgctatcg aagcttctac cagatactgt     780

agggcgaacg gtactgttgt cttggttggt ttgccagccg gtgcaaagtg ctcctctgat     840

gtcttcaacc acgttgtcaa gtctatctcc attgtcggct cttacgtggg gaacagagct     900

gataccagag aagccttaga tttctttgcc agaggtctag tcaagtctcc aataaaggta     960

gttggcttat ccagtttacc agaaatttac gaaaagatgg agaagggcca aattgctggt    1020

cgttacgttg ttgacacttc taaataa                                        1047


<210> 9
<211> 246
<212> PRT
<213> Cupriavidus necator

<220>
<221> SOURCE
<222> 1..246
<223> /mol_type="protein"
      /organism="Cupriavidus necator"

<400> 9
Met Thr Gln Arg Ile Ala Tyr Val Thr Gly Gly Met Gly Gly Ile Gly
1               5                   10                  15
Thr Ala Ile Cys Gln Arg Leu Ala Lys Asp Gly Phe Arg Val Val Ala
            20                  25                  30
Gly Cys Gly Pro Asn Ser Pro Arg Arg Glu Lys Trp Leu Glu Gln Gln

35                  40                  45
Lys Ala Leu Gly Phe Asp Phe Ile Ala Ser Glu Gly Asn Val Ala Asp
    50                  55                  60
Trp Asp Ser Thr Lys Thr Ala Phe Asp Lys Val Lys Ser Glu Val Gly
65                  70                  75                  80
Glu Val Asp Val Leu Ile Asn Asn Ala Gly Ile Thr Arg Asp Val Val
                85                  90                  95
Phe Arg Lys Met Thr Arg Ala Asp Trp Asp Ala Val Ile Asp Thr Asn
            100                 105                 110
Leu Thr Ser Leu Phe Asn Val Thr Lys Gln Val Ile Asp Gly Met Ala
        115                 120                 125
Asp Arg Gly Trp Gly Arg Ile Val Asn Ile Ser Ser Val Asn Gly Gln
    130                 135                 140
Lys Gly Gln Phe Gly Gln Thr Asn Tyr Ser Thr Ala Lys Ala Gly Leu
145                 150                 155                 160
His Gly Phe Thr Met Ala Leu Ala Gln Glu Val Ala Thr Lys Gly Val
                165                 170                 175
Thr Val Asn Thr Val Ser Pro Gly Tyr Ile Ala Thr Asp Met Val Lys
            180                 185                 190
Ala Ile Arg Gln Asp Val Leu Asp Lys Ile Val Ala Thr Ile Pro Val
        195                 200                 205
Lys Arg Leu Gly Leu Pro Glu Glu Ile Ala Ser Ile Cys Ala Trp Leu
    210                 215                 220
Ser Ser Glu Glu Ser Gly Phe Ser Thr Gly Ala Asp Phe Ser Leu Asn
225                 230                 235                 240
Gly Gly Leu His Met Gly
                245

<210> 10
<211> 741
<212> DNA
<213> Cupriavidus necator

<220>
<221> source
<222> 1..741
<223> /mol_type="DNA"
      /organism="Cupriavidus necator"

<400> 10
atgactcagc gcattgcgta tgtgaccggc ggcatgggtg gtatcggaac cgccatttgc      60

cagcggctgg ccaaggatgg ctttcgtgtg gtggccggtt gcggccccaa ctcgccgcgc     120

cgcgaaaagt ggctggagca gcagaaggcc ctgggcttcg atttcattgc ctcggaaggc     180

aatgtggctg actgggactc gaccaagacc gcattcgaca aggtcaagtc cgaggtcggc     240

gaggttgatg tgctgatcaa caacgccggt atcacccgcg acgtggtgtt ccgcaagatg     300

acccgcgccg actgggatgc ggtgatcgac accaacctga cctcgctgtt caacgtcacc     360

aagcaggtga tcgacggcat ggccgaccgt ggctggggcc gcatcgtcaa catctcgtcg     420

gtgaacgggc agaaggccca gttcggccag accaactact ccaccgccaa ggccggcctg     480

catggcttca ccatggcact ggcgcaggaa gtggcgacca agggcgtgac cgtcaacacg     540

gtctctccgg gctatatcgc caccgacatg gtcaaggcga tccgccagga cgtgctcgac     600

aagatcgtcg cgacgatccc ggtcaagcgc ctgggcctgc cggaagagat cgcctcgatc     660

tgcgcctggt tgtcgtcgga ggagtccggt ttctcgaccg gcgccgactt ctcgctcaac     720

ggcggcctgc atatgggctg a                                               741

<210> 11
<211> 241
<212> PRT
<213> Zoogloea ramigera

<220>
<221> SOURCE
<222> 1..241
<223> /mol_type="protein"
      /organism="Zoogloea ramigera"

<400> 11
Met Ser Arg Val Ala Leu Val Thr Gly Gly Ser Arg Gly Ile Gly Ala
1               5                   10                  15
Ala Ile Ser Ile Ala Leu Lys Ala Ala Gly Tyr Lys Val Ala Ala Ser
            20                  25                  30
Tyr Ala Gly Asn Asp Asp Ala Ala Asn Ala Phe Lys Ala Glu Thr Gly
        35                  40                  45
Ile Ala Val Tyr Lys Trp Asp Val Ser Ser Tyr Glu Ala Cys Val Glu
    50                  55                  60
Gly Ile Ala Lys Val Glu Ala Asp Leu Gly Pro Ile Asp Val Leu Val
65                  70                  75                  80
Asn Asn Ala Gly Ile Thr Lys Asp Ala Met Phe His Lys Met Thr Pro
                85                  90                  95
Asp Gln Trp Asn Ala Val Ile Asn Thr Asn Leu Thr Gly Leu Phe Asn
            100                 105                 110
Met Thr His Pro Val Trp Ser Gly Met Arg Asp Arg Ser Phe Gly Arg
        115                 120                 125
Ile Val Asn Ile Ser Ser Ile Asn Gly Gln Lys Gly Gln Met Gly Gln
    130                 135                 140
Ala Asn Tyr Ser Ala Ala Lys Ala Gly Asp Leu Gly Phe Thr Lys Ala
145                 150                 155                 160
Leu Ala Gln Glu Gly Ala Ala Lys Gly Ile Thr Val Asn Ala Ile Cys
                165                 170                 175
Pro Gly Tyr Ile Gly Thr Glu Met Val Arg Ala Ile Pro Glu Lys Val
            180                 185                 190
Leu Asn Glu Arg Ile Ile Pro Gln Ile Pro Val Gly Arg Leu Gly Glu
        195                 200                 205
Pro Asp Glu Ile Ala Arg Ile Val Val Phe Leu Ala Ser Asp Glu Ala
    210                 215                 220
Gly Phe Ile Thr Gly Ser Thr Ile Ser Ala Asn Gly Gly Gln Phe Phe
225                 230                 235                 240
Val


<210> 12
<211> 726
<212> DNA
<213> Zoogloea ramigera

<220>
<221> source
<222> 1..726
<223> /mol_type="DNA"
      /organism="Zoogloea ramigera"

<400> 12
atgagtcgtg tagcattggt aacgggggga tcgcggggca tcggcgcagc catttcgatt      60

gcgctgaagg cggcgggata caaggtggct gccagctatg ccggcaatga cgatgcggcc     120

aacgccttca aggcggaaac gggcatcgcc gtctacaagt gggacgtgtc gagctacgag     180

gcctgcgtcg aggggatcgc caaggtggag gccgatctcg gcccgattga cgttctcgtc     240

aacaatgcgg gcatcaccaa ggacgcgatg ttccacaaga tgacgcccga ccagtggaat     300

gcggtcatca acaccaacct cacgggtctc ttcaacatga cccatccggt ctggtccggc     360

atgcgcgacc gcagcttcgg ccgcatcgtc aacatctcct cgatcaacgg ccagaagggc     420

cagatgggtc aggcgaacta ttccgccgcc aaggccggcg acctcggctt caccaaggcg     480

ctggcgcagg aaggcgcggc caagggcatc acggtcaacg ccatctgccc cggctatatc     540

ggtacggaaa tggtgcgcgc cattccggaa aaggtgctga acgagcggat catcccgcag     600

atcccggtcg gccgcctcgg cgagccggac gagatcgccc gcatcgtcgt cttcctcgcc     660

tcggacgagg ccggcttcat caccggctcg accatctcgg cgaacggcgg ccagttcttc     720

gtctaa                                                                726

<210> 13
<211> 254
<212> PRT
<213> Geobacillus stearothermophilus

<220>
<221> SOURCE
<222> 1..254
<223> /mol_type="protein"
      /organism="Geobacillus stearothermophilus"

<400> 13
Met Ser Gln Arg Phe Ala Gly Arg Val Ala Phe Val Thr Gly Gly Ser
1               5                   10                  15
Arg Gly Ile Gly Lys Ala Ile Val Thr Arg Phe Ala Glu Glu Gly Ala
            20                  25                  30
Lys Val Ala Phe Ile Asp Leu Asn Glu Glu Ala Leu Glu Ala Thr Ala
        35                  40                  45
Ala Glu Leu Arg Glu Lys Gly Tyr Asp Val Tyr Ala Lys Val Ala Ser
    50                  55                  60
Val Thr Asp Arg Glu Gln Val Glu Thr Thr Met Gln Glu Val Val Asp
65                  70                  75                  80
Arg Phe Gly Ser Leu Asp Ile Leu Val Asn Asn Ala Gly Val Ile Arg
                85                  90                  95
Asp Asn Leu Leu Phe Lys Met Thr Asp Asp Asp Trp Gln Thr Val Met
            100                 105                 110
Asp Val His Leu Lys Gly Ala Phe Tyr Cys Ala Arg Ala Ala Gln Lys
        115                 120                 125
Tyr Met Val Glu Lys Gly Tyr Gly Arg Ile Ile Asn Ile Ser Ser Thr
    130                 135                 140
Ser Ala Leu Gly Asn Arg Gly Gln Ala Asn Tyr Ser Ala Ala Lys Ala
145                 150                 155                 160
Gly Ile Gln Gly Phe Thr Lys Thr Leu Ala Ile Glu Leu Gly Lys Phe
                165                 170                 175
Gly Ile Thr Thr Asn Ala Ile Ala Pro Gly Phe Ile Glu Thr Asp Met
            180                 185                 190
Thr Lys Ala Thr Ala Glu Arg Leu Gly Ile Ser Phe Glu Gln Leu Ile
        195                 200                 205
Gln Ala Ser Val Ala Asn Ile Pro Val Gly Arg Ser Gly Arg Pro Glu
    210                 215                 220
Asp Ile Ala His Ala Val Ala Phe Phe Ala Asp Glu Arg Ser Ser Phe
225                 230                 235                 240
Val Asn Gly Gln Val Leu Tyr Val Ala Gly Gly Pro Lys Cys
                245                 250

<210> 14
<211> 765
<212> DNA
<213> Geobacillus stearothermophilus

<220>
<221> source
<222> 1..765
<223> /mol_type="DNA"
      /organism="Geobacillus stearothermophilus"

<400> 14
atgtctcaac gttttgcagg tcgtgtggcg tttgtgaccg gaggaagccg cggcatcggc      60

aaggcgattg tcacccgctt cgcggaagaa ggggcgaaag tggcattcat cgatttaaat     120

gaagaagcgc ttgaagcgac ggctgccgag ctgcgggaga aagggtatga cgtgtacgcc     180

aaagtggcga gcgtcaccga ccgcgaacaa gtggaaacga cgatgcaaga ggtcgtcgac     240

cggttcggct cgcttgatat tcttgtcaac aacgccggcg tcatccgcga caacttgctc     300

tttaaaatga cggacgacga ctggcaaacg gtcatggacg ttcatttgaa aggggcgttt     360

tattgcgccc gcgccgcgca aaaatatatg gtcgaaaaag ggtacgggcg catcatcaac     420

atttcgtcga cctccgcgct cggaaaccgc ggccaggcga actattcggc ggcgaaagcc     480

ggcattcaag ggtttacgaa aacgttggcg atcgagctcg gcaagttcgg cattacgacg     540

aacgccatcg ctccaggatt cattgaaacc gatatgacaa aggcaaccgc tgagcggctt     600

ggcatttcgt ttgagcagct cattcaggcg agcgtcgcca acatcccggt cggacgcagt     660

ggccgtccgg aagacatcgc ccatgcagtc gcgtttttcg ccgacgaacg gtcgtcgttt     720

gtcaacggcc aagtgctgta tgtggcaggt ggtccaaaat gttaa                     765


<210> 15
<211> 261
<212> PRT
<213> Streptomyces violaceoruber

<220>
<221> SOURCE
<222> 1..261
<223> /mol_type="protein"
      /organism="Streptomyces violaceoruber"

<400> 15
Met Ala Thr Gln Asp Ser Glu Val Ala Leu Val Thr Gly Ala Thr Ser
1               5                   10                  15
Gly Ile Gly Leu Glu Ile Ala Arg Arg Leu Gly Lys Glu Gly Leu Arg
            20                  25                  30
Val Phe Val Cys Ala Arg Gly Glu Glu Gly Leu Arg Thr Thr Leu Lys
        35                  40                  45
Glu Leu Arg Glu Ala Gly Val Glu Ala Asp Gly Arg Thr Cys Asp Val
    50                  55                  60
Arg Ser Val Pro Glu Ile Glu Ala Leu Val Ala Ala Val Val Glu Arg
65                  70                  75                  80
Tyr Gly Pro Val Asp Val Leu Val Asn Asn Ala Gly Arg Pro Gly Gly
                85                  90                  95
Gly Ala Thr Ala Glu Leu Ala Asp Glu Leu Trp Leu Asp Val Val Glu

                100                 105                 110
Thr Asn Leu Thr Gly Val Phe Arg Val Thr Lys Gln Val Leu Lys Ala
        115                 120                 125
Gly Gly Met Leu Glu Arg Gly Thr Gly Arg Ile Val Asn Ile Ala Ser
    130                 135                 140
Thr Gly Gly Lys Gln Gly Val Val His Ala Ala Pro Tyr Ser Ala Ser
145                 150                 155                 160
Lys His Gly Val Val Gly Phe Thr Lys Ala Leu Gly Leu Glu Leu Ala
                165                 170                 175
Arg Thr Gly Ile Thr Val Asn Ala Val Cys Pro Gly Phe Val Glu Thr
            180                 185                 190
Pro Met Ala Ala Ser Val Arg Glu His Tyr Ser Asp Ile Trp Glu Val
        195                 200                 205
Ser Thr Glu Glu Ala Phe Asp Arg Ile Thr Ala Arg Val Pro Ile Gly
    210                 215                 220
Arg Tyr Val Gln Pro Ser Glu Val Ala Glu Met Val Ala Tyr Leu Ile
225                 230                 235                 240
Gly Pro Gly Ala Ala Ala Val Thr Ala Gln Ala Leu Asn Val Cys Gly
                245                 250                 255
Gly Leu Gly Asn Tyr
            260

<210> 16
<211> 786
<212> DNA
<213> Streptomyces violaceoruber

<220>
<221> source
<222> 1..786
<223> /mol_type="DNA"
      /organism="Streptomyces violaceoruber"

<400> 16
atggccacgc aggactccga agtcgcactg gtgacgggtg cgaccagcgg aatcgggctg      60

gagatcgccc gcaggctcgg taaggagggg ttgcgcgtgt tcgtctgcgc gcgcggcgaa     120

gaaggactgc ggacgacgct gaaggagttg cgggaggcgg gcgtggaggc ggacgggcgg     180

acctgcgacg tccgctcggt cccggagatc gaggctctcg tggcggcggt ggtcgagcgt     240

tacggtccgg tcgacgtgct ggtgaacaac gcgggccggc ccggcggcgg cgccacggcc     300

gaactcgccg acgaactctg gctcgatgtc gtggagacca acctcaccgg cgtgttccgg     360

gtgaccaaac aggtcctcaa ggcgggcggc atgctcgaac ggggcacggg ccgaatcgtc     420

aacatcgcct cgaccggcgg aaagcagggt gtggtgcacg ccgcgcccta ctccgcctcg     480

aagcacggcg tggtcggctt caccaaggcg ctcggtctcg aactggcgag gaccggcatc     540

acggtgaacg ccgtctgccc cggattcgtc gagacgccga tggccgcgtc cgtgcgcgag     600

cactactcgg acatctggga ggtgtcgacc gaggaggcct tcgaccggat caccgcgcgc     660

gtgccgatcg gccggtacgt gcagccgtcc gaggtggcgg agatggtggc gtacctgatc     720

gggcccggtg cggccgcggt caccgcgcag gcgctgaacg tctgcggcgg gctggggaac     780

tactaa                                                                786


<210> 17
<211> 254

<212> PRT
<213> Pichia finlandica

<220>
<221> SOURCE
<222> 1..254
<223> /mol_type="protein"
      /organism="Pichia finlandica"

<400> 17
Met Ser Tyr Asn Phe His Asn Lys Val Ala Val Val Thr Gly Ala Leu
1               5                   10                  15
Ser Gly Ile Gly Leu Ser Val Ala Lys Lys Phe Leu Gln Leu Gly Ala
            20                  25                  30
Lys Val Thr Ile Ser Asp Val Ser Gly Glu Lys Lys Tyr His Glu Thr
        35                  40                  45
Val Val Ala Leu Lys Ala Gln Asn Leu Asn Thr Asp Asn Leu His Tyr
    50                  55                  60
Val Gln Ala Asp Ser Ser Lys Glu Glu Asp Asn Lys Lys Leu Ile Ser
65                  70                  75                  80
Glu Thr Leu Ala Thr Phe Gly Gly Leu Asp Ile Val Cys Ala Asn Ala
                85                  90                  95
Gly Ile Gly Lys Phe Ala Pro Thr His Glu Thr Pro Phe Asp Val Trp
            100                 105                 110
Lys Lys Val Ile Ala Val Asn Leu Asn Gly Val Phe Leu Leu Asp Lys
        115                 120                 125
Leu Ala Ile Asn Tyr Trp Leu Glu Lys Ser Lys Pro Gly Val Ile Val
    130                 135                 140
Asn Met Gly Ser Val His Ser Phe Val Ala Ala Pro Gly Leu Ala His
145                 150                 155                 160
Tyr Gly Ala Ala Lys Gly Gly Val Lys Leu Leu Thr Gln Thr Leu Ala
                165                 170                 175
Leu Glu Tyr Ala Ser His Gly Ile Arg Val Asn Ser Val Asn Pro Gly
            180                 185                 190
Tyr Ile Ser Thr Pro Leu Ile Asp Glu Val Pro Lys Glu Arg Leu Asp
        195                 200                 205
Lys Leu Val Ser Leu His Pro Ile Gly Arg Leu Gly Arg Pro Glu Glu
    210                 215                 220
Val Ala Asp Ala Val Ala Phe Leu Cys Ser Gln Glu Ala Thr Phe Ile
225                 230                 235                 240
Asn Gly Val Ser Leu Pro Val Asp Gly Gly Tyr Thr Ala Gln
                245                 250

<210> 18
<211> 765
<212> DNA
<213> Pichia finlandica

<220>
<221> source
<222> 1..765
<223> /mol_type="DNA"
      /organism="Pichia finlandica"

<400> 18
atgtcttata atttccataa caaggttgca gttgttactg gagctctatc aggaatcggc      60

ttaagcgtcg caaaaaagtt ccttcagctc ggcgccaaag taacgatctc tgatgtcagt     120

ggagagaaaa aatatcacga gactgttgtt gctctgaaag cccaaaatct caacactgac     180

aacctccatt atgtacaggc agattccagc aaagaagaag ataacaagaa attgatttcg     240

gaaactctgg caacctttgg gggcctggat attgtttgtg ctaatgcagg aattggaaag     300

ttcgctccca cccatgaaac acccttcgac gtatggaaga aggtgattgc tgtgaatttg 360

aatggagtat tcttactgga taagctagcc atcaattact ggctagagaa aagcaaaccc 420

ggcgtaattg tcaacatggg atcagtccac tcttttgtag cagctcctgg ccttgcgcat 480

tatggagctg caaaaggcgg tgtcaaactg ttaacacaaa cattggctct ggagtacgca 540

tctcatggta ttcgtgtaaa ttctgtcaat ccggggtaca tttcgactcc tttgatagat 600

gaggttccga aagagcggtt ggataaactt gtaagcctgc accctattgg gcgtctgggt 660

cgtccagagg aagttgctga tgcagtcgca tttctgtgtt cccaggaggc cactttcatc 720

aacggcgttt ctttgccggt tgacggtggg tacacagcac aataa 765

<210> 19
<211> 282
<212> PRT
<213> Clostridium acetobutylicum

<220>
<221> SOURCE
<222> 1..282
<223> /mol_type="protein"
/organism="Clostridium acetobutylicum"

<400> 19
Met Lys Lys Val Cys Val Ile Gly Ala Gly Thr Met Gly Ser Gly Ile
1               5                   10                  15
Ala Gln Ala Phe Ala Ala Lys Gly Phe Glu Val Val Leu Arg Asp Ile
            20                  25                  30
Lys Asp Glu Phe Val Asp Arg Gly Leu Asp Phe Ile Asn Lys Asn Leu
        35                  40                  45
Ser Lys Leu Val Lys Lys Gly Lys Ile Glu Glu Ala Thr Lys Val Glu
    50                  55                  60
Ile Leu Thr Arg Ile Ser Gly Thr Val Asp Leu Asn Met Ala Ala Asp
65                  70                  75                  80
Cys Asp Leu Val Ile Glu Ala Ala Val Glu Arg Met Asp Ile Lys Lys
                85                  90                  95
Gln Ile Phe Ala Asp Leu Asp Asn Ile Cys Lys Pro Glu Thr Ile Leu
            100                 105                 110
Ala Ser Asn Thr Ser Ser Leu Ser Ile Thr Glu Val Ala Ser Ala Thr
        115                 120                 125
Lys Arg Pro Asp Lys Val Ile Gly Met His Phe Phe Asn Pro Ala Pro
    130                 135                 140
Val Met Lys Leu Val Glu Val Ile Arg Gly Ile Ala Thr Ser Gln Glu
145                 150                 155                 160
Thr Phe Asp Ala Val Lys Glu Thr Ser Ile Ala Ile Gly Lys Asp Pro
                165                 170                 175
Val Glu Val Ala Glu Ala Pro Gly Phe Val Val Asn Arg Ile Leu Ile
            180                 185                 190
Pro Met Ile Asn Glu Ala Val Gly Ile Leu Ala Glu Gly Ile Ala Ser
        195                 200                 205
Val Glu Asp Ile Asp Lys Ala Met Lys Leu Gly Ala Asn His Pro Met
    210                 215                 220
Gly Pro Leu Glu Leu Gly Asp Phe Ile Gly Leu Asp Ile Cys Leu Ala
225                 230                 235                 240
Ile Met Asp Val Leu Tyr Ser Glu Thr Gly Asp Ser Lys Tyr Arg Pro
                245                 250                 255
His Thr Leu Leu Lys Lys Tyr Val Arg Ala Gly Trp Leu Gly Arg Lys
            260                 265                 270
Ser Gly Lys Gly Phe Tyr Asp Tyr Ser Lys
        275                 280

<210> 20
<211> 849
<212> DNA
<213> Clostridium acetobutylicum

<220>
<221> source
<222> 1..849
<223> /mol_type="DNA"
      /organism="Clostridium acetobutylicum"

<400> 20
atgaaaaagg tatgtgttat aggtgcaggt actatgggtt caggaattgc tcaggcattt      60

gcagctaaag gatttgaagt agtattaaga gatattaaag atgaatttgt tgatagagga     120

ttagatttta tcaataaaaa tctttctaaa ttagttaaaa aaggaaagat agaagaagct     180

actaaagttg aaatcttaac tagaatttcc ggaacagttg accttaatat ggcagctgat     240

tgcgatttag ttatagaagc agctgttgaa agaatggata ttaaaaagca gatttttgct     300

gacttagaca atatatgcaa gccagaaaca attcttgcat caaatacatc atcactttca     360

ataacagaag tggcatcagc aactaaaaga cctgataagg ttataggtat gcatttcttt     420

aatccagctc ctgttatgaa gcttgtagag gtaataagag gaatagctac atcacaagaa     480

acttttgatg cagttaaaga gacatctata gcaataggaa aagatcctgt agaagtagca     540

gaagcaccag gatttgttgt aaatagaata ttaataccaa tgattaatga agcagttggt     600

atattagcag aaggaatagc ttcagtagaa gacatagata aagctatgaa acttggagct     660

aatcacccaa tgggaccatt agaattaggt gattttatag gtcttgatat atgtcttgct     720

ataatggatg ttttatactc agaaactgga gattctaagt atagaccaca tacattactt     780

aagaagtatg taagagcagg atggcttgga agaaaatcag gaaaaggttt ctacgattat     840

tcaaaataa                                                             849


<210> 21
<211> 393
<212> PRT
<213> Cupriavidus necator

<220>
<221> SOURCE
<222> 1..393
<223> /mol_type="protein"
      /organism="Cupriavidus necator"

<400> 21
Met Thr Asp Val Val Ile Val Ser Ala Ala Arg Thr Ala Val Gly Lys
1               5                   10                  15
Phe Gly Gly Ser Leu Ala Lys Ile Pro Ala Pro Glu Leu Gly Ala Val
            20                  25                  30
Val Ile Lys Ala Ala Leu Glu Arg Ala Gly Val Lys Pro Glu Gln Val
        35                  40                  45
Ser Glu Val Ile Met Gly Gln Val Leu Thr Ala Gly Ser Gly Gln Asn
    50                  55                  60
Pro Ala Arg Gln Ala Ala Ile Lys Ala Gly Leu Pro Ala Met Val Pro

65                  70                  75                  80
Ala Met Thr Ile Asn Lys Val Cys Gly Ser Gly Leu Lys Ala Val Met
                85                  90                  95
Leu Ala Ala Asn Ala Ile Met Ala Gly Asp Ala Glu Ile Val Val Ala
            100                 105                 110
Gly Gly Gln Glu Asn Met Ser Ala Ala Pro His Val Leu Pro Gly Ser
        115                 120                 125
Arg Asp Gly Phe Arg Met Gly Asp Ala Lys Leu Val Asp Thr Met Ile
    130                 135                 140
Val Asp Gly Leu Trp Asp Val Tyr Asn Gln Tyr His Met Gly Ile Thr
145                 150                 155                 160
Ala Glu Asn Val Ala Lys Glu Tyr Gly Ile Thr Arg Glu Ala Gln Asp
                165                 170                 175
Glu Phe Ala Val Gly Ser Gln Asn Lys Ala Glu Ala Ala Gln Lys Ala
            180                 185                 190
Gly Lys Phe Asp Glu Glu Ile Val Pro Val Leu Ile Pro Gln Arg Lys
        195                 200                 205
Gly Asp Pro Val Ala Phe Lys Thr Asp Glu Phe Val Arg Gln Gly Ala
    210                 215                 220
Thr Leu Asp Ser Met Ser Gly Leu Lys Pro Ala Phe Asp Lys Ala Gly
225                 230                 235                 240
Thr Val Thr Ala Ala Asn Ala Ser Gly Leu Asn Asp Gly Ala Ala Ala
                245                 250                 255
Val Val Val Met Ser Ala Ala Lys Ala Lys Glu Leu Gly Leu Thr Pro
            260                 265                 270
Leu Ala Thr Ile Lys Ser Tyr Ala Asn Ala Gly Val Asp Pro Lys Val
        275                 280                 285
Met Gly Met Gly Pro Val Pro Ala Ser Lys Arg Ala Leu Ser Arg Ala
    290                 295                 300
Glu Trp Thr Pro Gln Asp Leu Asp Leu Met Glu Ile Asn Glu Ala Phe
305                 310                 315                 320
Ala Ala Gln Ala Leu Ala Val His Gln Gln Met Gly Trp Asp Thr Ser
                325                 330                 335
Lys Val Asn Val Asn Gly Gly Ala Ile Ala Ile Gly His Pro Ile Gly
            340                 345                 350
Ala Ser Gly Cys Arg Ile Leu Val Thr Leu Leu His Glu Met Lys Arg
        355                 360                 365
Arg Asp Ala Lys Lys Gly Leu Ala Ser Leu Cys Ile Gly Gly Gly Met
    370                 375                 380
Gly Val Ala Leu Ala Val Glu Arg Lys
385                 390

<210> 22
<211> 1182
<212> DNA
<213> Cupriavidus necator

<220>
<221> source
<222> 1..1182
<223> /mol_type="DNA"
      /organism="Cupriavidus necator"

<400> 22
atgactgacg ttgtcatcgt atccgccgcc cgcaccgcgg tcggcaagtt tggcggctcg      60

ctggccaaga tcccggcacc ggaactgggt gccgtggtca tcaaggccgc gctggagcgc     120

gccggcgtca agccggagca ggtgagcgaa gtcatcatgg gccaggtgct gaccgccggt     180

tcgggccaga accccgcacg ccaggccgcg atcaaggccg gcctgccggc gatggtgccg     240

gccatgacca tcaacaaggt gtgcggctcg ggcctgaagg ccgtgatgct ggccgccaac     300

gcgatcatgg cgggcgacgc cgagatcgtg gtggccggcg gccaggaaaa catgagcgcc     360

gccccgcacg tgctgccggg ctcgcgcgat ggtttccgca tgggcgatgc caagctggtc 420

gacaccatga tcgtcgacgg cctgtgggac gtgtacaacc agtaccacat gggcatcacc 480

gccgagaacg tggccaagga atacggcatc acacgcgagg cgcaggatga gttcgccgtc 540

ggctcgcaga acaaggccga agccgcgcag aaggccggca agtttgacga agagatcgtc 600

ccggtgctga tcccgcagcg caagggcgac ccggtggcct tcaagaccga cgagttcgtg 660

cgccagggcg ccacgctgga cagcatgtcc ggcctcaagc ccgccttcga caaggccggc 720

acggtgaccg cggccaacgc ctcgggcctg aacgacggcg ccgccgcggt ggtggtgatg 780

tcggcggcca aggccaagga actgggcctg accccgctgg ccacgatcaa gagctatgcc 840

aacgccggtg tcgatcccaa ggtgatgggc atgggcccgg tgccggcctc caagcgcgcc 900

ctgtcgcgcg ccgagtggac cccgcaagac ctggacctga tggagatcaa cgaggccttt 960

gccgcgcagg cgctggcggt gcaccagcag atgggctggg acacctccaa ggtcaatgtg 1020

aacggcggcg ccatcgccat cggccacccg atcggcgcgt cgggctgccg tatcctggtg 1080

acgctgctgc acgagatgaa gcgccgtgac gcgaagaagg gcctggcctc gctgtgcatc 1140

ggcggcggca tgggcgtggc gctggcagtc gagcgcaaat aa 1182

<210> 23
<211> 393
<212> PRT
<213> Zoogloea ramigera

<220>
<221> SOURCE
<222> 1..393
<223> /mol_type="protein"
/organism="Zoogloea ramigera"

<400> 23
Met Ser Thr Pro Ser Ile Val Ile Ala Ser Ala Ala Arg Thr Ala Val
1               5                   10                  15
Gly Ser Phe Asn Gly Ala Phe Ala Asn Thr Pro Ala His Glu Leu Gly
            20                  25                  30
Ala Thr Val Ile Ser Ala Val Leu Glu Arg Ala Gly Val Ala Ala Gly
        35                  40                  45
Glu Val Asn Glu Val Ile Leu Gly Gln Val Leu Pro Ala Gly Glu Gly
    50                  55                  60
Gln Asn Pro Ala Arg Gln Ala Ala Met Lys Ala Gly Val Pro Gln Glu
65                  70                  75                  80
Ala Thr Ala Trp Gly Met Asn Gln Leu Cys Gly Ser Gly Leu Arg Ala
                85                  90                  95
Val Ala Leu Gly Met Gln Gln Ile Ala Thr Gly Asp Ala Ser Ile Ile
            100                 105                 110
Val Ala Gly Gly Met Glu Ser Met Ser Met Ala Pro His Cys Ala His
        115                 120                 125
Leu Arg Gly Gly Val Lys Met Gly Asp Phe Lys Met Ile Asp Thr Met
    130                 135                 140
Ile Lys Asp Gly Leu Thr Asp Ala Phe Tyr Gly Tyr His Met Gly Thr
145                 150                 155                 160
Thr Ala Glu Asn Val Ala Lys Gln Trp Gln Leu Ser Arg Asp Glu Gln
                165                 170                 175
Asp Ala Phe Ala Val Ala Ser Gln Asn Lys Ala Glu Ala Ala Gln Lys

180                 185                 190
Asp Gly Arg Phe Lys Asp Glu Ile Val Pro Phe Ile Val Lys Gly Arg
        195                 200                 205
Lys Gly Asp Ile Thr Val Asp Ala Asp Glu Tyr Ile Arg His Gly Ala
    210                 215                 220
Thr Leu Asp Ser Met Ala Lys Leu Arg Pro Ala Phe Asp Lys Glu Gly
225                 230                 235                 240
Thr Val Thr Ala Gly Asn Ala Ser Gly Leu Asn Asp Gly Ala Ala Ala
                245                 250                 255
Ala Leu Leu Met Ser Glu Ala Glu Ala Ser Arg Arg Gly Ile Gln Pro
            260                 265                 270
Leu Gly Arg Ile Val Ser Trp Ala Thr Val Gly Val Asp Pro Lys Val
        275                 280                 285
Met Gly Thr Gly Pro Ile Pro Ala Ser Arg Lys Ala Leu Glu Arg Ala
    290                 295                 300
Gly Trp Lys Ile Gly Asp Leu Asp Leu Val Glu Ala Asn Glu Ala Phe
305                 310                 315                 320
Ala Ala Gln Ala Cys Ala Val Asn Lys Asp Leu Gly Trp Asp Pro Ser
                325                 330                 335
Ile Val Asn Val Asn Gly Gly Ala Ile Ala Ile Gly His Pro Ile Gly
            340                 345                 350
Ala Ser Gly Ala Arg Ile Leu Asn Thr Leu Leu Phe Glu Met Lys Arg
        355                 360                 365
Arg Gly Ala Arg Lys Gly Leu Ala Thr Leu Cys Ile Gly Gly Gly Met
    370                 375                 380
Gly Val Ala Met Cys Ile Glu Ser Leu
385                 390

<210> 24
<211> 1182
<212> DNA
<213> Zoogloea ramigera

<220>
<221> source
<222> 1..1182
<223> /mol_type="DNA"
      /organism="Zoogloea ramigera"

<400> 24
atgagtactc catcaatcgt catcgccagc gccgcccgca ccgcggtcgg ttccttcaac      60

ggcgctttcg ccaacacgcc cgcccatgaa ctcggggcga ccgtgatttc ggcggttctc     120

gagcgcgcgg gcgttgcggc gggcgaggtg aacgaggtga ttctcggcca ggtgctgccg     180

gccggcgaag gccagaaccc ggcccgccag gccgccatga aggccggcgt gccgcaggag     240

gcgaccgcct ggggcatgaa ccagctttgc ggctcgggcc tgcgcgccgt cgcgctcggc     300

atgcagcaga tcgccacggg cgatgcgagc atcatcgtcg ccggcggcat ggaatccatg     360

tcaatggccc cgcattgcgc gcatctgcgc ggcggcgtga agatgggcga tttcaagatg     420

atcgacacga tgatcaagga cggcctgacc gacgccttct acggctacca catgggcacg     480

accgccgaga atgtcgccaa gcagtggcag ctttcccgcg acgagcagga cgccttcgcc     540

gtcgcctcgc agaacaaggc cgaggccgcc cagaaggacg gccgcttcaa ggacgagatc     600

gttcccttca tcgtcaaggg ccgcaagggc gacatcacgg tcgatgccga cgaatatatc     660

cgccacggcg cgacgctcga ttcaatggcg aagctccgcc cggccttcga caaggaaggc     720

acggtgacgg ccggcaacgc ctccggcctc aatgacggcg cggccgcggc cctcctgatg     780

agcgaagcgg aagcctcgcg ccgcggcatc cagccgctcg gccgcatcgt ttcctgggcg     840

acggtcggcg tcgatcccaa ggtcatgggc accggcccga tcccggcctc ccgcaaggcg     900

ctcgagcgcg ccggctggaa gatcggcgat ctcgacctcg tggaagccaa cgaagccttc     960

gcggcgcagg cctgcgcggt caacaaggac ctcggctggg atccgtccat cgtcaacgtc    1020

aacggcggtg ccatcgccat cggccacccg atcggcgcgt ccggcgcccg catcctcaac    1080

acgctcctct tcgagatgaa gcgtcgcggc gcccgcaagg gtctcgccac gctctgcatc    1140

ggcggcggca tgggcgtggc gatgtgcatc gaaagtcttt aa                       1182

<210> 25
<211> 394
<212> PRT
<213> Escherichia coli

<220>
<221> SOURCE
<222> 1..394
<223> /mol_type="protein"
      /organism="Escherichia coli"

<400> 25
Met Lys Asn Cys Val Ile Val Ser Ala Val Arg Thr Ala Ile Gly Ser
1               5                   10                  15
Phe Asn Gly Ser Leu Ala Ser Thr Ser Ala Ile Asp Leu Gly Ala Thr
            20                  25                  30
Val Ile Lys Ala Ala Ile Glu Arg Ala Lys Ile Asp Ser Gln His Val
        35                  40                  45
Asp Glu Val Ile Met Gly Asn Val Leu Gln Ala Gly Leu Gly Gln Asn
    50                  55                  60
Pro Ala Arg Gln Ala Leu Leu Lys Ser Gly Leu Ala Glu Thr Val Cys
65                  70                  75                  80
Gly Phe Thr Val Asn Lys Val Cys Gly Ser Gly Leu Lys Ser Val Ala
                85                  90                  95
Leu Ala Ala Gln Ala Ile Gln Ala Gly Gln Ala Gln Ser Ile Val Ala
            100                 105                 110
Gly Gly Met Glu Asn Met Ser Leu Ala Pro Tyr Leu Leu Asp Ala Lys
        115                 120                 125
Ala Arg Ser Gly Tyr Arg Leu Gly Asp Gly Gln Val Tyr Asp Val Ile
    130                 135                 140
Leu Arg Asp Gly Leu Met Cys Ala Thr His Gly Tyr His Met Gly Ile
145                 150                 155                 160
Thr Ala Glu Asn Val Ala Lys Glu Tyr Gly Ile Thr Arg Glu Met Gln
                165                 170                 175
Asp Glu Leu Ala Leu His Ser Gln Arg Lys Ala Ala Ala Ala Ile Glu
            180                 185                 190
Ser Gly Ala Phe Thr Ala Glu Ile Val Pro Val Asn Val Val Thr Arg
        195                 200                 205
Lys Lys Thr Phe Val Phe Ser Gln Asp Glu Phe Pro Lys Ala Asn Ser
    210                 215                 220
Thr Ala Glu Ala Leu Gly Ala Leu Arg Pro Ala Phe Asp Lys Ala Gly
225                 230                 235                 240
Thr Val Thr Ala Gly Asn Ala Ser Gly Ile Asn Asp Gly Ala Ala Ala
                245                 250                 255
Leu Val Ile Met Glu Glu Ser Ala Ala Leu Ala Ala Gly Leu Thr Pro
            260                 265                 270
Leu Ala Arg Ile Lys Ser Tyr Ala Ser Gly Gly Val Pro Pro Ala Leu
        275                 280                 285
Met Gly Met Gly Pro Val Pro Ala Thr Gln Lys Ala Leu Gln Leu Ala

290                 295                 300
Gly Leu Gln Leu Ala Asp Ile Asp Leu Ile Glu Ala Asn Glu Ala Phe
305                 310                 315                 320
Ala Ala Gln Phe Leu Ala Val Gly Lys Asn Leu Gly Phe Asp Ser Glu
                325                 330                 335
Lys Val Asn Val Asn Gly Gly Ala Ile Ala Leu Gly His Pro Ile Gly
            340                 345                 350
Ala Ser Gly Ala Arg Ile Leu Val Thr Leu Leu His Ala Met Gln Ala
        355                 360                 365
Arg Asp Lys Thr Leu Gly Leu Ala Thr Leu Cys Ile Gly Gly Gly Gln
    370                 375                 380
Gly Ile Ala Met Val Ile Glu Arg Leu Asn
385                 390

<210> 26
<211> 1185
<212> DNA
<213> Escherichia coli

<220>
<221> source
<222> 1..1185
<223> /mol_type="DNA"
      /organism="Escherichia coli"

<400> 26
atgaaaaatt gtgtcatcgt cagtgcggta cgtactgcta tcggtagttt taacggttca      60

ctcgcttcca ccagcgccat cgacctgggg gcgacagtaa ttaaagccgc cattgaacgt     120

gcaaaaatcg attcacaaca cgttgatgaa gtgattatgg gtaacgtgtt acaagccggg     180

ctggggcaaa atccggcgcg tcaggcactg ttaaaaagcg ggctggcaga aacggtgtgc     240

ggattcacgg tcaataaagt atgtggttcg ggtcttaaaa gtgtggcgct tgccgcccag     300

gccattcagg caggtcaggc gcagagcatt gtggcggggg gtatggaaaa tatgagttta     360

gccccctact tactcgatgc aaaagcacgc tctggttatc gtcttggaga cggacaggtt     420

tatgacgtaa tcctgcgcga tggcctgatg tgcgccaccc atggttatca tatggggatt     480

accgccgaaa acgtggctaa agagtacgga attacccgtg aaatgcagga tgaactggcg     540

ctacattcac agcgtaaagc ggcagccgca attgagtccg gtgcttttac agccgaaatc     600

gtcccggtaa atgttgtcac tcgaaagaaa accttcgtct tcagtcaaga cgaattcccg     660

aaagcgaatt caacggctga agcgttaggt gcattgcgcc cggccttcga taaagcagga     720

acagtcaccg ctgggaacgc gtctggtatt aacgacggtg ctgccgctct ggtgattatg     780

gaagaatctg cggcgctggc agcaggcctt acccccctgg ctcgcattaa aagttatgcc     840

agcggtggcg tgccccccgc attgatgggt atggggccag tacctgccac gcaaaaagcg     900

ttacaactgg cggggctgca actggcggat attgatctca ttgaggctaa tgaagcattt     960

gctgcacagt tccttgccgt tgggaaaaac ctgggctttg attctgagaa agtgaatgtc    1020

aacggcgggg ccatcgcgct cgggcatcct atcggtgcca gtggtgctcg tattctggtc    1080

acactattac atgccatgca ggcacgcgat aaaacgctgg ggctggcaac actgtgcatt    1140

ggcggcggtc agggaattgc gatggtgatt gaacgcttga attaa                    1185

<210> 27
<211> 38
<212> DNA
<213> Artificial Sequence

<220>
<221> source
<222> 1..38
<223> /mol_type="DNA"
      /note="An artificially synthesized primer sequence"
      /organism="Artificial Sequence"

<400> 27
gacggtacct atatatgact gatgttgtca tcgtatcc                              38


<210> 28
<211> 34
<212> DNA
<213> Artificial Sequence

<220>
<221> source
<222> 1..34
<223> /mol_type="DNA"
      /note="An artificially synthesized primer sequence"
      /organism="Artificial Sequence"

<400> 28
cacaagctta ttatttacgt tcaactgcca gcgc                                  34


<210> 29
<211> 35
<212> DNA
<213> Artificial Sequence

<220>
<221> source
<222> 1..35
<223> /mol_type="DNA"
      /note="An artificially synthesized primer sequence"
      /organism="Artificial Sequence"

<400> 29
gacggtacct atatatgagt actccatcaa tcgtc                                 35


<210> 30
<211> 34
<212> DNA
<213> Artificial Sequence

<220>
<221> source
<222> 1..34
<223> /mol_type="DNA"
      /note="An artificially synthesized primer sequence"
      /organism="Artificial Sequence"

<400> 30
cacaagctta ttaaagactt tcgatgcaca tcgc                                  34

<210> 31
<211> 22
<212> DNA
<213> Artificial Sequence

<220>
<221> source
<222> 1..22
<223> /mol_type="DNA"
      /note="An artificially synthesized primer sequence"
      /organism="Artificial Sequence"

<400> 31
ggaatccatg tcaatggccc cg                                              22


<210> 32
<211> 20
<212> DNA
<213> Artificial Sequence

<220>
<221> source
<222> 1..20
<223> /mol_type="DNA"
      /note="An artificially synthesized primer sequence"
      /organism="Artificial Sequence"

<400> 32
gctcgattca atggcgaagc                                                 20


<210> 33
<211> 22
<212> DNA
<213> Artificial Sequence

<220>
<221> source
<222> 1..22
<223> /mol_type="DNA"
      /note="An artificially synthesized primer sequence"
      /organism="Artificial Sequence"

<400> 33
caatgcgggg ccattgacat gg                                              22


<210> 34
<211> 23
<212> DNA
<213> Artificial Sequence

<220>
<221> source
<222> 1..23
<223> /mol_type="DNA"
      /note="An artificially synthesized primer sequence"
      /organism="Artificial Sequence"

<400> 34
cggagcttcg ccattgaatc gag                                             23

<210> 35
<211> 37
<212> DNA
<213> Artificial Sequence

<220>
<221> source
<222> 1..37
<223> /mol_type="DNA"
      /note="An artificially synthesized primer sequence"
      /organism="Artificial Sequence"

<400> 35
gacggtacct atatatgaaa aattgtgtca tcgtcag                                37


<210> 36
<211> 35
<212> DNA
<213> Artificial Sequence

<220>
<221> source
<222> 1..35
<223> /mol_type="DNA"
      /note="An artificially synthesized primer sequence"
      /organism="Artificial Sequence"

<400> 36
cacaagctta ttaattcaag cgttcaatca ccatc                                  35


<210> 37
<211> 37
<212> DNA
<213> Artificial Sequence

<220>
<221> source
<222> 1..37
<223> /mol_type="DNA"
      /note="An artificially synthesized primer sequence"
      /organism="Artificial Sequence"

<400> 37
gaggaattca tatatgactc aacgtattgc gtatgtg                                37


<210> 38
<211> 29
<212> DNA
<213> Artificial Sequence

<220>
<221> source
<222> 1..29
<223> /mol_type="DNA"
      /note="An artificially synthesized primer sequence"
      /organism="Artificial Sequence"

<400> 38
cagactagta ttagcccatg tgcaggccg                                         29


<210> 39

<211> 23
<212> DNA
<213> Artificial Sequence

<220>
<221> source
<222> 1..23
<223> /mol_type="DNA"
      /note="An artificially synthesized primer sequence"
      /organism="Artificial Sequence"

<400> 39
catggcttca ctatggcact ggc                                               23


<210> 40
<211> 23
<212> DNA
<213> Artificial Sequence

<220>
<221> source
<222> 1..23
<223> /mol_type="DNA"
      /note="An artificially synthesized primer sequence"
      /organism="Artificial Sequence"

<400> 40
gccagtgcca tagtgaagcc atg                                               23


<210> 41
<211> 36
<212> DNA
<213> Artificial Sequence

<220>
<221> source
<222> 1..36
<223> /mol_type="DNA"
      /note="An artificially synthesized primer sequence"
      /organism="Artificial Sequence"

<400> 41
gaggaattca tatatgagtc gtgtagcatt ggtaac                                  36


<210> 42
<211> 29
<212> DNA
<213> Artificial Sequence

<220>
<221> source
<222> 1..29
<223> /mol_type="DNA"
      /note="An artificially synthesized primer sequence"
      /organism="Artificial Sequence"

<400> 42
cagactagta ttagacgaag aactggccg                                         29


<210> 43
<211> 31

<212> DNA
<213> Artificial Sequence

<220>
<221> source
<222> 1..31
<223> /mol_type="DNA"
      /note="An artificially synthesized primer sequence"
      /organism="Artificial Sequence"

<400> 43
gaggaattca tatatggcca cgcaggactc c                                    31


<210> 44
<211> 29
<212> DNA
<213> Artificial Sequence

<220>
<221> source
<222> 1..29
<223> /mol_type="DNA"
      /note="An artificially synthesized primer sequence"
      /organism="Artificial Sequence"

<400> 44
cagactagta ttagtagttc cccagcccg                                       29


<210> 45
<211> 35
<212> DNA
<213> Artificial Sequence

<220>
<221> source
<222> 1..35
<223> /mol_type="DNA"
      /note="An artificially synthesized primer sequence"
      /organism="Artificial Sequence"

<400> 45
gaggaattca tatatgtctc aacgttttgc aggtc                                 35


<210> 46
<211> 31
<212> DNA
<213> Artificial Sequence

<220>
<221> source
<222> 1..31
<223> /mol_type="DNA"
      /note="An artificially synthesized primer sequence"
      /organism="Artificial Sequence"

<400> 46
cagactagta ttaacatttt ggaccacctg c                                    31


<210> 47
<211> 39
<212> DNA

<213> Artificial Sequence

<220>
<221> source
<222> 1..39
<223> /mol_type="DNA"
      /note="An artificially synthesized primer sequence"
      /organism="Artificial Sequence"

<400> 47
gaggaattca tatatgaaaa aggtatgtgt tataggtgc                              39


<210> 48
<211> 40
<212> DNA
<213> Artificial Sequence

<220>
<221> source
<222> 1..40
<223> /mol_type="DNA"
      /note="An artificially synthesized primer sequence"
      /organism="Artificial Sequence"

<400> 48
cagactagta ttattttgaa taatcgtaga aaccttttcc                            40


<210> 49
<211> 42
<212> DNA
<213> Artificial Sequence

<220>
<221> source
<222> 1..42
<223> /mol_type="DNA"
      /note="An artificially synthesized primer sequence"
      /organism="Artificial Sequence"

<400> 49
gaggaattct aaaatgtctt ataatttcca taacaaggtt gc                          42


<210> 50
<211> 38
<212> DNA
<213> Artificial Sequence

<220>
<221> source
<222> 1..38
<223> /mol_type="DNA"
      /note="An artificially synthesized primer sequence"
      /organism="Artificial Sequence"

<400> 50
tcgactagta ttattgtgct gtgtacccac cgtcaacc                              38


<210> 51
<211> 39
<212> DNA
<213> Artificial Sequence

<220>
<221> source
<222> 1..39
<223> /mol_type="DNA"
      /note="An artificially synthesized primer sequence"
      /organism="Artificial Sequence"

<400> 51
ggctctggag tacgcatctc atggtattcg tgtaaattc                          39


<210> 52
<211> 39
<212> DNA
<213> Artificial Sequence

<220>
<221> source
<222> 1..39
<223> /mol_type="DNA"
      /note="An artificially synthesized primer sequence"
      /organism="Artificial Sequence"

<400> 52
gaatttacac gaataccatg agatgcgtac tccagagcc                          39


<210> 53
<211> 34
<212> DNA
<213> Artificial Sequence

<220>
<221> source
<222> 1..34
<223> /mol_type="DNA"
      /note="An artificially synthesized primer sequence"
      /organism="Artificial Sequence"

<400> 53
gtaagcctgc accctattgg gcgtctgggt cgtc                               34


<210> 54
<211> 34
<212> DNA
<213> Artificial Sequence

<220>
<221> source
<222> 1..34
<223> /mol_type="DNA"
      /note="An artificially synthesized primer sequence"
      /organism="Artificial Sequence"

<400> 54
gacgacccag acgcccaata gggtgcaggc ttac                               34


<210> 55
<211> 37
<212> DNA
<213> Artificial Sequence

<220>
<221> source
<222> 1..37
<223> /mol_type="DNA"
      /note="An artificially synthesized primer sequence"
      /organism="Artificial Sequence"

<400> 55
gaccatatga aagttacaaa tcaaaaagaa ctaaaac                              37


<210> 56
<211> 40
<212> DNA
<213> Artificial Sequence

<220>
<221> source
<222> 1..40
<223> /mol_type="DNA"
      /note="An artificially synthesized primer sequence"
      /organism="Artificial Sequence"

<400> 56
ctgttaatta aaatgatttt atatagatat ccttaagttc                           40


<210> 57
<211> 21
<212> DNA
<213> Artificial Sequence

<220>
<221> source
<222> 1..21
<223> /mol_type="DNA"
      /note="An artificially synthesized primer sequence"
      /organism="Artificial Sequence"

<400> 57
ctatagaagc atacgtttcg g                                               21


<210> 58
<211> 21
<212> DNA
<213> Artificial Sequence

<220>
<221> source
<222> 1..21
<223> /mol_type="DNA"
      /note="An artificially synthesized primer sequence"
      /organism="Artificial Sequence"

<400> 58
ccgaaacgta tgcttctata g                                               21


<210> 59
<211> 39
<212> DNA
<213> Artificial Sequence

<220>

<221> source
<222> 1..39
<223> /mol_type="DNA"
      /note="An artificially synthesized primer sequence"
      /organism="Artificial Sequence"

<400> 59
ggaccatggt tgatttcgaa tattcaatac caactagaa                              39


<210> 60
<211> 47
<212> DNA
<213> Artificial Sequence

<220>
<221> source
<222> 1..47
<223> /mol_type="DNA"
      /note="An artificially synthesized primer sequence"
      /organism="Artificial Sequence"

<400> 60
cgatctagaa ttacacagat tttttgaata tttgtaggac ttcggag                     47


<210> 61
<211> 24
<212> DNA
<213> Artificial Sequence

<220>
<221> source
<222> 1..24
<223> /mol_type="DNA"
      /note="An artificially synthesized primer sequence"
      /organism="Artificial Sequence"

<400> 61
gatggaaatc cgtgggatat tgtg                                              24


<210> 62
<211> 24
<212> DNA
<213> Artificial Sequence

<220>
<221> source
<222> 1..24
<223> /mol_type="DNA"
      /note="An artificially synthesized primer sequence"
      /organism="Artificial Sequence"

<400> 62
cacaatatcc cacggatttc catc                                              24


<210> 63
<211> 27
<212> DNA
<213> Artificial Sequence

<220>
<221> source

<222> 1..27
<223> /mol_type="DNA"
      /note="An artificially synthesized primer sequence"
      /organism="Artificial Sequence"

<400> 63
gtttaccatc tcgtctgcgt gatgttg                                          27


<210> 64
<211> 27
<212> DNA
<213> Artificial Sequence

<220>
<221> source
<222> 1..27
<223> /mol_type="DNA"
      /note="An artificially synthesized primer sequence"
      /organism="Artificial Sequence"

<400> 64
caacatcacg cagacgagat ggtaaac                                          27


<210> 65
<211> 874
<212> PRT
<213> Thermoanaerobacter pseudethanolicus

<220>
<221> SOURCE
<222> 1..874
<223> /mol_type="protein"
      /organism="Thermoanaerobacter pseudethanolicus"

<400> 65
Met Pro Asn Leu Leu Gln Glu Arg Arg Glu Val Lys Glu Lys Thr Glu
1               5                   10                  15
Val Lys Glu Thr Leu Asp Val Lys Arg Gln Ile Asp Gln Leu Val Glu
            20                  25                  30
Lys Ala Gln Arg Ala Gln Glu Lys Phe Met Ser Tyr Thr Gln Glu Gln
        35                  40                  45
Ile Asp Lys Ile Val Lys Ala Met Ala Leu Ala Gly Ile Glu Asn His
    50                  55                  60
Val Arg Leu Ala Lys Leu Ala His Glu Glu Thr Lys Met Gly Val Tyr
65                  70                  75                  80
Glu Asp Lys Ile Thr Lys Asn Leu Phe Ala Val Glu Tyr Val Tyr Asn
                85                  90                  95
Tyr Ile Lys Asp Lys Lys Thr Val Gly Val Leu Ser Glu Asn Leu Glu
            100                 105                 110
Glu Asn Tyr Met Glu Val Ala Glu Pro Val Gly Val Ile Ala Gly Val
        115                 120                 125
Thr Pro Val Thr Asn Pro Thr Ser Thr Thr Met Phe Lys Ser Leu Ile
    130                 135                 140
Ala Ile Lys Thr Arg Asn Pro Ile Ile Phe Ser Phe His Pro Lys Ala
145                 150                 155                 160
Leu Lys Cys Ser Ile Glu Thr Ala Lys Thr Met Tyr Glu Ala Ala Leu
                165                 170                 175
Lys Ala Gly Ala Pro Glu Gly Cys Ile Gly Trp Ile Glu Thr Pro Ser
            180                 185                 190
Ile Glu Ala Thr Gln Leu Leu Met Thr His Pro Gly Val Ser Leu Ile
        195                 200                 205
Leu Ala Thr Gly Gly Ala Gly Met Val Lys Ala Ala Tyr Ser Ser Gly
    210                 215                 220

Lys Pro Ala Leu Gly Val Gly Pro Gly Asn Val Pro Cys Tyr Ile Glu
225                 230                 235                 240
Lys Thr Ala Asn Ile Lys Arg Ala Val Ser Asp Leu Ile Leu Ser Lys
                245                 250                 255
Thr Phe Asp Asn Gly Thr Val Cys Ala Ser Glu Gln Ala Val Ile Ile
            260                 265                 270
Asp Glu Glu Ile Ala Asp Glu Val Lys Lys Leu Met Lys Glu Tyr Gly
        275                 280                 285
Cys Tyr Phe Leu Asn Lys Glu Glu Thr Lys Met Leu Glu Glu Phe Ala
    290                 295                 300
Ile Asp Lys Asn Thr Gly Leu Met Asn Pro Ala Val Val Gly Gln Pro
305                 310                 315                 320
Ala Thr Lys Ile Ala Glu Met Ala Gly Phe Lys Val Pro Glu Asn Thr
                325                 330                 335
Lys Ile Leu Val Ala Glu Tyr Pro Ala Val Gly Pro Glu Tyr Pro Leu
            340                 345                 350
Ser Arg Glu Lys Leu Ser Pro Ile Leu Ala Leu Tyr Thr Val Lys Asp
        355                 360                 365
Tyr Asn Glu Gly Ile Lys Arg Cys Glu Glu Met Thr Glu Phe Gly Gly
    370                 375                 380
Leu Gly His Ser Ala Val Ile His Ser Glu Asn Gln Gln Ile Ile Asn
385                 390                 395                 400
Glu Phe Ala Lys Arg Val Arg Ala Gly Arg Ile Leu Val Asn Ala Pro
                405                 410                 415
Ala Ser Gln Gly Ala Ile Gly Asp Ile Tyr Asn Thr Ala Ile Pro Ser
            420                 425                 430
Leu Thr Leu Gly Cys Gly Thr Met Gly Arg Asn Ser Thr Thr Asp Asn
        435                 440                 445
Val Ser Val Tyr Asn Leu Ile Asn Ile Lys Arg Val Phe Ile Arg Lys
    450                 455                 460
Glu Arg Met Lys Trp Phe Arg Val Pro Pro Gln Ile Tyr Phe Glu Arg
465                 470                 475                 480
Gly Ser Leu Gln Tyr Leu Ser Gln Val Lys Gly Lys Lys Ala Phe Ile
                485                 490                 495
Val Thr Asp Pro Val Met Val Lys Leu Gly Phe Val Asp Lys Val Thr
            500                 505                 510
Tyr Gln Leu Asp Lys Ala Asn Ile Lys Tyr Glu Ile Phe Ser Glu Val
        515                 520                 525
Glu Pro Asp Pro Ser Val Asp Thr Val Glu Lys Gly Val Lys Ile Met
    530                 535                 540
Arg Gly Phe Glu Pro Asp Leu Leu Ile Ala Val Gly Gly Gly Ser Ala
545                 550                 555                 560
Ile Asp Ala Ala Lys Gly Met Trp Leu Phe Tyr Glu Tyr Pro Asp Thr
                565                 570                 575
Lys Phe Glu Asp Leu Arg Leu Lys Phe Met Asp Ile Arg Lys Arg Thr
            580                 585                 590
Tyr Arg Phe Pro Glu Leu Gly Lys Lys Ala Leu Phe Ile Ala Ile Pro
        595                 600                 605
Thr Thr Ser Gly Ser Gly Thr Gly Ser Glu Val Thr Ala Phe Ala Val
    610                 615                 620
Ile Thr Asp Lys Lys Lys Asn Ile Lys Tyr Pro Leu Thr Asp Tyr Glu
625                 630                 635                 640
Leu Thr Pro Asp Ile Ala Ile Ile Asp Pro Asp Leu Thr Met Thr Ile
                645                 650                 655
Pro Pro Ser Val Thr Ala Asp Thr Gly Met Asp Ala Leu Thr His Ala
            660                 665                 670
Ile Glu Ala Tyr Val Ser Val Met Ala Ser Asp Tyr Thr Asp Ala Leu
        675                 680                 685
Ala Glu Lys Ala Ile Lys Leu Ile Phe Glu Tyr Leu Pro Lys Ala Tyr
    690                 695                 700
Lys Asn Gly Gln Asp Lys Val Ala Arg Glu Lys Met His Asn Ala Ser
705                 710                 715                 720
Cys Ile Ala Gly Met Ala Phe Thr Asn Ala Phe Leu Gly Ile Asn His
                725                 730                 735
Ser Met Ala His Ile Leu Gly Ala Lys Phe His Leu Pro His Gly Arg

                740                 745                 750
Ala Asn Ala Ile Leu Leu Pro Tyr Val Ile Glu Tyr Asn Ala Glu Leu
        755                 760                 765
Pro Lys Lys Phe Ala Ser Phe Pro Gln Tyr Glu Tyr Pro Lys Ala Ala
    770                 775                 780
Glu Lys Tyr Ala Glu Ile Ala Lys Phe Leu Gly Leu Pro Ala Ser Thr
785                 790                 795                 800
Ile Glu Glu Gly Val Lys Ser Leu Ile Glu Ala Ile Lys Asn Leu Met
                805                 810                 815
Lys Glu Leu Asn Leu Pro Leu Thr Leu Lys Glu Ala Gly Ile Asn Lys
            820                 825                 830
Glu Glu Phe Glu Lys Gln Ile Met Glu Met Ser Asp Ile Ala Phe Asn
        835                 840                 845
Asp Gln Cys Thr Gly Ser Asn Pro Arg Met Pro Leu Val Ser Glu Ile
    850                 855                 860
Ala Glu Ile Tyr Arg Lys Ala Tyr Gly Glu
865                 870

<210> 66
<211> 2625
<212> DNA
<213> Thermoanaerobacter pseudethanolicus

<220>
<221> source
<222> 1..2625
<223> /mol_type="DNA"
      /organism="Thermoanaerobacter pseudethanolicus"

<400> 66
atgcctaact tattacaaga aagaagagaa gtaaaagaaa aaacagaagt gaaagaaacg      60

ctggatgtta aaagacaaat agaccaatta gtagaaaaag cacaaagagc acaagaaaaa     120

ttcatgtctt atacccaaga acaaatagat aaaatagtta aagctatggc tttggctggt     180

attgaaaatc atgtgagatt ggcaaaatta gcccatgaag aaacaaaaat gggtgtttat     240

gaagacaaaa taacgaaaaa tctttttgca gtagaatatg tgtacaatta tataaaagac     300

aaaaagactg tcggcgtcct tagtgaaaac ttagaagaaa attatatgga agtggcagaa     360

cctgtaggag ttatagccgg cgtaacaccc gttacaaacc ctacttctac aacaatgttc     420

aaaagcttaa tcgctattaa aacaagaaat ccaataattt ttagctttca cccaaaagca     480

ttaaaatgta gtatagaaac agcaaagacc atgtacgagg cagcattaaa agctggtgca     540

ccagaaggat gcataggttg gattgaaacg ccttcaattg aagcaacgca acttctcatg     600

acacatcccg gcgtctccct tatcctcgca acaggcggcg ctggaatggt aaaagccgct     660

tacagctcag gaaaacccgc ccttggcgta ggtcctggca atgtcccctg ctacattgaa     720

aaaactgcaa atattaaaag agcagtctca gacctaatac ttagcaaaac ttttgacaat     780

ggcacagtat gcgcatcaga acaagctgtg ataatagacg aagaaatagc agatgaagtg     840

aaaaaattaa tgaaagaata tggttgctat tttctcaata aagaagagac taaaatgcta     900

gaagaattcg ctatagacaa aaacaccggt ttaatgaatc cagcagttgt aggtcaaccg     960

gcaacaaaaa ttgctgaaat ggctggattt aaagtacctg aaaacacaaa aatacttgta    1020

gctgaatatc ctgctgtagg accagaatat cctctctcca gagaaaagct aagtccaata    1080

cttgctttat acacagtcaa agattacaac gaaggcataa aaagatgtga agaaatgaca 1140

gaattcggcg gacttggtca ctctgcagta atacattctg aaaaccaaca aattattaat 1200

gaatttgcaa aaagagtacg agcaggaaga attcttgtaa acgcccctgc ttctcaagga 1260

gctattggag atatttataa cacggctatc ccatctttga ctttgggttg cggtacaatg 1320

ggtagaaatt caacaacaga taatgtaagt gtttataacc ttataaacat aaaacgagtt 1380

tttataagaa aagagagaat gaaatggttc agagttccac cacaaattta ttttgaaaga 1440

gggtcattac aatatctgtc acaagtaaaa ggcaaaaaag cctttattgt aacagaccct 1500

gtgatggtta aattaggttt tgtagataaa gtaacgtatc aattagataa ggcaaatatt 1560

aaatatgaaa tattctctga agttgaacca gatccttcag ttgacactgt agaaaaaggg 1620

gtaaaaataa tgagagggtt tgagcctgac ttactaatag ctgtaggtgg tggttctgca 1680

atagatgcag cgaagggaat gtggcttttc tacgaatacc ctgatacaaa attcgaagac 1740

ttaaggttaa aatttatgga cataagaaaa agaacttata gatttcctga actggggaaa 1800

aaagctttat ttatcgcaat accaaccacc agcggtagcg gtacaggttc tgaagttact 1860

gcctttgcag ttataacaga caaaaagaaa aatataaagt accctctcac agattacgaa 1920

ttaacaccag atatcgctat aatagaccct gacctcacaa tgacaatacc accctctgtc 1980

acagcagaca ctggtatgga cgctttaacc catgctattg aagcctatgt atctgtaatg 2040

gcatcagact atacagacgc tctcgcagaa aaagctataa aattgatatt tgaatatcta 2100

cctaaagcat acaaaaacgg gcaagataaa gtcgcccgcg aaaagatgca caatgcttca 2160

tgcatagctg gaatggcctt cacaaatgca tttttaggaa taaaccatag tatggctcat 2220

atacttggtg ctaaattcca tcttccacac ggaagagcaa atgcaatact tctgccctat 2280

gtcatagaat acaatgctga gttacctaaa aaatttgctt cattcccaca atatgaatat 2340

cccaaagcag ccgagaaata tgcagaaatt gcaaaattct tgggacttcc tgcatcaacc 2400

attgaagaag gagtaaaaag cctcattgaa gcaataaaaa atcttatgaa agagcttaat 2460

cttcctttga cacttaaaga agcaggcata aataaagaag aattcgaaaa acaaataatg 2520

gaaatgtctg atatagcctt taatgaccag tgcaccggtt caaacccaag aatgccattg 2580

gtatccgaaa ttgcagaaat atatagaaaa gcctatggag aataa 2625

<210> 67
<211> 378
<212> PRT
<213> Propionibacterium freudenreichii subsp. freudenreichii

<220>
<221> SOURCE
<222> 1..378
<223> /mol_type="protein"
/organism="Propionibacterium freudenreichii subsp. freudenreichii"

<400> 67
Met Asp Phe Ser Leu Thr Glu Asp Gln Gln Leu Met Val Asp Gly Phe
1               5                   10                  15
Thr Glu Leu Met Asn Ser Glu Ala Trp Glu Lys Tyr Phe His Glu Cys
            20                  25                  30
Asp Glu Asn Ser Glu Tyr Pro Glu Arg Trp Val Lys Ala Ile Cys Asp
        35                  40                  45
Leu Gly Phe Asp Arg Ile Leu Leu Pro Glu Glu Tyr Asp Gly Leu Gly
    50                  55                  60
Leu Gly Trp Gln Thr Leu Ala Ala Ala Tyr Glu Ala Leu Gly Arg Ala
65                  70                  75                  80
Gly Gly Pro Thr Tyr Val Leu Tyr Gln Leu Pro Gly Trp Asp Thr Val
                85                  90                  95
Ile Arg Glu Gly Thr Glu Glu Gln Lys Lys Asp Ile Leu Lys Phe Val
            100                 105                 110
Gly Ser Gly Lys Gln Met Leu Asn Tyr Ala Met Thr Glu Pro Ser Ala
        115                 120                 125
Gly Ser Ser Trp Asp Asp Met Ser Thr Thr Tyr Ser Arg Lys Gly Gly
    130                 135                 140
Lys Val Tyr Leu Asn Gly His Lys Thr Phe Ile Thr Ser Ser Met Lys
145                 150                 155                 160
Val Pro Tyr Leu Val Val Met Ala Arg Asp Ala Asp Asn Met Gly Thr
                165                 170                 175
Tyr Thr Glu Trp Phe Val Asp Met Ser Leu Pro Gly Ile Thr Lys Glu
            180                 185                 190
Pro Leu His Lys Leu Gly Leu Arg Met Asp Ser Cys Ala Asp Ile Tyr
        195                 200                 205
Phe Asp Asn Val Glu Leu Arg Glu Glu Asp Leu Phe Gly Thr Glu Gly
    210                 215                 220
Asn Gly Phe Lys Arg Gly Val Lys Asp Phe Asp Leu Glu Arg Phe Glu
225                 230                 235                 240
Val Ala Met Thr Asn Tyr Gly Thr Ala Tyr Cys Ala Phe Glu Asp Ala
                245                 250                 255
Ala Lys Tyr Ala Asn Gln Arg Val Gln Gly Gly Glu Ala Ile Ala Arg
            260                 265                 270
Lys Gln Leu Ile Gln Leu Lys Phe Ala Asp Met Lys Val Asp Ile Thr
        275                 280                 285
Asn Met Arg Asn Met Leu Tyr Glu Ile Ala Trp Lys Ala Asp Asn Asp
    290                 295                 300
Gln Leu Gly Arg Gly Asp Cys Ser Met Ala Lys Tyr Tyr Cys Ser His
305                 310                 315                 320
Ala Ala Ala Arg Val Val Asp Asn Ala Leu Gln Thr Leu Ala Gly Val
                325                 330                 335
Gly Val Thr Gly Glu His Arg Val Gln Arg Phe Tyr Arg Asp Leu Arg
            340                 345                 350
Val Asp Arg Val Ser Gly Gly Thr Asp Glu Met Met Ile Leu Ala Ala
        355                 360                 365
Gly Arg Gly Ala Leu Arg Asp Tyr Arg Ser
    370                 375

<210> 68
<211> 1137
<212> DNA
<213> Propionibacterium freudenreichii subsp. freudenreichii

<220>
<221> source
<222> 1..1137
<223> /mol_type="DNA"
      /organism="Propionibacterium freudenreichii subsp. freudenreichii"

<400> 68
atggatttct cattgaccga agaccagcag ctcatggtgg acggcttcac cgagctgatg      60

aacagtgagg catgggagaa gtacttccac gagtgtgacg agaactcgga gtatcccgag      120

cgctgggtca aggcgatctg cgacctcggc ttcgaccgca tcctgctgcc cgaggaatac 180

gacggcctgg gcctgggctg gcagacgttg gccgccgcct atgaggcgct cggccgcgct 240

ggtggcccca cctatgtgct ctaccaactg cccggctggg acacggtgat ccgcgaaggc 300

accgaggaac agaagaagga catcctcaag ttcgtgggca gcgggaagca gatgctcaac 360

tacgccatga ccgagcccag cgccgggtcg agctgggacg acatgtccac cacctacagc 420

cgcaagggcg gcaaggtgta cctcaacgga cacaagacct tcatcacctc atccatgaag 480

gtgccctacc tcgtggtgat ggcccgcgac gccgacaaca tgggcaccta caccgagtgg 540

ttcgtcgaca tgagccttcc gggcatcacc aaggagccgc tgcacaagct cggcctgcgc 600

atggattcgt gcgccgacat ctacttcgac aacgttgaac tgcgcgagga agacctgttc 660

ggcaccgagg gcaacggctt caagcgcggc gtgaaggact tcgacctgga gcgcttcgag 720

gtggccatga ccaactacgg caccgcctac tgcgccttcg aggatgcagc gaagtacgcc 780

aaccagcgcg tgcagggagg cgaggcgatt gcccgcaagc agctgatcca gctgaagttc 840

gccgacatga aggtggacat caccaatatg cgcaacatgc tctacgagat tgcctggaag 900

gccgacaacg accagctggg ccgcggcgac tgctccatgg cgaagtacta ctgctcccac 960

gccgcggccc gcgtggtcga caacgcgctg cagaccctcg ccggcgtggg cgtcaccggt 1020

gaacaccgcg tgcagcgctt ctaccgcgac ctccgcgtgg accgcgtctc cggtggaacc 1080

gacgaaatga tgatcctcgc cgccggccgt ggtgcactgc gcgactaccg cagctga 1137

<210> 69
<211> 44
<212> DNA
<213> Artificial Sequence

<220>
<221> source
<222> 1..44
<223> /mol_type="DNA"
/note="An artificially synthesized primer sequence"
/organism="Artificial Sequence"

<400> 69
gaccatatgc ctaacttatt acaagaacgc cgcgaagtaa aaga 44

<210> 70
<211> 45
<212> DNA
<213> Artificial Sequence

<220>
<221> source
<222> 1..45
<223> /mol_type="DNA"
/note="An artificially synthesized primer sequence"
/organism="Artificial Sequence"

<400> 70

gctgttaatt aattattctc cataggcttt gcgatatatt tctgc                    45

<210> 71
<211> 36
<212> DNA
<213> Artificial Sequence

<220>
<221> source
<222> 1..36
<223> /mol_type="DNA"
      /note="An artificially synthesized primer sequence"
      /organism="Artificial Sequence"

<400> 71
gaccatatgg atttctcatt gaccgaagac cagcag                              36

<210> 72
<211> 35
<212> DNA
<213> Artificial Sequence

<220>
<221> source
<222> 1..35
<223> /mol_type="DNA"
      /note="An artificially synthesized primer sequence"
      /organism="Artificial Sequence"

<400> 72
gctgttaatt aactacggta gtcgcgcagt gcacc                               35